# EUROPEAN PATENT APPLICATION

(11) **EP 2 600 154 A1**
(43) Date of publication of application: **05.06.2013**
(21) Application number: 11765817.9
(22) Date of filing: 31.03.2011
(51) Int. Cl.: G01N 33/68, A61K 45/00, C12Q 1/68, G01N 27/447, G01N 27/62, G01N 33/58, G01N 37/00, G06F 19/28

(54) **METHOD FOR GENERATING DATA SET FOR INTEGRATED PROTEOMICS, INTEGRATED PROTEOMICS METHOD USING DATA SET FOR INTEGRATED PROTEOMICS THAT IS GENERATED BY THE GENERATION METHOD, AND METHOD FOR IDENTIFYING CAUSATIVE SUBSTANCE USING SAME**

(30) Priority: 31.03.2010 JP 2010081525; 31.03.2010 JP 2010081524
(71) Applicant: National University Corporation Kumamoto University, Kumamoto-shi Kumamoto 860-8555 (JP)
(72) Inventor: ARAKI Norie, Kumamoto-shi Kumamoto 860-8556 (JP); MIZUGUCHI Souhei, Kumamoto-shi Kumamoto 860-8556 (JP); KOBAYASHI Daiki, Kumamoto-shi Kumamoto 860-8556 (JP); TSUBOTA Nobuyuki, Kumamoto-shi Kumamoto 860-8556 (JP); MORIKAWA Takashi, Kumamoto-shi Kumamoto 860-8556 (JP); KURATSU Junichi, Kumamoto-shi Kumamoto 860-8556 (JP); WILSON MORIFUJI Masayo, Kumamoto-shi Kumamoto 860-8556 (JP)
(74) Representative: Uchida, Kenji
(86) International application number: PCT/JP2011/058366
(87) International publication number: WO 2011/125917

(57) **Abstract**

Provided are a method for generating a data set for integrated proteomics analysis, whereby expression level variations of both of proteins and genes can be integrally united together and, moreover, highly accurate and appropriate analysis results can be obtained compared with the existing cases where the expression variation amount of proteins or genes is singly analyzed, an integrated proteomics analysis method, a method for identifying a protein causative of a disease or the like using these methods, and a method of using the same. The aforesaid method for generating a data set for integrated proteomics analyses comprises: a protein identity number-assigning step for assigning common identity numbers to the expression variation amount data of individual proteins; a gene identity number-assigning step for assigning common identity numbers to the expression variation amount data of individual genes; a data-binding step for binding together the set of the expression variation amount data; a data-rejecting step for rejecting, among the individual expression variation amount data constituting the thus bound data set, data showing a p-value equal to or greater than a specific level; and a data-selecting step for selecting one data, from a set of data having the same common identity number assigned thereto, on the basis of a definite requirement to thereby generate the data set to be subjected to integrated proteomics analyses. Further, the data set for integrated proteomics analyses thus generated is subjected to GO analysis and network analysis to thereby identify a protein causative of a disease, a pathological condition or the like. Furthermore, the causative protein thus identified is usable, for example, a tumor marker or a clinical target.

## Description

### TECHNICAL FIELD

The present invention relates to a generation method for generating a set of data for an integrated proteomic analysis, an integrated proteomic analysis method using a set of data for an integrated proteomic analysis generated by the generation method therefor, and a method for identifying a causative substance using the same. More specifically, the present invention relates to the generation method for the generation of a set of data for an integrated proteomic analysis based on a set of comprehensive data of amounts of variations in expression of active substances such as proteins and a set of comprehensive data of amounts of variations in expression of genes, the integrated proteomic analysis method using the data sets for the integrated proteomics analyses, as well as the method for identifying the causative substances such as proteins using the integrated proteomic analysis method.

### BACKGROUND TECHNOLOGY

Since the analysis of the full base sequence of a human genome has been completed in 2003, various attempts have so far been made to analyze full base sequences of genomes of various organisms, and the analyses of the full base sequences of several organisms including the human being have already been finished. As a result of the completion of analysis of the human total genomic sequence, it has been greatly expected that a group of disease-associated genes of various diseases including cancers and so on would be elucidated, thereby leading to development of appropriate methods for treating and preventing diseases. As studies of disease-associated genes have advanced, however, it has now been recognized that genomic information may not be involved directly in treatment and prevention of diseases and consequently reasonable methods for the treatment and prevention of diseases such as cancers may not be developed solely on the basis of the genomic information.

At this end, therefore, it has now come to an understanding that a state of the gene expression in cells of a living body has to be grasped comprehensively by measuring and analyzing amounts of variations in mRNA expression levels at the gene portion on a DNA sequence at a cell level by way of sequence analyses of not only genes causative of diseases but also a collection containing all gene transcription products (including mRNAs and primary transcription products) present in cells.

As a result of studies for retrieving disease-associated genes for many years, a group of disease-associated genes and a group of cell cycle checkpoint adjustment molecules, so-called a gate keeper, have been discovered. They are especially important only in the particular cells of a certain tissue and may become causes of an oncogenesis of particular tissues or cells by a breakdown of their functions due to deletion, mutation, et cetera of genes. It is also known that functions of the proteins produced by these genes may be regulated generally by activating or inactivating the proteins, for example, by post-translational modifications or fragmentation such as phosphorylation or proteolysis. Further, it is known that the functions of the proteins are regulated by a complex crosstalk network including, for example, a sequential connection or interaction with other various disease-associated proteins, a group of physiologically active proteins or nucleic acids, and so on.

In order to elucidate causes of the life phenomenon of an organism such as diseases, therefore, it has further come to an understanding that a comprehensive and accurate grasp is needed regarding details of function-expressing mechanisms of pathological conditions, a biological individuality of an object, real time pathological conditions, a state of progress of diseases, and so on, by means of not only transcriptomic analyses of genes involved in the expression of proteins working as substances causative of diseases but also proteomic analyses of proteins working causative substances.

Moreover, as the latest technical innovations of proteomic analyses have advanced, proteins and post-translationally modified molecules such as transcription factors, which are present in minute amounts and likely to change rapidly in a living body and consequently which have so far been considered to be very difficult to obtain information, can now be analyzed to provide information on such proteins and molecules in a highly sensitive and quantitative way and with a high throughput. Therefore, by making full use of such proteomic analysis technology and information of database, information on intercellular molecular networks involved most in an onset or progress of pathological conditions, sensitivity to medicines, etc. can be extracted by integrating information on the proteomic analysis of various proteins obtainable from pathological samples, etc. with data of the transcriptomic analysis for comprehensively analyzing variations in gene expression using DNA microarrays and quantitative PCR, etc.

The conventional proteomic analyses, however, have generally been involved in retrieving pathological conditions, progressive states of various diseases, and so on, at a protein level, by detecting and identifying proteins mainly by way of differential display and database retrieval, which were isolated, analyzed and retrieved by analyses at a gene level using an appropriate comprehensive protein analysis device and DNA microarray methods, and narrowing a target protein down at a stand-alone fashion.

It can be further noted herein that the conventional proteomic analyses were not so intelligent to be applied to a high-throughput clinical examination because the analysis was required to be made at a manual level in many aspects using a technical background based on experiences of researchers. As one means for solving these situations, therefore, an array system such as a protein chip system has been developed. Such an array system, however, was not sufficient in terms of precision, analysis time, automation, on-line, and so on.

In order to meet such demands, the present inventors have constructed a protein structure analysis system mainly composed of a high performance liquid chromatography (HPLC) having an advantage of making various detection systems and reaction system on line on the basis of the concept that the high-throughput on-line analysis system is essential for the proteomic analysis (Patent Document No. 1). This system makes it feasible to identify proteins at a peptide level and improve an accuracy of analysis to a remarkable extent.

The high-throughput comprehensive analysis as described above, however, reveals that information on all proteins in tissues and cells cannot be comprehensively analyzed only by the differential display analysis using a two-dimensional electrophoresis or a LC-shot gun method, although it has dramatically contributed to shortening a period of research or diagnosis of diseases including cancers, etc., and developing a genomic medicine market.

Mass spectrometers which have been conventionally used for an analysis of an amino acid information on peptides utilize various ionization methods (e.g., MALDI method, EST method, etc..) and different separation and detection techniques, which may be appropriate for the identification of certain peptide species, but inappropriate for the identification of other peptide species and vice versa. Therefore, an analysis using only one mass spectrometry technique, or only one analysis methodology, may be said to be insufficient because it cannot identify all proteins inside tissues and cells in a comprehensive fashion. In order to comprehensively identify all proteins present in the tissues and cells, therefore, it is needed to use plural analysis methods using plural mass spectrometers.

In the event where proteins of tissues or cells are to be analyzed comprehensively, an enormous amount of data is generated from plural analysis apparatuses. In addition, such data may contain data of various types and data of different and independent language types outgoing from plural analysis apparatuses. Therefore, this is the great barrier for conventional analysis methods to comprehensively analyze such data as written in different languages, etc. in an integrated and high-throughput fashion.

In other words, as described above, the conventional proteomic analysis techniques could not analyze and integrate such analysis data by a common format generated in different languages from plural analysis apparatuses. It has so far been difficult, therefore, to reproduce intermolecular protein networks and post-translational modifications as well as interactional functions of decomposition products of intermolecular proteins, and so on, which are highly important, for example, for a proteomic analysis of pathological conditions. Moreover, the information of mRNAs obtainable by DNA microarray analysis, real time PCR analysis, etc. to be used for the transcriptomic researches could not be analyzed easily because it was very difficult to integrate it with information on the proteomic analysis obtained from the same sample, although the information on mRNAs is associated greatly with information on proteins. Further, even if an enormous volume of data could be gathered by analyses based on different concepts using plural analysis apparatuses, it has been highly difficult to connect those data together and interpret them in a coordinate and integral fashion. Accordingly, an original and comprehensive analysis could not be achieved in the actual situation. Therefore, it has encountered great difficulties until now in confirming an assessment or reproduction of post-translational modifications and interactional functions with decomposition products of intermolecular proteins and so on, although such an assessment or reproduction was needed for the proteomic analysis. Under the conventional circumstances, accordingly, it has been very difficult to integrate and analyze such data even if a new technique and device based on a new concept could be developed, as long as an integrated concept applicable to integrate such data could not be discovered.

Therefore, the conventional proteomic analyses have used raw data consisting of a set of data of amounts of variations in expressions of proteins, obtained by various transcriptomic analysis and proteomic analysis techniques, individually and separately for analyzing functions of proteins. As a result, they have left an issue unsolved that an interpretation and utilization of results of such analyses would be questionable and it was made very difficult to efficiently and flexibly understand and analyze a total image of the variations in expression of proteins, particularly intermolecular proteins, from such results of the raw data.

One of the reasons may lies in the fact that the total image of results of those comprehensive analyses cannot be looked over and analyzed by integrating it on an identical level due to differences in meanings and qualities of information obtained each from the results of analyses using various techniques of transcriptomic analyses and proteomic analyses as well as output modes of such analysis results.

In other words, proteins are expressed by transcription and translation of the corresponding genes. After translation, the proteins may be modified in various ways, for example, by way of phosphorylation, methylation, acetylation, glycosylation, cleavage at a particular site, and the like, thereby exhibiting various molecular behaviors as well as being localized and varied in various states in a living body.

It is now noted herein that the transcriptomic analysis shows a state of the in vivo expression of proteins before translation, while various proteomic analysis reflects states of the in vivo expression of proteins and their modifications after translation. Each of the transcriptomic analyses and the proteomic analyses can provide information on different in vivo behaviors of molecules having qualities specialized in each of the analysis techniques. It is also to be noted that, in reality, the amounts of variations in the gene expression and their states of post-translational modifications are not always consistent with those in vivo states of proteins. Therefore, in the event that data are evaluated and analyzed individually by each of the comprehensive analysis methods, the results of this analysis may be said to be obtained based on biased information on the expression of molecules specialized in each of the analysis methods and, as a consequence, it goes without saying that the whole picture of the in vivo functions of the molecules and their networks are far from clarification.

For instance, in the event that an analysis of protein functions would be implemented solely on the basis of information on amounts of variations in the gene expression obtained by various gene analysis methods as used for many studies on life phenomena, this analysis may in fact present results, in many occasions, which have many noises or which may be different and wrong from those of an actual protein expressed in cells.

Further, there may arise some occasions that data of amounts of variations in gene expression and results of various proteomic analyses amounting to several ten thousands are to be analyzed separately and independently. On this occasion, however, there may be a high risk of missing a significant result or causing a misunderstanding, or an enormous amount of time as well as experienced skills and knowledge relating to data analysis will be needed to presume a right result.

In order to efficiently proceed with a proteomic analysis, the present inventors have developed an analysis program enabling an analysis of molecular functions at a high precision and a high speed as well as for a short time, while ensuring a reliability of data (Patent Document No. 2). This analysis program enables an integrated and centralized management, as a database for a proteomic analysis, of a voluminous amount of data of information on proteins of many and various data types obtainable from various analysis apparatuses and analysis software used in the field of research on proteomic analyses, and data of protein molecules led from the field of research on genomics and transcriptomics analyses and a storage of results of functional analyses performed by data mining as a database for a functional analysis. This analysis program can also distinguish the integrated information of proteins classified based on experiences by researchers themselves or by an automated computation from highly reliable raw data obtained by experiments, thereby establishing a highly accurate analysis of molecular functions rapidly and shortly, while ensuring a highly reliable data (Patent Document No. 2).

This prior art proteomic analysis system can convert and integrate data stored in a proteomic analysis database into data of an identical language and an identical type, the database storing various data containing at least a mass spectrometric signal pattern for a mass spectrograph, electrophoresis patterns and various different features, which are obtained by the proteomic analysis method using tissues or cells derived from patients with various pathological conditions or disease model animals or cultured cells, and retrieve other public databases accessible through networks, thereby extracting a group of specific proteins and specific intermolecular signaling networks. From those results as obtained above, the protein functions can be predicted and verified with particular protein chips, thereby enabling a collection of information on an analysis of pathological conditions and information on most securable treatment means and new medicines to be developed.

As a web-accessible program for performing an integrated functional annotation and classification relating to plural molecules, there is known a program called DAVID (Database of Annotation, Visualization and Integrated Discovery). This DAVID, however, encounters disadvantageous issues as will be described hereinafter, when compared to a method for the creation of a set of data for the integrated proteomic analysis according to the present invention.

In other words, the DAVID have the disadvantages as follows: (1) the DAVID obtained by the data mining does not give a sufficient consideration to differences in significance (quality) of the results obtained by various research techniques, that is, to the problems as presented in this description. Therefore, the interpretation of the results obtained by various comprehensive analyses of variations in expression may result merely in the information analysis based on existing databases depending solely on an ID (an identity number assigned to each molecule) and an amount of expression of an individual molecule with varying expression levels. (2)Further, it cannot be utilized while raw data of the analysis results containing detailed numerical information etc. are left as they are. (3) Moreover, information involved in post-translational modifications of proteins obtainable by mass spectrometry and 2D-DIGE etc. cannot be utilized for analysis.

It is to be noted herein, however, that the prior art proteomic analysis systems may enable a comprehensive analysis of a molecule group having common functions, for example, associated with a certain disease, however, they cannot specify a causative molecule associated with the function that is common to the molecular group.

Furthermore, in studies on diseases, it now becomes important to explain a physiological phenomenon of each one molecule located in a cell or tissue from the molecular side as well as analyze all associated proteins expressing in cells along the axis of time in a comprehensive fashion, that is, comprehensive information obtained by proteomic analyses. Therefore, one of the most significant problems involved in research in diseases as well as treatment, prevention and diagnosis of diseases is to develop a methodology that aims at performance and practical application of proteomic analysis, for instance, which may provide comprehensive information regarding intercellular proteins causing expression variations and mechanisms causing the expression variations as well as processes of a breakdown of normal cell activity causing the onset of diseases as well as an application of the information to clinical medicine of diseases.

In addition, it is needed to ascertain a reproducibility of data by reproducing a particular proteinous molecule in a living body even if whatever highly reliable data could be obtained for the particular proteinous molecule as a result of retrieval by an analysis program. Further, in order to develop usage of such a particular protein for detection and diagnosis of a particular gene mutation involving a morbidity of a particular disease, susceptivity to medicines, and side effects of medicines, etc., it is needed to arrange for contents that can specify a particular probe for a particular event. Moreover, there are many events that can be analyzed for the first time by a combination of plural probes even if it cannot be analyzed by a single probe.

### Prior Art Documents:

Patent Document No. 1: Japanese Patent Publication No. 2003-149,223
Patent Document No. 2: Japanese Patent Publication No. 2006-294014

### SUMMARY OF THE INVENTION

Hence, in order to obtain compressive information by conducting a time-dependent proteomics analysis of all intercellular expressed proteins associated with an in vivo physiological phenomenon of a certain molecule in living cells or tissues, the present inventors have created an integrated proteomic analysis system by combining data obtainable by a transcriptomic analysis technique for comprehensively analyzing variations in gene expression with data obtainable by a proteomic analysis technique for comprehensively analyzing variations in protein expression and identifying the proteins, thereby forming a research strategy, applying the integrated proteomic analysis system to investigate the causes of diseases and so on and develop new medicines. The present invention has been completed accordingly based on these findings.

More particularly, the present invention has the objects to provide a generation method for generating a set of data for an integrated proteomic analysis obtained by analyzing two groups of different samples, for example, having identical pathological observations of a disease yet having different medicinal dynamics and behaviors separately by a transcriptomic analysis technique for comprehensively analyzing variations in gene expression using DNA microarrays, quantitative PCRs, etc., and a proteomic analysis technique for comprehensively analyzing and identifying variations in protein expression using a combination of proteomic high performance liquid chromatography (HPLC), two-dimensional electrophoresis, etc. with mass spectrometry (MS) as well as integrating data obtained by the transcriptomic analysis with data obtained by the proteomic analysis; to provide an integrated proteomic analysis method. The present invention has another object to provide a research strategy for applying the integrated proteomic analysis system to an investigation of the causes of diseases and so on and development of new medicines by retrieving and identifying a group of molecules associated with diseases in a living body.

The integrated proteomic analysis system according to the present invention enables a comprehensive analysis of behaviors of many molecules mixed in a sample in a short time and in an accurate fashion as well as an integrated analysis of expression analysis, retrieval, and identification of various proteins, thereby applying results of analysis to not only the field of medicine including, for example, development of new medicines but also the field of biology and agriculture, and the like.

The proteomic analysis method according to the present invention permits a comprehensive analysis of, for instance, an amount of a variation in expression levels of a substance which is considered to cause a disease as one of the human life phenomena, thereby anticipating the resulting substance as a causative substance. Further, although there may be many occasions that a certain medicine is effective for one group of patients with an identical disease yet ineffective for another group of patients therewith, the proteomic analysis method according to the present invention enables a comprehensive analysis of the causative substance, thereby effectively applying results of analysis to treatments and preventions of diseases and treatments of prognosis as well.

Further, the integrated proteomic analysis method according to the present invention enables a statistical generation of a set of data of amounts of variations in expression levels of an individual protein from results of an analysis of each of the sample groups and an application of the resulting data set to a genetic function analysis (gene ontology: GO analysis) and a network analysis including, for example, an in vivo signaling path analysis, a metabolic path analysis, etc. These analyses may predict in vivo behaviors of medicines in each patient group and functions of particular proteins associated therewith as well as differences in behaviors or functions of medicines. This prediction may also be utilized for treatment or prevention of diseases associated with a particular protein by enhancing or deleting their functions, etc. Numerous attempts have so far been made to identify new tumor markers by utilizing these techniques (for example, see Japanese Patent Publication No. 2010-14,689).

Therefore, the present invention has the object to provide a generation method for the generation of a set of data for an integrated proteomic analysis, which enables an overview and an understanding of results of a comprehensive analysis of amounts of variations in expression of both of proteins and genes by taking them into account in an integral fashion. The present invention can provide more accurate and appropriate results of analysis when compared with conventional analyses of the expression variation amounts of proteins and genes, respectively, and it can be applied to investigation of causes of diseases or development of new medicines. The present invention has another object to provide an integrated proteomic analysis method which is useful in particular for investigation of causes of diseases or development of new medicines.

Moreover, the present invention has a further object to provide a method for retrieving and identifying a causative protein associated with the cause of a certain disease, comprising a retrieval and identification of a particular substance involved in in vivo behaviors of medicines in each patient group by statistically generating a data set of expression variation amounts of an individual protein from the results of the above integrated proteomic analysis of each sample group and by implementing GO analysis or network analyses including, for example, signaling path analysis and metabolic path analysis, and the like.

In order to achieve the above objects, the present invention in one aspect provides a generation method for generating a data set for an integrated proteomic analysis for use with a proteomic analysis applicable to a protein function analysis and development of a new medicine on the basis of a comprehensive data set of protein expression variation amounts, i.e., amounts of variations in protein expression levels, between two different sample groups and a comprehensive data set of gene expression variation amounts, i.e., amounts of variations in gene expression levels, between the two different sample groups, which comprises:
a common protein identity number assignment step for assigning a common protein identity number to data of an expression variation amount, i.e., an amount of a variation in expression levels of an individual protein constituting the comprehensive data set of the protein expression variation amounts, the common protein identity number linking to a protein identity number of the protein in a first database and a gene identity number of the gene encoding the identical protein in a second database;
a common gene identity number assignment step for assigning a common gene identity number to data of an expression variation amount of an individual gene, i.e., an amount of a variation in gene expression levels constituting a comprehensive data set of protein expression variation amounts, i.e., amounts of variations in gene expression levels, the common gene identity number linking to a gene identity number of the gene in a third database and the protein identity number of the protein expressed from the corresponding gene in the first database;
a data connection step for connecting the comprehensive data set of the protein expression variation amounts obtained through the protein identity number assignment step to the comprehensive data set of the gene expression variation amounts obtained through the gene identity number assignment step and synthesizing an integrated data set from the protein expression variation amount data and the gene expression variation amount data of the respective comprehensive data set;
a data rejection step for rejecting data of the integrated data set when a p-value of the data obtained by a statistical significance test of the protein expression variation amount data or the gene expression variation amount data between the two different sample groups among the respective expression variation amount data constituting the connected data set is equal to or greater than a predetermined value or when an F-value obtained by a variance (ANOVA) analysis of the data obtained thereby is equal to or lower than a predetermined value; and
a data acceptance step for selecting either of the data for the integrated proteomic analysis based on a predetermined condition, the data being provided with an identical common identity number between the protein expression variation amount data and the gene expression variation amount data among the integrated data set obtained through the data rejection step.

The present invention in its preferred embodiment provides the generation method for generating the integrated proteomic analysis data set, wherein the comprehensive data set comprises a set of data obtainable by a comprehensive analysis of variations in protein expression levels using a high performance liquid chromatography (HPLC) and a mass spectrometry (MS) and/or a set of data of the proteins containing information on post-translational modifications obtainable by a 2-dimensional fluorescence difference gel electrophoresis (2D-DIGE) and a mass spectrometry (MS).

The present invention in another preferred embodiment provides the generation method for generating the integrated proteomic analysis data set, wherein the comprehensive data set of the gene expression variation amounts is a set of data obtainable by a DNA microarray analysis.

The present invention in another preferred embodiment provides the generation method for generating the integrated proteomic analysis data set, wherein the predetermined condition set in the data acceptance step is data involved in amounts of variations in expression levels of proteins.

The present invention in another mode provides an integrated proteomic analysis method wherein the integrated proteomic analysis data set generated by the generation method for generating the integrated proteomic analysis data set is applied to the integrated proteomic analysis.

The present invention in its preferred embodiment provides the integrated proteomic analysis method, wherein a molecular network significant in a living body is grasped by changing a color representative of a molecule corresponding to each of the data relating to the expression variation amount data visualized by the integrated proteomic analysis in accordance with a value of each of the expression variation amount data constituting the integrated proteomic analysis data set.

The present invention in another mode provides a method for the identification of a causative protein wherein the integrated proteomic analysis data set generated by the generation method according to the present invention is analyzed by the gene ontology analysis (GO analysis) and the network analysis including, for example, an in vivo signaling path analysis or a metabolic pathanalysis to identify a protein as the causative protein, the protein being the protein having the largest expression variation amount or its post-translationally modified protein or the protein which is linked nearby upstream or downstream to the protein having the largest expression aviation amount obtained by the network analysis.

The present invention in its preferred embodiment provides the method for the identification of the causative protein wherein the causative protein is identified which is associated with a disease or a pathological condition caused to occur by abnormality involved in, for example, cell proliferation, cell division or apoptosis including, but being not limited to, carcinogenesis, susceptivity to medicines, resistance to medicines, metastasis, immune deficiency, and so on.

The present invention further provides a method for using the causative protein as a marker for a disease or a pathological condition caused to occur by abnormality relating to cell proliferation, cell division or apoptosis including, but being not limited to, carcinogenesis, sensitivity to medicines, resistance to medicines, metastasis, immune deficiency, and so on. Further, the present invention provides a method for the inhibition of the expression of the causative protein wherein an event such as, for example, a disease or a pathological condition, which may be caused to occur by the causative protein, is treated or prevented by inhibiting the expression of the causative protein.

The present invention in its preferred embodiment provides the method for the identification of the causative protein, the use of the causative protein, as well as the method for the inhibition of the causative protein, wherein the causative protein may comprise vimentin, phosphorylated vimentin, Ephrins, hypoxia-inducible factor-1 (HIF-1), and so on.

The present invention provides the generation method for the generation of integrated proteomic analysis and the integrated proteomic analysis method, which enable an analysis of a total image of in vivo behaviors of molecules while integrally taking the expression variation amounts of both of the proteins and the genes into consideration and, furthermore, which can provide a more efficient, more accurate and appropriate analysis compared to conventional analyses in which the expression variation amounts of each of the proteins or the genes are analyzed solely by a single analysis method.

The retrieving method for retrieving the causative proteins according to the present invention can provide the great merits that it may facilitate development of medicines targeting proteins or genes because it may retrieve a particular protein associated with in vivo dynamics of medicines.

### BRIEF DESCRIPTION OF THE ACCOMPANYING DRAWINGS

Fig. 1 is an explanatory illustration showing a scheme of transcriptomic analysis and proteomic analysis according to a working example of the present invention.
Fig. 2 is a list showing a portion of a comprehensive data set of expression variation amounts of proteins according to a working example of the present invention.
Fig. 3 is a list showing a portion of a comprehensive data set of expression variation amounts of genes according to a working example of the present invention.
Fig. 4 is a concept illustration showing steps of the generation of a data set for integrated proteomic analysis according to the working example of the present invention.
Fig.5 is a list showing a portion of the comprehensive data set of the expression variation amounts of the proteins in a protein identity number assignment step.
Fig.6 is a list showing a portion of the comprehensive data set of the expression variation amounts of the genes in a gene identity number assignment step.
Fig. 7 is a list showing a portion of a connected data set in the data connection step.
Fig. 8 is a list showing a portion of the connected data set in the data rejection step and the data acceptance step.
Fig. 9 is a schematic illustration showing an integrated proteomics data mining system using iPEACH/MANGO.

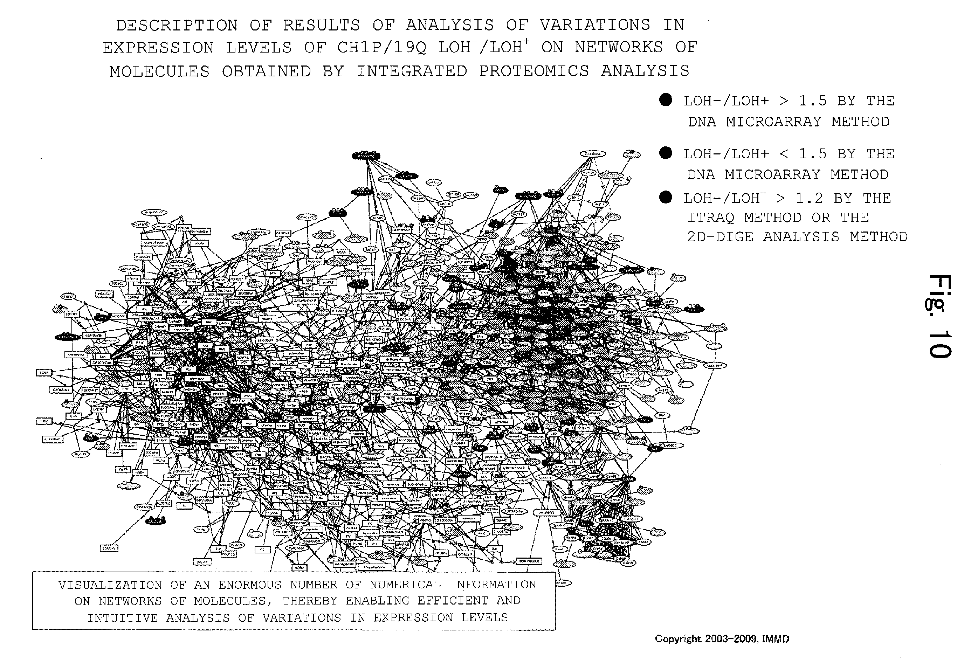

Fig. 10 is an explanatory illustration showing results of the integrated proteomic analysis using the integrated proteomics data mining system. In this figure, the molecules having a value of the expression variation amount of LOH-/LOH⁺ > 1.5 are expressed violet in color and those having a value of the expression variation amount of LOH⁻/LOH⁺ < 1.5 are expressed blue in color, when they were analyzed by the DNA microarray method, as well as the molecules having a value of the expression variation amount of LOH⁻/LOH⁺ > 1.2 are expressed red in color when they were analyzed by the iTRAQ method or the 2D-DIGE analysis method. It is provided, however, that each of the molecules is indicated by shading due to a limitation of printing paper.
Fig. 11 is a network chart showing a network of molecules extracted by iPEACH, whose expression is upregulated in glioma cells resistant to chemotherapy.
Fig. 12A is a diagram showing amounts of vimentins expressed in tissues of LOH⁺ and LOH⁻ AO patients.
Fig. 12B is Western blotting patterns showing vimentin in the glioma tissues.
Fig. 12C is a drawing showing the expression of ephrin receptor A4 (EphA4) in the LOH⁺ and LOH⁻ tissues of an AO patient, respectively.
Fig. 13 is a diagram showing an overall survival rate of AO/AOA patients (an upper view) and an overall survival rate of progression-free patients (a lower view).
Fig. 14 is an illustration showing primary structures of vimentin spots separated and identified in the LOH⁻ tissue by 2D-DIGE.
Fig. 15 is a schematic diagram showing a hypothesis indicative of a vimentin activation loop in the glioma cells resistant to chemotherapy.
Fig. 16 is a schematic illustration showing metabolites obtainable by the metabolism of a DNA alkylating chemotherapeutic, temozolomide (TMZ), in glioma cells.
Fig. 17 is a diagram showing a list of the top 30 proteins with the expression upregulated among those identified from tissue samples of 1p/19q LOH⁻ glioma patients by iTRAQ analysis method.
Fig. 18 is a diagram showing a list of the top 30 proteins with the expression upregulated among those identified from tissue samples of 1p/19q LOH- glioma patient by 2D-DIGE analysis method.
Fig. 19 shows a profile of phosphorylated proteins.
Fig. 20 is an electropherogram showing vimentin spots identified in the tissue separated from a LOH- patient by 2D-DIGE analysis.
Fig. 21A is a drawing showing results of the vimentin expression by an immunohistochemical analysis of tissues of an AO patient and the glioma cell line using a vimentin antibody.
Fig. 21B is a Western blotting pattern of vimentin in an AO tissue.
Fig. 22 is a drawing showing results of silencing of vimentin using siRNA during the growth of glioma cells (U373 and A172), respectively.
Fig. 23 is a drawing showing differences in resistances of glioma cell line (U373 and A172) to TMZ. (A): chemoresistance to TMZ by varying concentrations of TMZ. (B): chemoresistance of vimentin siRNA and a control vimentin siRNA to TMZ, respectively.
Fig. 24 is a network chart showing a molecular network of postulated functional regulation of vimentin in glioma cells.
Fig. 25 is a drawing showing results of glioma cells U373 treated with a PAK (p21 activated kinase) inhibitor. The left-handed drawing shows the glioma cell U373 treated with the PAK inhibitor, and the right-handed drawing shows a negative control.
Fig. 26 is a drawing showing results of chemosensitivity to medicine upregulated by the PAK inhibitor in the glioma cell U373.
Fig. 27 is a drawing showing fragmentation of vimentin in glioma cell line U373.
Fig. 28 is a drawing showing results of a quantitative analysis of intensity of each vimentin by 1D-Western blotting using an anti-vimentin antibody.
Fig. 29 is a drawing showing a chemoresistance of a glioma cell line U373 with its expression upregulated by fragmentation of vimentin.
Fig. 30 is a drawing showing a cell localization of vimentin in glioma cell U373 and A172.
Fig. 31A is a drawing showing N-terminal fragments of vimentin translocated into the cellular nuclear of glioma cell A172.
Fig. 31B is a drawing showing N-terminal fragments of vimentin localized in the nuclear of a malignant LOH- and LOH⁺ glioma cell, respectively.
Fig. 31C is a drawing showing N-terminal fragments of vimentin shifted to the nuclear of glioma cell line.
Fig. 32A is a drawing showing ephrin receptor A4 (EphA4) mRNA upregulated by ephrin A1.
Fig. 32B is a drawing showing EphA4 mRNA upregulated by an N-terminal vimentin fragment.
Fig. 33 is a drawing showing upregulated expression of Eand serotonin, respectively, for chemoresistance of glioma cells U373.
Fig. 34 is a network chart showing a network relating to a HIF signal.
Fig. 35 is another network chart showing a network relating to a HIF signal.
Fig. 36 is drawings showing states of the growth of a tongue cancer cells (SQUU-A cell and SQUU-B cell) in the course of time by a 3DCC system.
Fig. 37 is a drawing showing states of the growth of tongue cancer cells in the course of time by a 3DCC system.
Fig. 38 is a drawing showing the rate of the volume of a SQUU-B cell cluster with respect to SQUU-A cells by a 3DCC system.
Fig. 39 is drawings showing states of the HIF-1 expression in SQUU-B cells with a GFP reporter gene (HIF response element: HRE) incorporated therein.
Fig. 40 is drawings showing EphA4 , Cdc42 and vimentin with the expression upregulated in the AO/AOA tissue and the glioma cell line of 1p/19q LOH⁺/LOH⁻ patients, respectively.
Fig. 41 is drawings showing states of phosphorylation and fragmentation of vimentin in the AO/AOA tissue and the glioma cell line of 1p/19q LOH⁺/LOH⁻ patients by activation of PAK1 and calpain, respectively.
Fig. 42 is drawings showing results of an analysis of the phosphorylation of a protein in the AO/AOA tissue of a 1p/19q LOH⁺/LOH⁻ patient.
Fig. 43 is drawings showing results of an analysis of mass spectrometry and an analysis of an N-terminal amino acid sequence in a GBM tissue and an AO/AOA tissue, respectively.
Fig. 44 is a table showing sites of phosphorylation identified on vimentin.
Fig. 45 is a table showing N-terminal amino acid sequences of vimentin in the GBM tissue and the AO/AOA tissue by Edman degradation, respectively.
Fig. 46 is drawings showing N-terminal vimentin fragments translocated into the cellular nuclear of 1p/19q LOH⁻AO/AOA glioma cell.
Fig. 47 is drawings showing N-terminal vimentin fragments accumulated in the nuclear of the glioma tissue associated with a decreased overall survival and progression-free survival periods of AO/AOA patients.
Fig. 48 is drawing showing information of an N-terminal vimentin fragment which may work as a candidate of a transcription factor for EphA4.

### MODES FOR CARRYING OUT THE INVENTION

The present invention relates to a generation method for the generation of a data set for an integrated proteomic analysis, a method for an integrated proteomic analysis using the data set for the integrated proteomic analysis generated by the generation method therefor, and a method of the identification of a causative protein using the same.

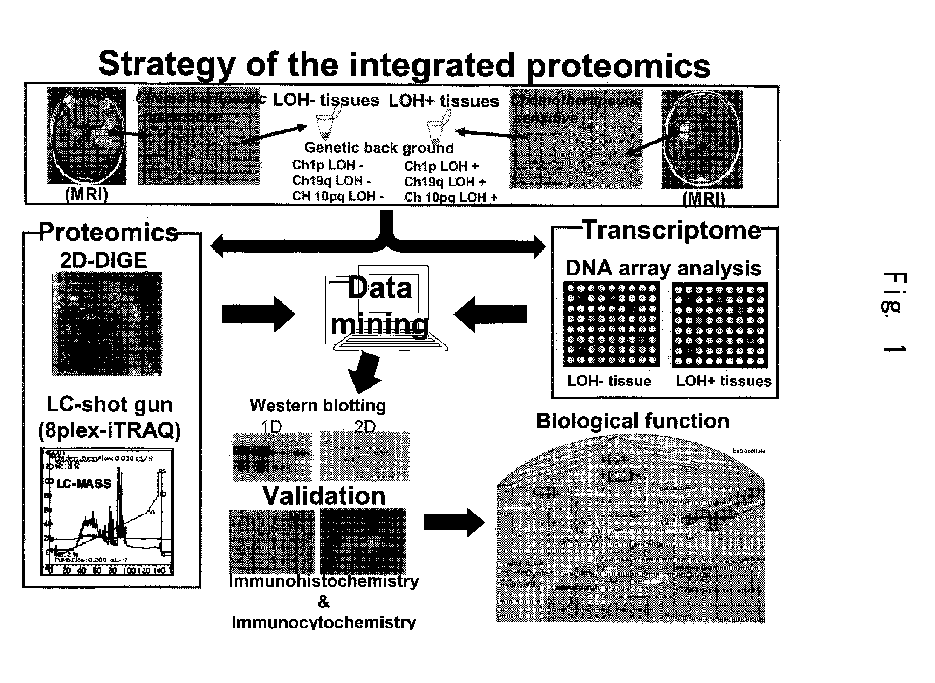

A brief description will be made regarding the generation method for the generation of the data set for the integrated proteomic analysis according to the present invention with reference to Fig. 1. As shown in the drawing, the generation method therefor comprises the generation of the data set for the integrated proteomic analysis to be applicable to a proteomic analysis on the basis of a set of comprehensive data of amounts of variations in expression of proteins and a set of comprehensive data of amounts of variations in expression of genes between two different sample groups.

The term "sample" used herein is intended to mean a cell extract from, for example, cells of animals, plants, microorganisms, etc., including, but being not limited to, DNAs and gene transcription products, proteins expressed from genes, post-translational proteins, and so on.

The terms "two different sample groups" used herein are intended to mean two samples obtainable from individuals or organisms having different states, regardless of species of animals, plants, microorganisms, etc. The individual or organism originating the sample groups may belong to a single species or plural species.

The terms "set of comprehensive data of amounts of variations in expression of proteins" and related terms are intended to mean a set of data of amounts of variations in quantitated values of plural proteins contained in a sample group obtained by a computation based on amounts of variations in quantitated values of plural proteins in another sample group, each of the amounts of variations in quantitated values of plural proteins being obtained by the integrated proteomic analyses of the two different sample groups using a combination of high performance liquid chromatography (HPLC) and two-dimensional electrophoresis with mass spectrometry (MS) and DNA microarrays, etc.

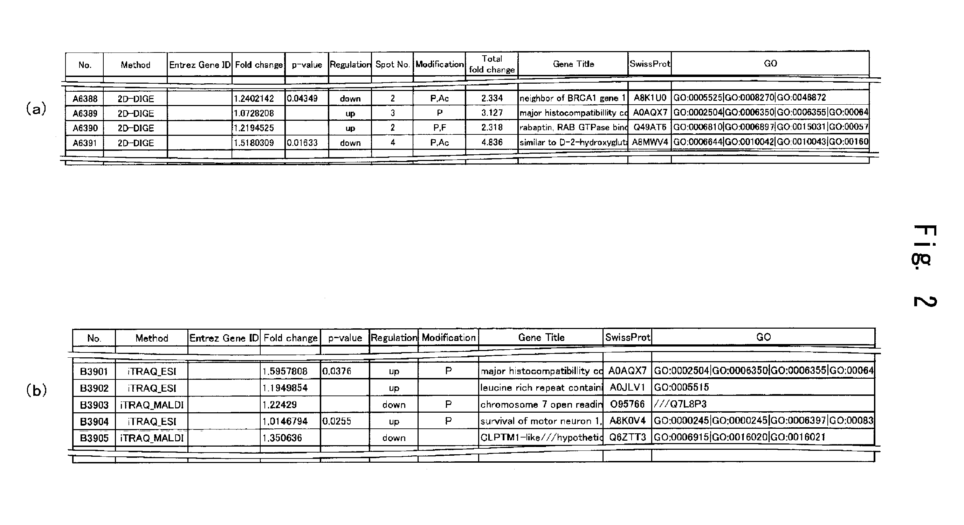

Specifically, Fig. 2(a) shows a data set generated by analyzing the two different samples by fluoresceinated two-dimensional difference gel electrophoresis (2D-DIGE), quantitating each spot by mass spectrometry, and computing amounts of variations of each spot.

Fig. 2(b) shows a data set generated by treating the two different sample groups with iTRAQ(R) reagents (Applied Biosystems), separating and quantitating each of the proteins by nanoLC/MS/MS, followed by computing an amount of a variation in expression of an individual protein. As shown in the drawing, the term "Method" is intended to mean an method of analysis; the terms "Entrez Gene ID" to mean a common identity number as will be described hereinafter; the terms "Fold change" to mean an expression variation amount; the term "p-value" to mean a p-value indicating results of a significance test of the expression variation amount; the term "Regulation" to mean a tendency of a variation (upregulation or downregulation) of a protein; the terms "Spot No." to mean numbers of spots identified by the 2D-DIGE method; the term "Modification" to mean a kind of post-translational modifications predicted; the terms "Total fold change" to mean a total sum of the expression variation amounts of spots identified as the identical protein by the 2D-DIGE method although the modification is different in quality; the terms "Gene title" to mean a gene name and a definition of a gene function; the term "SwissProt" to mean an accession number (identity number) of the Swiss-Prot as a first database of proteins; and the term "GO" to mean a gene ontology classification. The Swiss-Prot is a database of amino acid sequences provided jointly by Swiss Institute of Bioinformatics (SIB) and European Bioinformatics Institute (EBI). As abbreviations described in the column "Modification", the abbreviation "p" means phosphorylation, "Ac" means acetylation, and "F" means fragmentation.

As described above, the comprehensive data set of the protein expression variation amounts contains data of post-translational modifications of the proteins identified so that the integrated proteomic analysis using the data set for the integrated proteomic analysis, as will be in detail described hereinafter, may provide the results of analysis reflecting the post-translational modifications of the proteins in addition to variations in expression of genes and proteins as well.

In a working embodiment of the present invention, generally, the comprehensive data set of the gene expression variation amounts may be obtained by the DNA microarray technique, and the comprehensive data set of the protein expression variation amounts may be obtained by the 2D-DIGE method or LC/MS/MS method. It is to be noted, however, that they are not limited to them and any proteomic analysis techniques may be used as long as they may be applied to integrated proteomic analysis and provide a comprehensive data set of protein expression variation amounts between the two different sample groups, including, for example, various transcriptomic analysis techniques and proteomic analysis techniques.

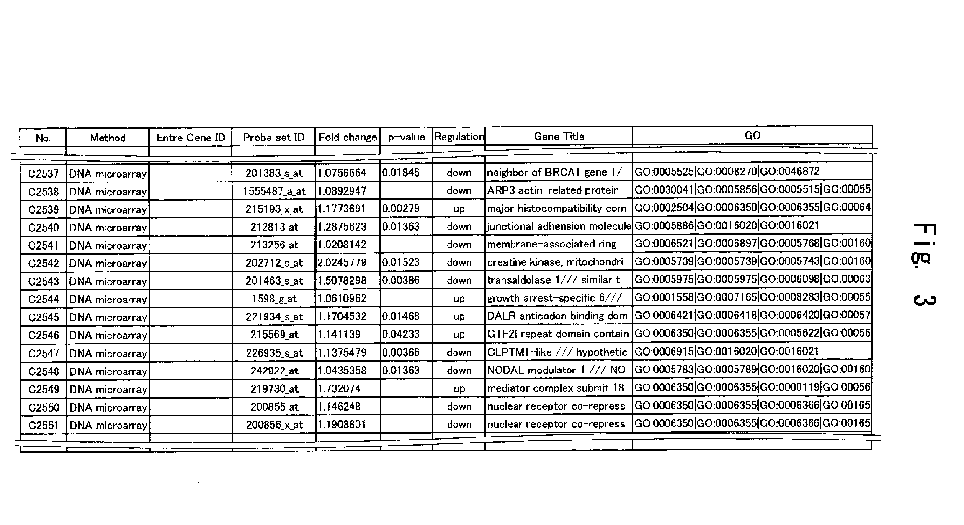

The comprehensive data set of the gene expression variation amounts is intended herein to mean a data set of amounts of variations in expression of genes, which may be obtained by analyzing the two different sample groups by way of analysis techniques, e.g., DNA microarrays, and computing the amounts of variations in expression levels of plural genes contained in one of the two different sample on the basis of the amounts of variations in expression levels of plural proteins contained in the other sample group. Specifically, the data set are shown partially in Fig. 3. In the drawing, the term "Method" is intended to mean a method of analysis; the terms "Entrez Gene ID" to mean a common identity number as will be described hereinafter; the terms "Probe set ID" to mean an identity number of a probe assigned to the DNA microarray; the terms "Fold change" to mean an amount of a variation in expression; the terms "Probe set ID" to mean an identity number of a probe assigned to the DNA microarray; the term "p-value" to mean a p-value indicating the result of a significance test of an expression variation amount; the term "Regulation" to mean a direction of variation (upregulation or downregulation) of a protein; the terms "Gene title" to mean a gene name and a definition of a gene function; and the term "GO" to mean a gene ontology classification.

In accordance with the present invention, the data set to be used for an analysis of proteins functions by way of the integrated proteomic analysis may be generated on the basis of a set of comprehensive data of amounts of variations in expression levels of proteins (hereinafter also referred to as "protein expression variation amount data set") between a group of two different samples and a set of comprehensive data of amounts of variations in expression levels of genes (hereinafter also referred to as "gene expression variation amount data set") therebetween.

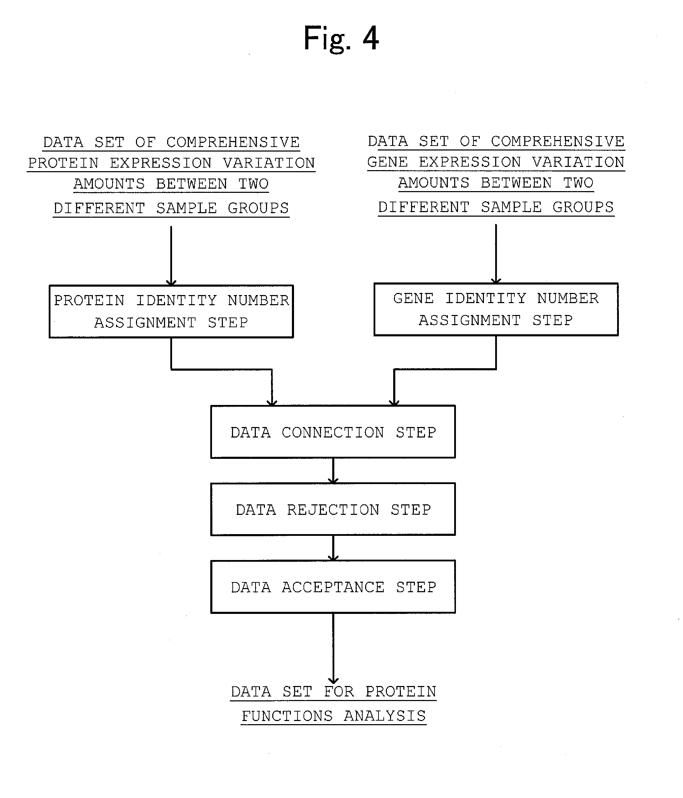

More specifically, as shown in Fig. 4, the present invention is characterized by the generation of the data set for the integrated proteomic analysis to be used for an analysis of functions of proteins via the protein identity number assignment step, the gene identity number assignment step, the data connection step, the data rejection step, and the data acceptance step.

The protein identity number assignment step comprises a process in which data of the expression variation amount of an individual protein constituting the comprehensive protein expression variation amount data set is provided with a common protein identity number that is linked to an identity number of the corresponding protein in a first database and an identity number of a gene encoding the protein in a second database.

The first database of proteins as used herein may be interpreted as a database for identifying a protein, which is prestored, for example, by mass spectrometry devices to be used for 2D-DIGE analyses or mass spectrometry devices attached to LC/MS. In particular, recent mass spectrometry devices and computers for use with data analyses are installed in many cases with the database so as to be capable of providing IDs of generally well known Swiss-Prot, etc. As shown in Fig. 1, too, the protein expression variation amount data of an individual protein is provided with a Swiss-Prot ID as an identity number in the first database.

The identity number in the second database of the genes encoding the proteins may comprise an identity number assigned by GenBank provided by U.S. National Center for Biotechnology Information (NCBI), EMBL provided by European Molecular Biology Laboratory, or DDBJ provided by National Institute of Genetics in Japan, or an identity number of an integrated database Entrez operated by U.S. National Library of Medicine (NLM) and NCBI with the object to perform the strategy of integrally managing those database as described above while deleting redundancy. As will be described hereinafter, in a working embodiment of the present invention, Entrez identity numbers may be provided as the identity numbers in the second database, although shown in blank spaces in Fig. 2. It is to be noted herein that the Entrez identity numbers assigned to the comprehensive data set of the protein expression variation amounts, shown in Fig. 2, may also function as a common identity number as will be described next.

The common identity number is an identity number that is linked to the identity number in the first database and the identity number in the second database.

For instance, in the event that the Swiss-Prot identity number is used as the identity number of the first database and the DDJB identity number is used as the identity number of the second database, both of these identity numbers are not linked directly to each other. It is difficult, accordingly, to review these data integrally although a certain gene corresponds to a certain protein in a particular gene-protein expression system. In a working example of the present invention, therefore, it can be made feasible to readily integrate the expression variation amount data of a certain gene with the expression variation amount data of a protein corresponding to the gene in an identical expression system by linking to the identity number of the first database and the identity number of the second database.

As the common identity number to be used for the present invention, there may be used any identity number as long as it can link the identity number of the first database and the identity number of the second database, and it is not limited to a particular one. As an example of such common identity number, an Entrez identity number may be used.

Next, a description will be made regarding the gene identity number assignment step. The gene identity number assignment step is involved in the step in which the gene expression variation amount data of an individual gene constituting the comprehensive gene expression variation amount data set is provided with a common identity number that is linked to an identity number of the gene in a third database and the identity number of the protein in the first database, which is expressed from the gene.

As the gene identity number of the third database, there may be used, for example, the "Probe set ID" assigned to a DNA microarray.

The identity number of the protein expressed from the corresponding gene may be interpreted as an identity number of the Swiss-Prot.

Moreover, as the common identity number with the identity number of the third database and the identity number of the first database linked thereto, there may be used, for example, the Entrez identity number, as in the protein identity number assignment step as described above.

The data connection step is involved in the step in which the connected data set constituted by the protein expression variation amount data of an individual protein and the gene expression variation amount data of an individual gene may be formed by connecting the comprehensive protein expression variation amount data set obtained in the above protein identity number assignment step to the comprehensive gene expression variation amount data set obtained in the above gene identity number assignment step.

Specifically, in the data connection step, data of an analysis obtained by implementing the integrated proteomic analysis method including, for example, 2D-DIGE, LC/MS, etc. is integrated with data of an analysis obtained by implementing the transcriptomic analysis method including, for example, the DNA microarray method. In other words, all the results of analyses consisting of the results of an analysis of proteins obtained by the iTRAQ method and the 2D-DIGE method and the results of an analysis of mRNAs obtained by the DNA microarray method are converted and integrated into the data set for the integrated proteomic analysis (integrated Protein Expression Analysis Chart; iPEACH), which is written by a text file type only in each one line on an identical format.

In a more specific description, the connected data set may be obtained by connecting the comprehensive protein expression variation amount data set obtained by various analysis methods to the comprehensive gene expression variation amount data set obtained by various analysis methods and reordering the expression variation amount data of the individual protein and the expression variation amount data of the individual gene in sequential order based on the common identity numbers.

The connected data set as obtained above is then treated in the data rejection step in which data having a p-value equal to or higher than a predetermined value is rejected for listing. The p-value may be obtained by a significance test of amounts of variations in protein expression or amounts of variations in gene expression between the two different sample groups among the respective expression variation amount data constituting the connected data set.

The connected data set obtained in the data rejection step is then treated in the data acceptance step. In the data acceptance step, either of the data that is provided with an identical identity number of the protein expression variation amount data and the data that is provided with the identity number of the gene expression variation amount data, which is identical to the identity number of the protein expression amount data, is accepted on the basis of a predetermined condition, thereby forming a data set for analyzing functions of proteins.

The terms "predetermined condition" as used herein are intended to mean the condition, for instance, which directly reflects an in vivo behavior of a protein, when the identical common identity number is assigned to the protein expression variation amount data and the gene expression variation amount data. In other words, it is preferred to accept the protein expression variation amount data as a predetermined condition. It is also preferred as a notable molecule to accept the one that can be expected to accept the molecule as a notable molecule, among the protein expression variation amount data, which undergoes modifications of molecular functions by post-translational modifications.

By arranging the configuration of the data, the present invention can yield the results of an integrated analysis of proteins conforming to the significance of the variations in expression of proteins in actual cells.

As described above, the generation method for generating the integrated proteomic analysis data set according to the present invention enables the generation of the data set of the integrated proteomic analysis that can achieve more accurate and appropriate results of an analysis by taking the above steps into account and adding the expression variation amounts of proteins and genes integrally, when compared to results of an analysis obtained by conventionally analyzing amounts of variations in expression of proteins or genes solely and individually.

The following is a more specific description regarding the generation of the data set to be applied to an analysis of functions of proteins with reference to the accompanying drawings.

In a working embodiment of the present invention, the generation method for generating the integrated proteomic analysis data set is implemented, first, by the protein identity number assignment step in which the expression variation amount data of an individual protein constituting the protein expression variation amount data set as shown in Fig. 2 is provided with a common protein identity number that links to an identity number of the protein in the first database, e.g., a Swiss-Prot identity number, and an identity number of the gene encoding the corresponding protein in the second database, e.g., Entrez Gene ID.

In this working embodiment of the present invention, the protein expression variation amount data set obtained by implementing two kinds of analysis methods, i.e., 2D-DIGE and LC/MS (iTRAQ) are combined with each other and then provided with common identity numbers.

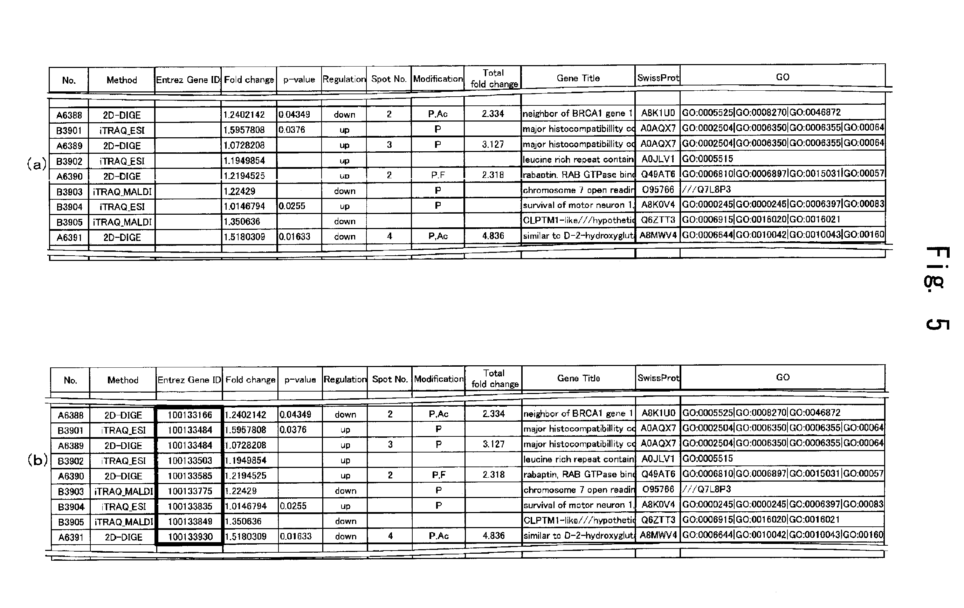

More specifically, the above two protein expression variation amount data sets as shown in Figs. 2(a) and 2(b) are connected together to form one protein expression variation amount data set as shown in Fig. 5(a). The two protein expression variation amount data sets as shown in Figs. 2(a) and 2(b), respectively, may be provided in advance with information (Spot No.) relating to variations of in vivo molecular states of the identical proteins obtained by the 2D-DIGE method and information (Modification) relating to post-translational modifications obtained from the results of an analysis by mass spectrometry. These results are reflected in the integrated data.

The protein expression variation amount data of the individual protein constituting the protein expression variation amount data set is then provided with an identity number of the gene encoding the individual protein in the second database, that is, Entrez Gene ID, as shown by a black box with a bold line in Fig. 5(b).

The Entrez Gene ID may be provided on the basis of "Gene Title" or "Swiss-Prot ID" provided by protein identification databases installed in mass spectrometers used for the identification of proteins found in 2D-DIGE or for iTRAQ LC shot-gun analysis.

The Entrez Gene IDs may not be provided automatically to the protein list, thus, it is preferred to have an automatic system to provide the Entrez Gene IDs computationally through internet, etc. directly from the Entrez Gene database because enormous numbers of proteins are normally identified in the differential proteomics such as 2D-DIGE and LC-shotguns, etc. in some cases, up to several tens of thousands.

As a program for a user's interface to be used in this connection, there may be used a software having the above functions which is installed in a computer of each user, or a program having the above functions which is stored in a server on an internet, thereby allowing each user to use it through an internet using a browser software pre-installed in the computer of each user.

Thereafter, the gene identity number assignment step may be performed by providing the gene expression variation amount of the individual gene constituting the gene expression variation amount data set as shown in Fig. 3 with a common gene identity number assigned by linking to the identity number of the gene in the third database, e.g., the Probe set ID of the DNA microarray, and the identity number of the protein expressed by the gene in the first database, e.g., the identity number of the Swiss-Prot.

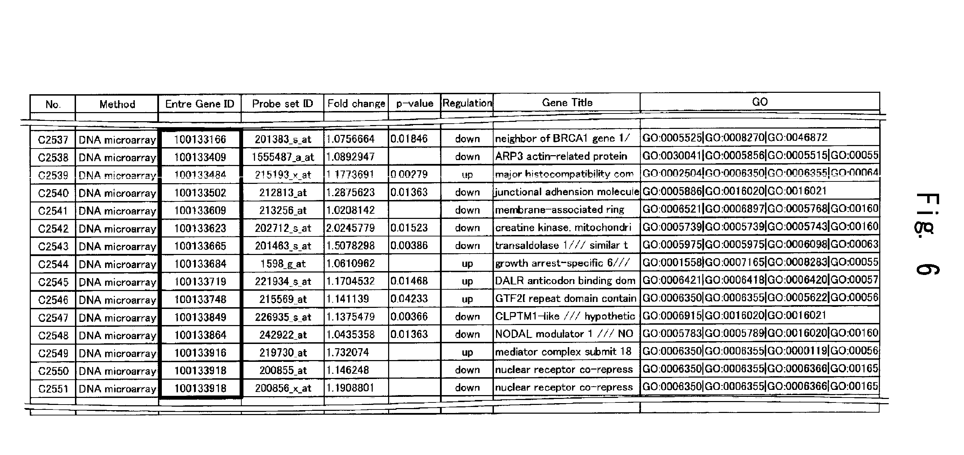

More specifically, in a working embodiment of the present invention, the gene expression variation amount data of the individual gene constituting the gene expression variation amount data set as shown in Fig. 3 is provided with the Entrez Gene ID as shown by a black box in a bold line in Fig. 6.

The Entrez Gene ID may be provided on the basis of the Probe set ID of the DNA microarray.

It is preferred to provide the Entrez Gene IDs automatically by referring to the Entrez Gene database using computers through internet, etc. because numbers of genes identified by the DNA microarray may amount to an enormous number of genes, in some cases, ranging from several thousands to several tens of thousands, although the Entrez Gene IDs may be provided manually, By providing the Entrez Gene IDs in the manner as described above, the enormous numbers of the gene expression variation amount data of the individual genes may be provided with the identity numbers of the second database while dramatically reducing input errors and labors.

As a program for a user's interface to be used in this connection, there may be used a software having the above functions which is installed in a computer of each user, or a program having the above functions which is stored in a server on an internet, thereby allowing each user to use it through an internet using a browser software pre-installed in the computer of each user.

Then, the comprehensive protein expression variation amount data set (see Fig. 5(b)) obtained in the protein identity number assignment step is connected to the comprehensive gene expression variation amount data set (see Fig. 6) obtained in the gene identity number assignment step, thereby forming the connected data set.

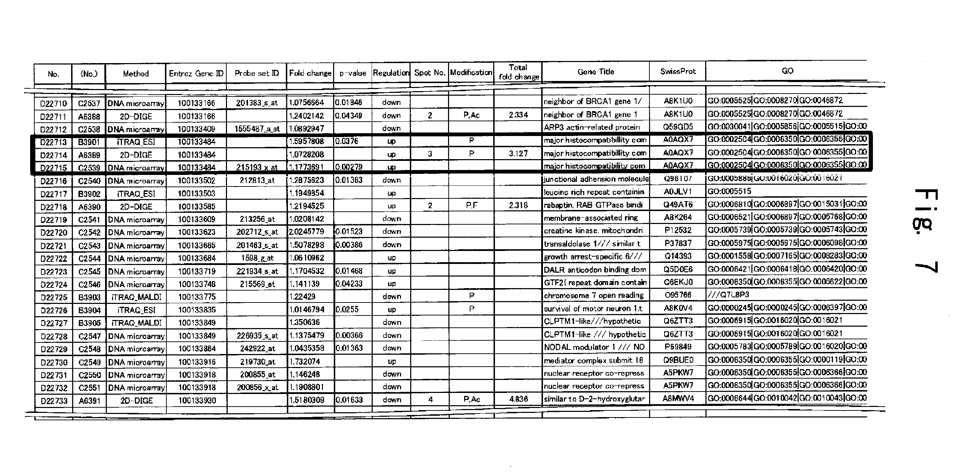

More specifically, as shown in Fig. 7, the connected data set may be formed by reordering the protein expression variation amount data of the individual protein and the gene expression variation amount data for each gene in sequential order of the common identity numbers.

By the reordering operation, the analysis data obtained by the integrated proteomic analysis such as 2D-DIGE and LC/MS, etc. may be integrated with the analysis data obtained by the transcriptomic analysis such as the DNA microarray method, etc., thereby generating the connected data set.

As the connected data set is reordered with the common identity numbers, it is possible to visually observe an expression system, in which a certain protein is expressed from a certain gene, in the form of a group of data categorized according to the analysis method. This is very useful in order to make a grasp of the expression variation amounts of genes and proteins.

The connected data set is then treated in the data rejection step. In the data rejection step, each of the expression variation amount data constituting the connected data set having the p-value equal to or larger than a predetermined value is rejected from listing.

The predetermined value that becomes a threshold of the p-value may be set to, for example, 0.05, although it is not limited to this value and it may be set to a lower value, for example, 0.01 or 0.005 in accordance with a desired accuracy. It is to be noted herein, however, that in the event that the predetermined value would be set to a value too low, numbers of the expression variation amount data to be rejected would be increased, thereby reducing an accuracy of analysis results. Attention has to be paid, therefore, in order to obtain a right analysis result. In this working embodiment of the present invention, the expression variation amount data having the p-value equal to or larger than the predetermined value is determined to be rejected, however, the expression variation amount data having an F-value equal to or lower than a predetermined value obtainable by an analysis of variance (ANOVA) may be also determined to be rejected.

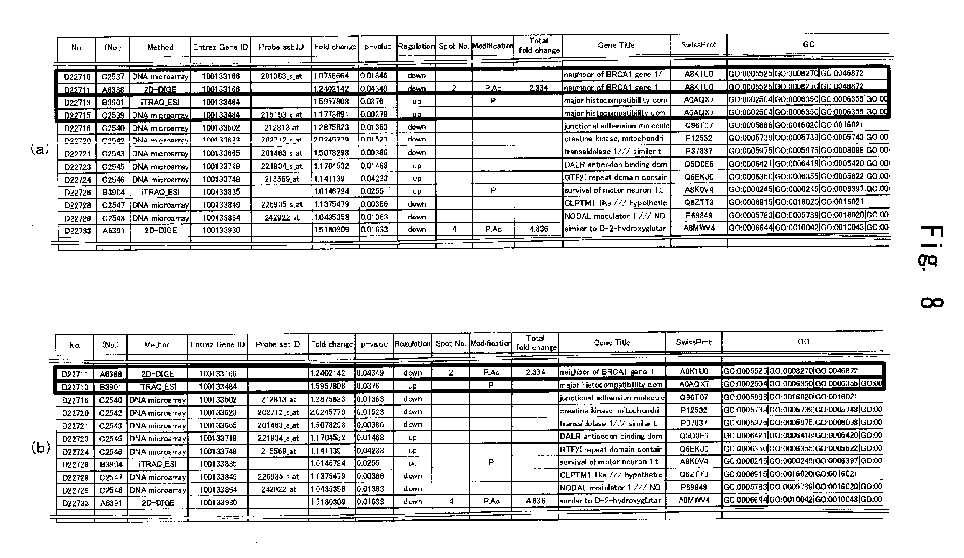

By implementing the data rejection step, the connected data set may be obtained in the form of a data set as shown in Fig. 8(a). From the data set, the expression variation amount data having the p-value equal to or larger than the predetermined value are deleted.

The connected data set is then treated in the data acceptance step after the data rejection step. In the data acceptance step, either of the protein expression variation amount data or the gene expression variation amount data, which is provided with the identical common identity number, is accepted as a set of data to be used for an integrated proteomic analysis to analyze functions of proteins.

In other words, as shown in Fig. 8(a), in the case where the protein expression variation amount data and the gene expression variation amount data, which are provided each with an identical common identity number, the protein expression variation amount data may be accepted as data constituting the connected data set, for instance, on the condition that the data is the protein expression variation amount data.

By operating the data acceptance step, the data set for use with an analysis of protein functions may be formed as shown by a black box in Fig. 8(b), in which the data provided in duplicate with the identical common identity number has already been deleted.

The integrated proteomic analysis data set generated in the above manner may be used, for example, for an GO analysis or a network analysis including, for example, a path analysis relating to in vivo signaling, a metabolic routine analysis, and so on, thereby allowing a functional analysis to be performed with a high efficiency and accuracy.

In other words, the integrated proteomic analysis data set according to the present invention is configured in such a manner that the whole picture of in vivo variations in expression of proteins can be understood effectively and readily by integrating the results of various gene expression analysis and protein expression analysis without spoiling their significance and detailed information (raw data) while adding information on post-translational modifications of proteins. Therefore, an analysis of in vivo molecular networks and a functional clarification of new molecules associated therewith can be performed by the use of the data set for the integrated proteomic analysis with a high accuracy.

It is to be noted herein, however, that, even if a particular protein molecule could be identified by an analysis of the integrated proteomic analysis data set according to the present invention as a highly reliable data on a program, it still remains questionable as to what highly reliable biological information could be obtained therefrom if it would be practically applied to an actual in vivo sample.

At this end, in accordance with the present invention, it is needed to construct a proteomics database using various tissue and cell samples associated with actual diseases and review as to whether a proteomics of pathological conditions can be actually developed by retrieving and identifying a pathologic marker that may function as a target for treatment of diseases with medicine or an intermolecular abnormal signaling network as a target for development of new medicines.

In order to retrieve the pathologic marker or the intermolecular abnormal signaling network, however, it is considered that such a retrieval may be performed for the first time by integrally and comprehensively assessing various results of analyses including, for example, genome analysis, mRNA expression analysis, protein expression analysis, specific post-translational modification and interaction analysis, and so on. As described above, however, as these methodologies are originally established on an individual basis, the languages, formats and display concepts of the output data used in these methodologies are originally different from each other, it has so far been difficult to merge these data and assess them in an integral fashion. In other words, even if a large number of information on networks of proteins indicating an abnormal activity of pathological tissues or cells or interactional functions of intercellular proteins could be analyzed individually by different concepts using plural devices, it has so far been very difficult to connect the information to one another and interpret it in an organic fashion. In the present invention, reviews has now been made as to an in vivo applicability of the integrated mining analysis program, which is useful to efficiently perform an analysis of pathologically associated molecules, to an analysis of mechanisms of functions of molecules in tumor tissues or cells and a retrieval of biomarkers associated therewith.

In accordance with the present invention, causative proteins or signaling paths of pathological conditions, etc., which are regulated in an abnormal way, may be specifically extracted from samples collected from brain tumor tissues of patients with brain tumor by integrally mining all information obtained by implementing the integrated proteomic analysis using the iTRAQ method and the 2D-DIGE method as well as the transcriptomic analysis using the DNA microarray method in a united fashion. More specifically, for instance, all of quantitative information of the expression of a group of specifically varying molecules obtained by a concurrent analysis by various differential analyses of the identical samples collected from the brain tumor tissues thereof are integrated to extract a new molecular signaling network involved in a chemoresistance to anticancer agents, followed by implementing a series of analyses to retrieve and identify the proteins that may be considered to be associated with the brain tumor and verifying whether the identified proteins are actually involved in the pathology of diseases.

This verification is aimed at solving the following matters relating to the analysis methodology using the integrated mining according to the present invention: to clarify an involvement in chemoresistance of, for instance, modes of variations in expression or changes of structures of a group of molecules including post-translationally modified molecules, the group of molecules being associated with chemoresistance to anticancer agents, by extracting an activated signaling molecular cascade involving in the chemoresistance in cancer tissues or cells, that is, to elucidate a part of mechanisms of the chemoresistance to tumor; and to verify the possibility of constructing a database of basic information for developing markers for clinical use to diagnose a pathological treatment plan and prognostic values in a more appropriate way as well as medicines effective in clinical treatment.

In this description, the present invention has been described by taking the sample collected from the brain tumor tissues of a patient with brain tumor as an example. It can be noted, however, that this example has been illustratively taken solely for brevity of explanation and, as a matter of course, the present invention is not intended to be limited at all to the brain tumor. Anything in the medical field as well as in any other field can be interpreted as being encompassed within the scope of this invention as long as it does not depart from the object of the present invention.

The following is a specific description of the integrated mining method using a brain tumor sample. The brain tumor sample was collected from brain tumor tissues removed from a patient with AO/AOA (anaplastic oligodendroglioma/astrosystoma) which is the only one that can demonstrate sensitivity to chemotherapy against malignant glioma. At the present time, there is no diagnostic marker that can precisely predict a prognosis of the AO/AOA so that attention has recently been paid as a significant problem to be tackled promptly for development of a marker for the sensitivity to chemotherapy of a patient at an early stage or a target for chemotherapy treatment, etc. Although there is only one report on a relevance of a loss of heterozygosity (LOH) at chromosome locus 1p/19q of the AO/AOA with the chemosensitivity, no information on any specific gene worthy of explanation of details of a causative relationship with the chemosensitivity has been reported at all. One of the reasons for no reports so far presented may be considered to be based on the possibility that the deletion on a genome might be considered as a translocation that is not necessarily related with a deletion of a gene or that another gene acting indirectly on a mechanism of the chemoresistance through a deleted gene group might be involved. Therefore, there may be considered to be limits that a decision of the detailed chemotherapic mechanism of the AO/AOA is drawn only at a genome or gene level.

The samples of the brain tumor tissues collected from AO/AOA patients are classified on the basis of a pathological check, a presence or absence of chromosomal abnormality, normality or abnormality, a difference in grades of malignancy, a presence or absence of sensitivity to anticancer agents, and so on. More specifically, for instance, the brain tumor samples are classified by the pathological check in two kinds, that is, a type having 1p/19q LOH (1p/19q LOH⁺) and a type having no 1p/19q LOH (1p/19q LOH⁻). Both of the LOH⁺ and LOH- have substantially the same pathological observations as they are generally known, however, it is known that they differ in sensitivity to anticancer agents and radiation such that the LOH⁺ patients are sensitive to anticancer agents and radiation and have a good prognosis yet the LOH⁻ patients are insensitive to anticancer agents and have a poor prognosis.

Thereafter, proteins and mRNAs are extracted each from the LOH⁺ and LOH⁻ samples. The resulting proteins are subjected at the same time to two kinds of proteomic analyses, that is, the 2D-DIGE method based on a two-dimensional electrophoresis method and the iTRAQ method based on a LC/MS using no electrophoresis. A group of molecules having differences at a protein level are then identified by implementing mass spectrometry to analyze variations of expression of proteins and post-translationally modified proteins, etc. The mRNAs are subjected to transcriptomic analyses using DNA microarrays to analyze variations of expression of the mRNAs.

The data obtained by the comprehensive analysis by the proteomic analysis such as the 2D-DIGE method and the iTRAQ method as well as the transcriptomic analysis are then arranged and integrated using iPEACH (integrated Protein Expression Analysis Chart) which is an application for analyzing the integrated proteomic analysis data set according to the present invention. This may perform the GO (gene ontology) analysis and the intramolecular network analysis using a KeyMolnet software (see Fig. 8).

The GO analysis and the intramolecular network analysis using the iPEACH may specify a significant set of data of the molecules varying in sensitivity to anticancer agents in the LOH⁺ and LOH- brain tumor tissues and consequently extract a signaling network involved in the sensitivity thereto in the brain tumor tissues. All the AO/AOA samples are verified for a group of molecules predicted to be associated with the molecular network using Western blotting and immunohistostaining. A similar verification may be performed for the LOH⁺ and LOH⁻ cultured glioma cells. Moreover, a biological activity may be measured using siRNAs, inhibitors and activators in an integrated fashion to perform tests for verification of the inhibition or activation of the chemosensitivity, thereby clarifying a molecular mechanism of the chemosensitivity in malignant glioma.

Next, a description will be made hereinafter regarding the iTRAQ method which is one of the proteomic analysis techniques for generating the integrated proteomic analysis data set. The iTRAQ method may be carried out first by labeling a mixture of two to four samples each with a different labeling agent and then by mixing the mixture together. Therefore, it may be implemented in a 4-plex or 8-plex way. As the labeling agents, there may be used the isobaric tag labeling agents that have each substantially the same composition but four to eight kinds of reporter ions. The samples may be mixed after the samples are fragmented by trypsin digestion and labeling the peptide-terminus of each sample with the above isobaric tag labeling agents. The resulting samples may be then fractioned by ion exchange, etc., followed by a reverse phase chromatography by a nanoLC and then a mass spectrometry using nanoESI-Qq-TOF and nanoMALDI-TOF-TOF. The four iTRAQ-labeled peptides fractioned in the same fraction may then be distinguished by molecular weights by the mass spectrometry and quantitated by differences of the molecular weights. At the same time, the proteins may be identified by the MS/MS spectrometry.

The 2D-DIGE method as another proteomic analysis method for generating the integrated proteomic analysis data set according to the present invention is basically designed in substantially the same manner as the iTRAQ method to analyze a mixture of samples with a confocal fluorescence gel scanner, the mixture of the samples which are sensitive to anticancer agents, insensitive thereto, and normal, containing proteins, i.e., three kinds of solubilized proteins, labeled with three fluorescence reagents, i.e., Cy2, Cy3, and Cy5, respectively, followed by mixing the proteins and implementing two-dimensional electrophoresis. A difference in expression of phosphorylated proteins may be analyzed by the same two-dimensional gel electrophoresis using a ProQ Diamond solution which stains a specific phosphorylated protein. The spot stained violet in color indicates the protein spot that specifically upregulates phosphorylation in the non-sensitive sample. The spot stained yellow in color indicates the protein that upregulates phosphorylation in the normal sample, that is, it is the protein spot group that downregulates phosphorylation in the non-sensitive sample.

The following is a brief description regarding the DNA microarray method which is used for the transcriptomic analysis for generating the integrated proteomic analysis data set according to the present invention. The mRNAs extracted from the same sample as used above for the proteomic analysis are subjected to a transcriptomic analysis using human cDNA oligochips, and the results of the transcriptomic analysis are compared to the results of the proteomic analysis implemented at the same time. It is found from the comparative results that numbers of molecules expressed by the mRNAs, which were detected in the identical sample, may greatly differ from expression patterns of the protein group identified by the proteomic analysis as specifically upregulating or downregulating the expression. As many of these molecules may often be found to be upregulated at a protein level but conversely downregulated at an mRNA level, this difference may be considered to reflect a time lag of expression and stability between the proteins and the mRNAs in cells. In other words, an event that an upregulation of expression of mRNA ends and starts downregulating as expression of the mRNA may reflect a phenomenon that the upregulation of the mRNA ends and decreases as it is reflected as proteins to a sufficient extent and the expression of the mRNA is induced and upregulated as the protein lacks as a result of clearance. More specifically, it is not considered to be strange that a situation is always caused to occur in cells during a certain period of time in which a certain protein is upregulated while the corresponding mRNA is downregulated or, conversely, a certain protein is downregulated while the mRNA is upregulated.

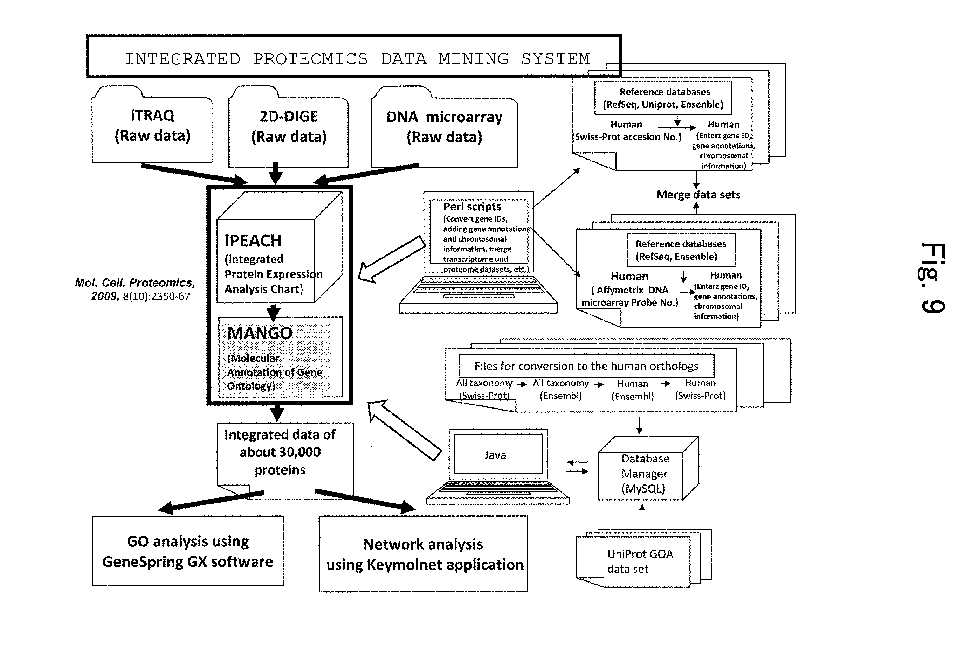

After the identification of the data set of all the molecules demonstrating some differences, data of expression levels of proteins and mRNAs, that is, the comprehensive data set of the protein expression variation amounts and the comprehensive data set of the expression variation amounts of mRNAs, are subjected to automatical data mining by the program (iPEACH) generated by arranging and integrating them by the generation method for the generation of the integrated proteomic analysis data set according to the present invention (see Fig. 9).

More specifically, this program (iPEACH) may convert a list into a text file type and read it on only one line in an identical format using Pearl Script to form a file in which accession numbers are integrated, the list containing all information on a group of molecules identified, respectively, by a gene analysis by DNA microarrays, which are confirmed as expressed in 1p/19q LOH⁺ and 1p/19q LOH- AO/AOA tissues, and a protein analysis by iTRAQ (ESI-Qq-TOF and MALDI-TOF-TOF) and 2D-DIGE (pH 3-11 and pH 4-7). This file is provided with an item 'gene description' (information on a definition and a function of a molecule), information on a chromosome locus, an analysis method used in integrated proteomics (i.e., DNA microarray, iTRAQ (MALDI-MS), iTRAQ (ESI-MS/MS), 2D-DIGE, etc.), item 'Gene Ontology annotation', the presence or absence and a frequency of post-translational modifications of a protein (information on a modified spot identified by the 2D-DIGE), and so on. In forming the integrated file, an algorithm is used for weighing individual molecules and assigning a priority order thereto among the data of the integrated file, thereby automatically listing the molecule at a higher rank as a notable molecule which can be confirmed as being post-translationally modified, too, even if it would be the identical protein. Further, this can generate the integrated file as a formatted text so as to be used directly for a molecular network analysis using, for example, GO analysis, KeyMolnet analysis, and so on by automatically assessing the molecules as significant to set a threshold and masking such that the molecules varying at values lower than the set threshold should be deleted from an object of analysis (JP 2010-81,525 A1). A unified file of an original analysis data as explained herein as a working example, may contain information on approximately 30,000 molecules as an object of analysis, and molecules may be extracted from those information, which are analyzed as statistically significant in respect of quantitation (see Fig. 8). It is to be noted herein that this iPEACH may immediately unify each data into a unified file so as to make the resulting file applicable to the GO analysis, pathway analyses and so on, thereby facilitating implementation of an extraction operation of a group of significant molecules.

Upon the generation of the integrated proteomic analysis data set (iPEACH), annotations (items to be listed in rows of a table) to be added to the iPEACH from raw data of the above analysis results may include, but be not limited to, items "fold change" and "p-value" each for the results of the iTRAQ (ESI) and iTRAQ (MALDI), as well as items "numbers of peptides identified", "numbers of post-translationally modified peptides (redundant)", "numbers of post-translational modifications (non-redundant)", "kinds of post-translational modifications", etc. each for the results of iTRAQ mass spectrometry (ESI) and iTRAQ mass spectrometry (MALDI). For the results of the 2D-DIGE method, annotations may include, but be not limited to, items "fold change", "p-value", "numbers of spots identified for each individual protein", "total fold change" (a total sum of variances of a total number of spots), "average fold change" (an average of variances of a total number of spots), "representative fold change" (a value of the spot having the largest variance among the total number of spots), "a kind of post-translational modifications of an analysis of the mass spectrometry (ESI) (non-redundant)" and so on. For the results of the DNA microarray, annotations may include, for example, items "fold change", "p-value", and so on.

As the annotations (the items to be illustrated in a row of the table) to be added to the iPEACH from NCBI (National Center for Biotechnology Information), there may be mentioned, for example, items "Entrez Gene ID", "Gene Symbol", "Map Location" (a location on a chromosome), "Description Type of Gene" (a name and definition of a gene), "OMIM-MIM number (ID)" (Online Mendelian Inheritance in Man), "OMIM-Clinical Synopsis (CS)", and OMIM-Title Word (TI)" (a name of OMIM record).

Further, the annotations (items to be illustrated in a row of the table) to be added to the iPEACH from KEGG (Kyoto Encyclopedia of Genes and Genomics) may include, for example, items "Entry" (an accession no. of the KEGG database; KEGG Ontology No.), "Gene Name" (a name of a gene on the KEGG database), "Definition" (a definition of a gene on the KEGG database), "KEGG Pathway" (information on KEGG Pathway based on the KO System), and "Class" (a hierarchical definition and classification of functions of a gene).

The annotations (the items to be described in rows of the table) to be added to the iPEACH from Ensemble may include, but be not limited to, items "Catalytic Activity of UniProt (cc line: comment)", "Cofactor of UniProt (cc line: comment)", "Disruption Phenotype of UniProt (cc line: comment)", "Enzyme Regulation of UniProt (cc line: comment)", "Developmental Stage of UniProt (cc line: comment)", "Interaction of UniProt (cc line: comment)", "Pathway (information on a post-translational modification) of UniProt (cc line: comment)", "Similarity of UniProt (cc line: comment)", "Subcellular Location of UniProt (cc line: comment)", "Carbohyd (information on a carbohydrate chain) of UniProt (FT line: footnote)", "Signal (information of a signaling peptide/Extent of a signal sequence) of UniProt (FT line: footnote)", "Transmem (information on a transmembrane region) of UniProt (FT line: footnote)", "Domain of UniProt (FT line: footnote)", "Ca bind (information on a calcium ion binding) of UniProt (FT line: footnote)", "Zn Fing (Zinc finger) of UniProt (FT line: footnote)", "DNA Bind (information on a DNA binding region) of UniProt (FT line: footnote)", "Motif (motif of biological interest) of UniProt (FT line: footnote)", "Binding (Binding site for any chemical group (co-enzyme, prosthetic group, etc.)) of UniProt (FT line: footnote)", "Mod Res (information 2 on a post-translational modification) of UniProt (FT line: footnote)", "Lipid (Covalent binding of a lipid moiety) of UniProt (FT line: footnote)", "Disulfid of UniProt (FT line: footnote)", "Crosslink of UniProt (FT line: footnote)", and the like.

Moreover, reviews were made of the narrowing-down of the activated signals by the GO analysis and the network analysis using the integrated proteomic analysis data as obtained above. More specifically, after implementation by the iPEACH analysis between the 1p/19q LOH⁺ and 1p/19q LOH- AO/AOA tissues, the analysis was implemented by focusing on the molecule group with their expression levels upregulating significantly in the 1p/19q LOH- tissues among the list of the molecule group assessed as the expression levels upregulating significantly. The results of this analysis revealed that EphA4 upregulated the variation in its mRNA expression levels to the greatest extent (14.9 times in the 1p/19q LOH⁻ tumor; p < 0.01) in variations in expression levels of mRNAs. On the other hand, a similar comparison in the variations in expression levels of the proteins revealed that vimentin varied its expression levels to the highest extent (in the 1p/19q LOH- tissues: 4.54 times higher than the 1p/19q LOH⁺ tissues; p < 0.01). It is further noted herein that six post-translationally modified vimentins were identified ranking the top thirty molecules that upregulated their expression levels. The upregulation of the expression levels of EphA4 and vimentin in the 1p/19q LOH⁻ glioma tissues resistant to anticancer agents implies that the molecular mechanism of resistance to anticancer agents may be involved somewhat in the signaling of a cell membrane surface receptor through EphA4, an upregulated expression of vimentin of an intercellular intermediate-sized filament, or an upregulation of its post-translational modifications.

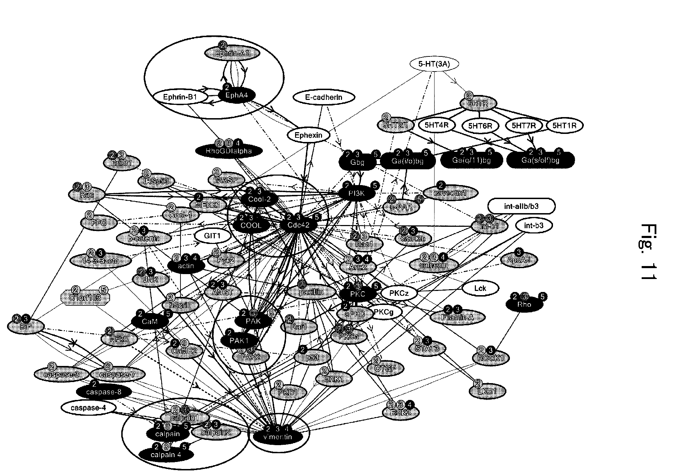

Thus, the interactional mechanism of these molecules may be presumed by implementing the GO analysis and the intercellular signal network analysis using the unified integrated file constructed by the iPEACH. The results of these analyses reveal that, among the GO terms annotated to the molecules as upregulating the specific expression in the 1p/19q LOH- AO/AOA tissues, the GO terms that are considered to be statistically associated significantly by the GO analysis may be involved in many cases in the regulation of the gene expression. These GO terms may include, for example, terms "Regulation of gene expression (p = 2.57E-08, Biological process)", "Regulation of transcription (p = 5.36E-07, Biological process)", "DNA binding (p = 2.86E-07, Molecular function)", and so on. Further, the above findings suggest the possibility of the signal activating the chemoresistance to anticancer agents specifically in in vivo cells regulating the transcriptional activity of a molecule associated with the chemoresistance. Moreover, as a network map is retrieved using KeyMolnet software by focusing particularly on EphA4 as a start point and vimentin as an end point in order to extract a network of associated activated molecules, it was found surprisingly that a group of the molecules located on the locus 1p/19q was extracted in a large number on a network. This result implies that an intramolecular network between EphA4 and vimentin, in particular, through Cdc42, p21 activated kinase (PAK1) involved in the phosphorylation of vimentin activating at downstream, calpain SS (calpain small subunit) relating to fragmentation of vimentin, and so on, may be important to the chemosensitivity in the glioma tissues (see Fig. 11).

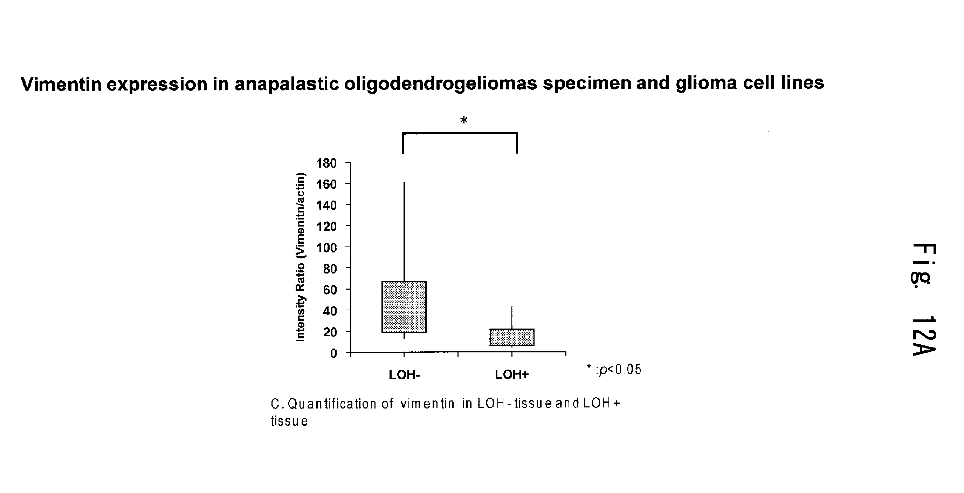

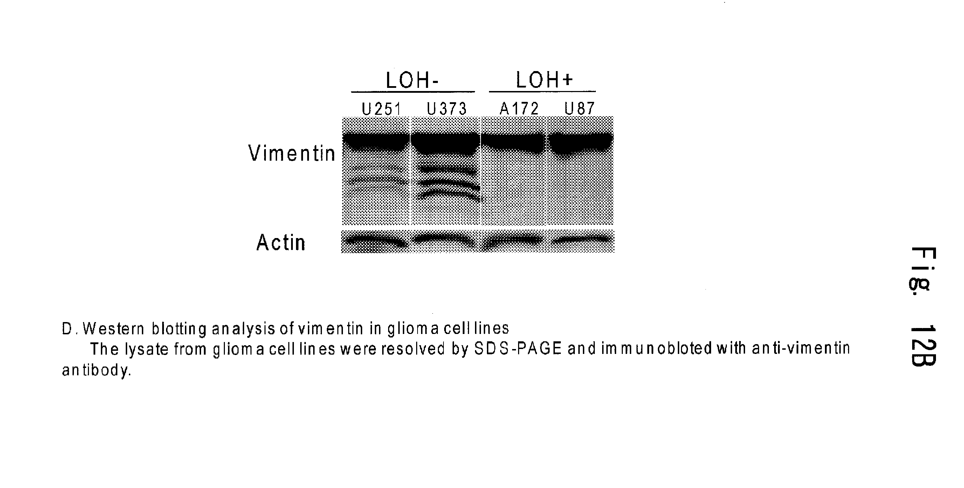

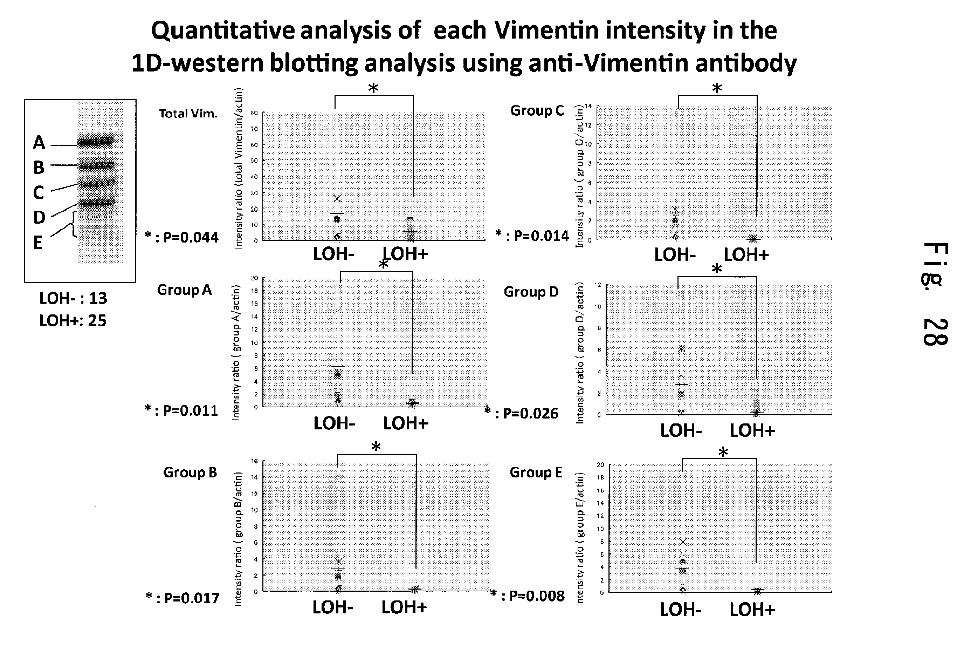

The molecular network extracted by an implementation of the GO analysis and the intermolecular signaling network analysis using the unified integrated file generated by the iPEACH was then subjected to biochemical verification. By paying attention to vimentin, ephrin receptor (Eph) and a group of regulating factors associated therewith, which are analyzed as the important molecules upregulating significantly by the GO analysis and the KeyMolnet analysis, their expression variation amounts were verified by the immunohistostaining and Western blotting methods. Western blotting was implemented using a monoclonal antibody to vimentin and ephrin receptor, respectively, and various cells and tissues: tissues derived from patients (a total of 36 specimens; LOH⁻: 18 specimens; LOH⁺: 18 specimens), U373 cells and U251 cells as cultured LOH⁻ glioma cells, and LOH⁺ U87MG cells and A172 cells. The results of the Western blotting analysis reveal that both of vimentin and ephrin receptor are upregulated significantly in both of the LOH- cells and tissues (see Figs. 12A, B). It was found that, in particular, the expression of vimentin was found upregulating remarkably in the LOH⁻ tissue and cells, and a significant upregulation of the expression in vimentin was recognized in characteristic vimentin-decomposed fragments (see Figs. 12A, 12B, 28).

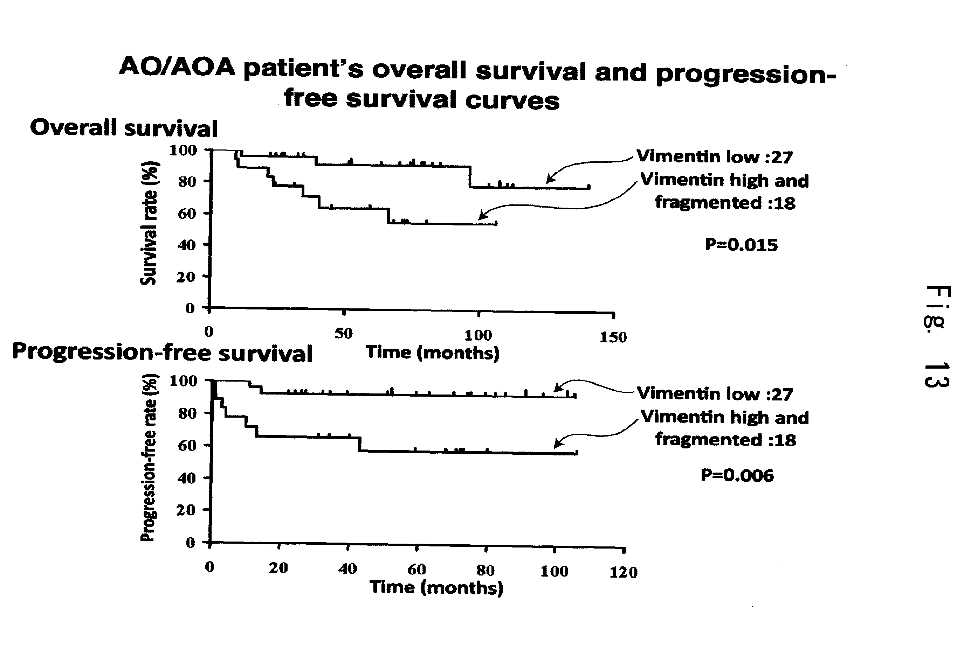

A statistical analysis was performed in order to investigate an influence of the presence or absence of a variation in expression levels of vimentin or E(EphA4) on the survival rate using samples collected from fifty-one 1p/19q LOH⁺/LOH⁻ AO/AOA patients. The results of this analysis revealed that the survival rate of a group of patients upregulating the expression of vimentin and E(EphA4) to a higher level was significantly lower than that of a group of patients downregulating the expression thereof. This suggests that a determination of the presence or absence of variations in expression levels of vimentin and ephrin receptor may work as an index for prognostication in the same manner as the presence or absence of the LOH. As a correlation was found between the upregulation of eight decomposed vimentin fragments and a decrease in the survival rate, it was further found that the upregulation and the decomposion of vimentin may have a relationship with a downregulation of the chemosensitivity in the AO/AOA (see Fig. 13).

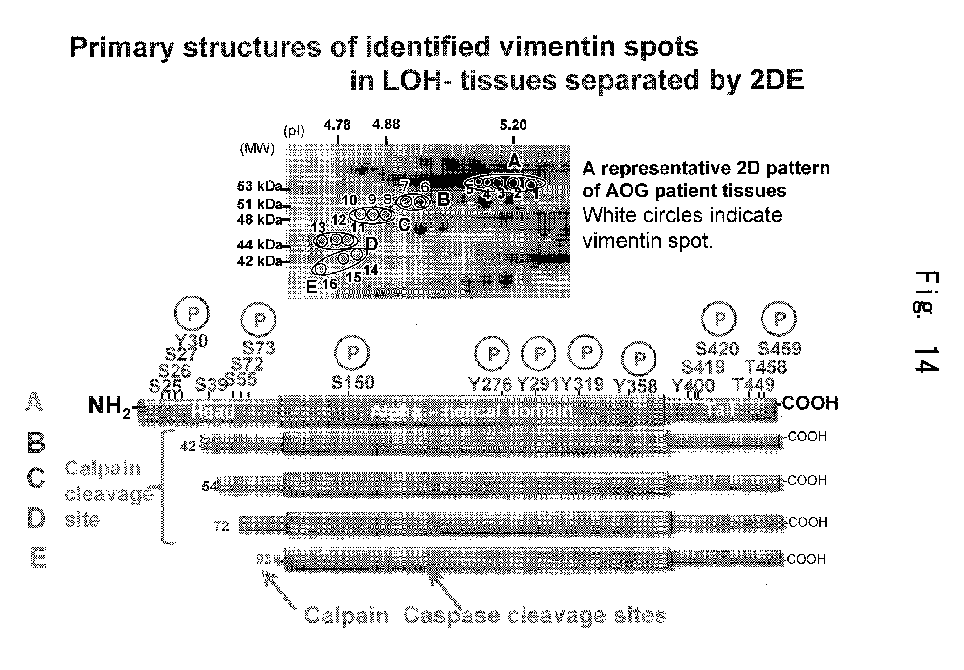

Moreover, the data of the vimentin spots were analyzed quantitatively and biologically in detail from the 2D-Western blotting data and the 2D-DIGE data. The detailed results revealed that a total number of sixteen spots of the modified vimentin were detected and eight spots out of the sixteen modified spots were found upregulating significantly in the LOH⁻ group. The spots were provided with a group number (A to E) in the order of descending molecular weights and a spot number (1 to 16) in the order of descending pIs. Primary structures of all the vimentin spots were analyzed by the mass spectrometry and the N-terminal sequence analysis. The results of detailed reviews revealed that all the vimentin spots preserved the C-terminal structures and their molecular weights were varied due to the cleavage of the N-terminal sites. Moreover, it was found that nineteen phosphorylated sites were identified and Tyr319, Tyr358, Tyr400 and Thr449 were new phosphorylated sites, while, surprisingly, the other three phosphorylated Ser sites were the sites that were phosphorylated by PAK1 and upregulated the expression significantly in the LOH⁻ group. More surprisingly, the N-terminal cleavage sites of groups B, C and D were all cleavage sites by calpain (Calpain SS being located at locus 19q) which upregulated significantly in LOH⁻ group (Fig. 14).

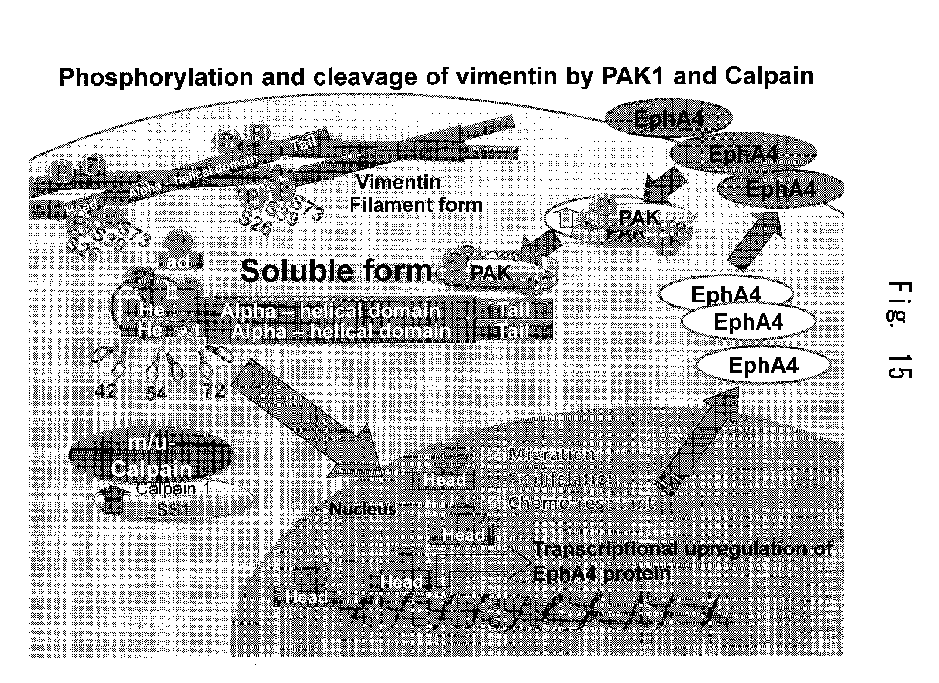

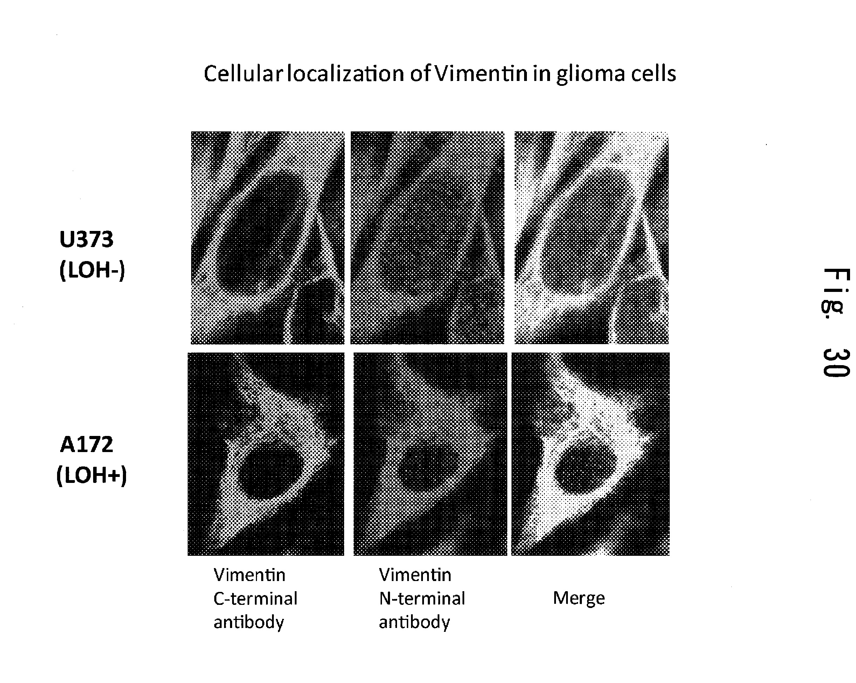

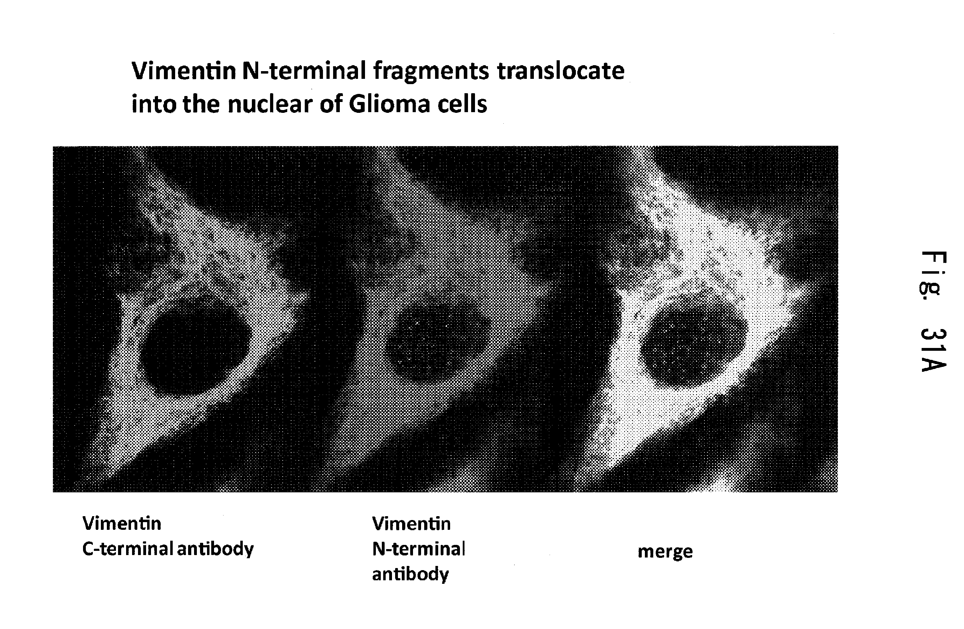

From the above facts, it may be imagined that a cascade consisting of ephrin → Cdc42 activation → PAK activation → vimentin phosphorylation → vimentin cleavage by calpain in respect of chemoresistance to anticancer agents may be activated in 1p/19q LOH⁻ glioma cells. An investigation of the intercellular localization of the cleaved vimentin fragments revealed that the fragmented N-terminal vimentins were translocated specifically into the cellular nuclei (Figs. 30, 31A-C). More surprisingly, it was found that the nuclear translocation of the fragmented N-terminal vimentins upregulated the expression of ephrin mRNA. From these findings, it may be considered that the glioma cells resistant to anticancer agents may possess a new activation loop through EphA4 activating the specific phosphorylation and modifications by cleavage of vimentin (Fig. 15).

Furthermore, in order to biologically verify the fact relationship, experiments for a biological verification of the activated molecular network using an anticancer agent were implemented. In other words, variations in sensitivity of the glioma cells to medicine, temozolomide (TMZ), which is a first choice medicine for malignant glioma, were analyzed using an inhibitor for the expression and activation of a group of the molecules involved in the cascade. [0127]

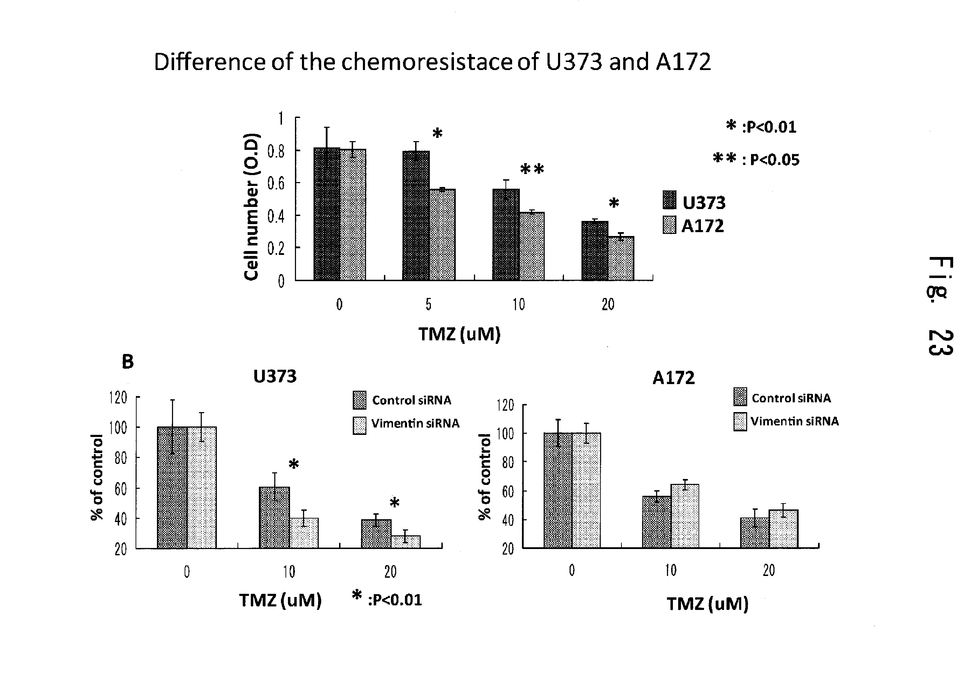

Reviews of the sensitivity of glioma cells U373 (LOH⁻) and A172 (LOH⁺) to TMZ were performed using vimentin siRNA. The results revealed that, although the U373 cells upregulated the expression of vimentin at a higher level compared with A172, the chemosensitivity to TMZ of the U373 cells was upregulated significantly by inhibiting the expression of vimentin by siRNA. On the other hand, the LOH+ cells, A172 cells, showed a relatively low expression level of vimentin and a high sensitivity to TMZ so that the effect of siRNA on the inhibition of the expression of vimentin was not found remarkable. In other words, it was found that the chemosensitivity to TMZ may be upregulated by downregulating the expression of vimentin in the glioma cells which were resistant to anticancer agents and whose vimentin activation loop was activated therein (see Fig. 23).

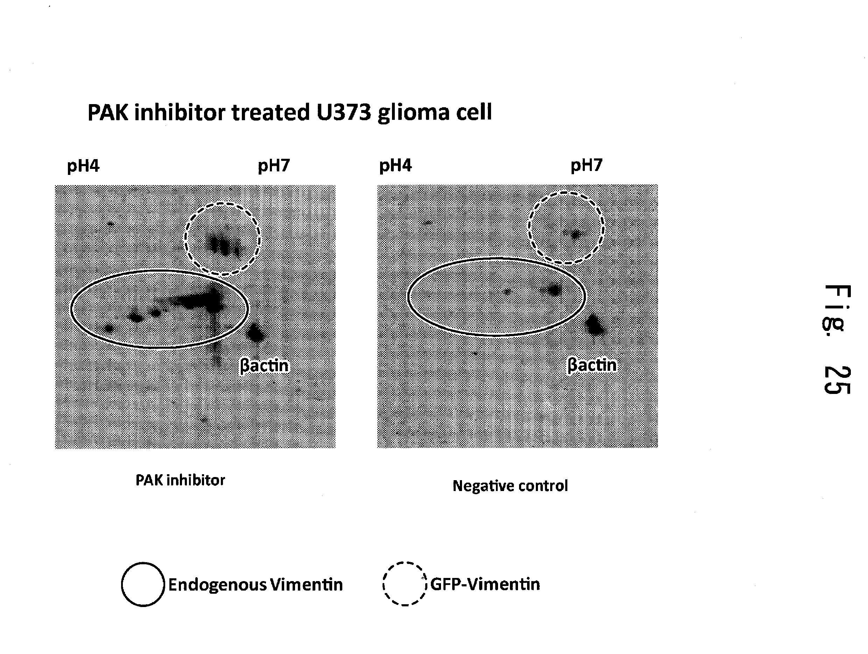

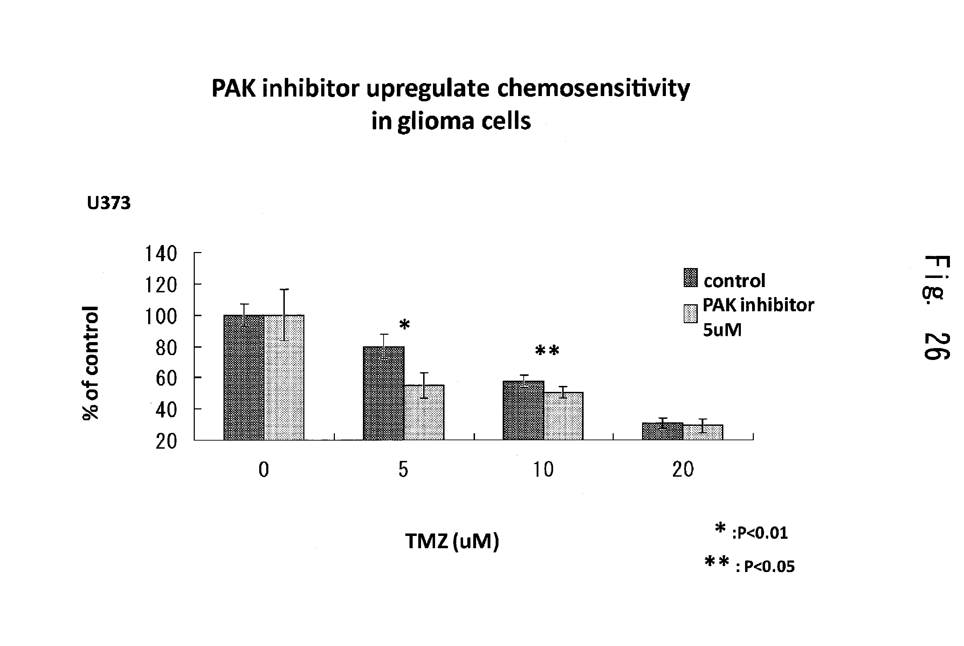

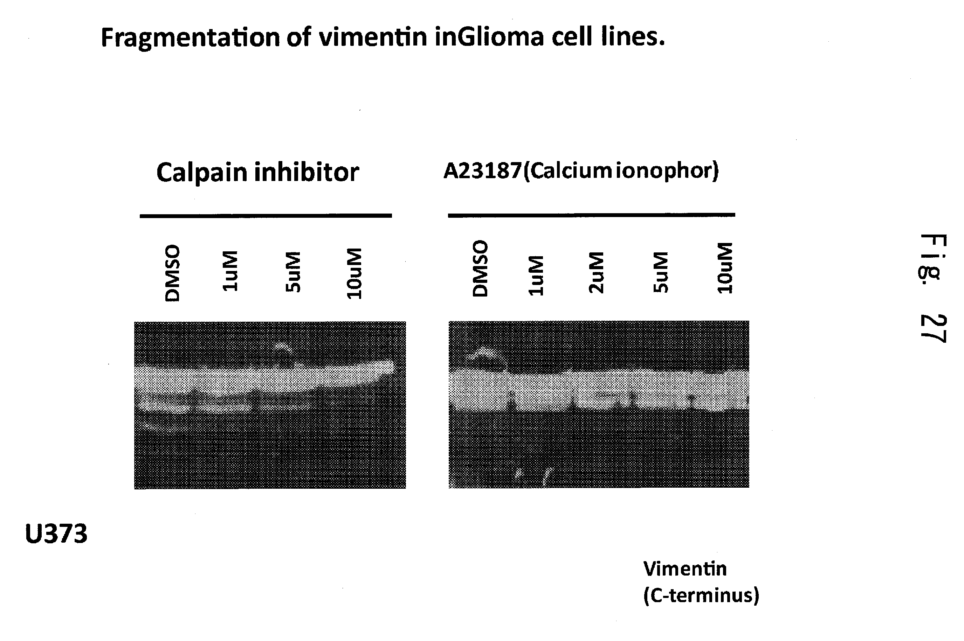

Then, the chemosensitivity to TMZ was reviewed in the same way by knocking down the gene portion involved in the expression of ephrin existing at the most upstream of the vimentin activation loop from model animals using a siRNA. The results revealed that ephrin could upregulate the sensitivity to TMZ significantly in the same manner as vimentin. Further, 2D-Western blotting showed that the phosphorylation and cleavage of vimentin located at its downstream could be inhibited by the activation inhibitor of PAK1. This effect on the inhibition was found to upregulate the sensitivity of the U373 cells to TMZ. In other words, the PAK1 activation is considered to upregulate the phosphorylation of vimentin and enhance the cleavage of vimentin as well, thereby leading to a downregulation of the sensitivity to TMZ (Figs. 25 and 26). Moreover, reviews were made about an influence of the inhibition of the calpain activation of a calpain inhibitor, a calpain activator or a calpain activation subunit SS with siRNA on the sensitivity to anticancer agents in connection with calpain involved in the cleavage of vimentin at the downstream of PAK1. As a result, both of the calpain inhibitor and the calpain activator showed a significant upregulation of the sensitivity of the glioma cells U373 to TMZ. It was also found that the activation of calpain inhibited the sensitivity to anticancer agents to a remarkable extent by activating calpain with calcium ionophore or A23187. Therefore, the fragmentation of vimentin with calpain was obviously found to downregulate the sensitivity of glioma to anticancer agents (Figs. 27, 28).

Furthermore, as the N-terminal vimentin fragments translocated into the cellular nuclear by the activation of the vimentin activation loop upregulates the transcriptional activity of ephrin, reviews have been performed as to whether the treatment of an ephrin ligand (ephrin A1) in cells may activate the activation loop of the intercellular vimentin and exert an influence on the sensitivity to anticancer agents. After U373 cells were treated with vimentin siRNA or control siRNA under serum-free culture conditions, ephrin A1 was added to the U373 cells, followed by incubation of the U373 cells so treated above and a sequential measurement for the transcriptional activity of the ephrin receptor by qRT-PCR. The results revealed that a sequential upregulation of the transcriptional activity of the ephrin receptor was measured in the control siRNA group, while the transcriptional activity of the ephrin receptor was remarkably inhibited in the vimentin siRNA treatment group. It was also found that the treatment with ephrin A1 downregulated the sensitivity of the U373 cells to TMZ to a significant extent.

In addition to the above observations and views, when taken it into consideration that the treatment with ephrin A1 upregulates the activity of PAK1 and the cellular nuclear translocation of the N-terminal vimentin fragments as well as a series of these reactions is inhibited by the PAK inhibitor and the calpain inhibitor, it has now been made clear that a series of signals of the cascade consisting of ephrin receptor → Cdc42 activation → PAK1 activation → vimentin phosphorylation → cleavage of vimentin with calpain → nuclear translocation of N-terminal vimentin fragments → elevation of transcriptional activity of ephrin receptor → activation of vimentin activation loop can be activated in the LOH- malignant glioma cells and this is one of the mechanisms of its chemoresistance to anticancer agents (see Fig. 15).

As described above, in accordance with the present invention, the methodology of the integrated proteomics using, for example, human tumor tissue cell samples may extract a group of proteins varying the amounts of expression levels and changing modification structures involved in the resistance to anticancer agents and the intercellular signal network through a group of responsible molecules relating to changes of their functions. This is the great advantages because this may assist in clarifying a part of the mechanism of the chemoresistance of anticancer agents and obtaining basic information on development of targets for treatment, markers for clinical use, and new medicines.

Generally, the advantages of the integrated proteomic analysis are based on a concurrent acquisition of information relating to quantitative variations in proteins themselves (iTRAQ, etc.) and variations in expression levels of proteins at a mRNA level before the protein transcription (DNA microarrays, qRT-PCR, etc.) as well as changes of proteins including post-translational modifications (2D-DIGE, etc.). On the other hand, as described above, an issue of the conventional techniques on dealing with results of integrated proteomics analyses lies in that, among others, it was difficult to compare and integrate results of analyses because there is no common format for combining the results of analyses obtained separately by plural proteomic analyses (2D-DIGE, iTRAQ, etc.) and transcriptomic analyses (DNA microarrays, etc.) as well as no methodology has been established to efficiently extract a significant information from the results of separate analyses and analyze an enormous number of molecules. Contrary to the above, the integrated proteomic analysis software according to the present invention, such as iPEACH and MANGO, may present the great advantages in solving the problems inherent in the conventional proteomics analyses, for example, in terms of an integration of all languages of molecules identified from raw data obtained individually by each of the analysis techniques, a coverage of information linked to information on post-translational modifications, quantitative values, chromosome information, information on linkage of GO, and the like, and an automatic generation of the integrated and unified files with weights and priority orders added thereto.

In accordance with the present invention, for instance, a unified file of an original analysis data using an AO/AOA glioma tissue samples may contain information of an enormous number of molecules amounting to ca. 30,000 molecules. From such an enormous volume of the information, more than 16,000 molecules were extracted as being statistically significant and a group of less than 140 molecules were sorted out as varying their expression levels significantly between the 1p/19q LOH⁺ and 1p/19q LOH⁻ tumor tissues. The molecule group consisting of less than the 140 molecules was then analyzed with the results that an intercellular signal, which could not be so far discovered by a sole use of the conventional techniques including the proteomics or DNA microarray analyses, was extracted for the first time by a comprehensive assessment and analysis of the results of all the information using the integrated mining technique according to the present invention. For the narrowed-down signals, it is needed to meticulously verify the signals one by one at a cell level using an inhibitor or an activator or a siRNA, etc., however, the present invention may be expected to offer good results as anticipated because the signals are narrowed down to the highest possible extent. Moreover, the present invention has succeeded in all the verification experiments for the new intercellular activated signal (vimentin activation loop) extracted by the analysis of glioma resistant to anticancer agents. In addition, the present invention was found to be useful for creating an idea of a methodology that may convert the resistance to anticancer agents to the sensitivity thereto.

Moreover, as an amount of a pathological sample is very limited, the information obtainable from the sample has to be utilized to the greatest possible extent. In this respect, the present invention may provide the great advantages in re-utilizing the resulting information for various meta-analyses by constructing a database from the files integrated by the iPEACH/MANGO software according to the present invention.

The present invention will now be described hereinafter in detail by way of working examples. It is to be understood, however, that the following working examples are described solely to illustrate the present invention in an exemplar way and they are described with no intention to limit the present invention. The present invention are not limited at all in any respect to the following working examples and, as a matter of course, encompasses any and every modification in accordance with design variations which do not depart from the objects and the technical concepts of the present invention.

### Example 1: Strategy of a united proteomics

Samples to be used for this example were collected from brain tumor tissues of patients with anaplastic oligodendroglioma/astrosystome (AO/AOA) obtained by surgical operations and then subjected to a histopathologic examination to classify a type of the LOH. Then, from these samples, proteins and mRNAs were extracted concurrently, and they were subjected to two kinds of proteomics analyses, one being the 2D-DIGE analysis and the iTRAQ analysis as well as the other being the DNA microarray analysis. All molecules demonstrating differences in expression levels were identified, and the expression levels of the proteins and the mRNAs were analyzed by an in silico united data mining, thereby verifying biological functions of those molecules in the glioma cells.

### LOH analysis step:

The LOH analysis step is involved in a classification of the brain tumor samples to be used for an object of analysis by the presence or absence of the LOH.
The brain tumor samples were embedded in an OCT compound and freezed after collection from the tumor tissues of the AO/AOA patients. After they were subjected to a HE staining test to check whether that were appropriate for a test which followed, they were divided into two groups, i.e., a group having the LOH (a LOH⁺ group) and a group having no LOH (a LOH- group), each group consisting of four specimens. Proteins and mRNAs were extracted from each specimen, followed by implementing analyses such as iTRAQ and 2D-DIGE.

### Analysis step by the iTRAQ method:

Tissue lysates were prepared by collecting tissue samples from the tumor tissues removed from patients with brain tumor. The tissues were solubilized in a lysate buffer composed of 9.8 M urea, 4% CHAPS, 1.45 µM pepstatin, 2 mM Na₂VO₄, 10 mM NaF, 1 µM okadaic acid, a 1% (v/v) protease inhibitor cocktail (GE Helthcare), and a 1% (v/v) nuclease mix (GE Helthcare), and then homogenized 100-fold with a pestle. To the homogenized lysate were added 1 mM DTT and 0.5 mM EDTA, and the mixture was re-homogenized 100-fold with a pestle. The resulting lysate was then centrifuged at 15,000 rpm at 4 °C for 20 minutes, followed by determining the protein concentration in the resulting supernatant using Bio-Rad protein assay kit (Bio-Rad Laboratories).

The lysates so prepared above were then labeled with iTRAQ reagents. An individual protein sample (100 µg) was treated with a 2-D Clean-Up Kit (GE Healthcare), and the resulting precipitates were solubilized in 9.8 M urea (10 µl). The labeling with the iTRAQ reagents was performed by an 8-plex method with the manufacturer's protocol modified to the lowest possible extent. In other words, the four samples obtained each from the LOH⁺ group and the LOHgroup of the tissues of AO/AOA patients, respectively, were treated with the iTRAQ reagents, followed by addition of a solubilizing buffer (20 µl) and a denaturing reagent (1 µl). Thereafter, the samples were reduced with a reducing agent (2 µl) and then alkylated, followed by implementing a reaction at 60°C for 1 hour and then another reaction at room temperature for 10 minutes by blocking the reduced cysteine residue with a cysteine blocking agent. Thereafter, the trypsin digestion was implemented at 37°C for 16 hours using a 1 µg/µl trypsin aqueous solution (12.5 µl). Then, the peptides in each of the samples were labeled with eight kinds of reporter iTRAQ reagents by thawing one vial of the respective reagents and reconstituting in 80 µl of ethanol. Thereafter, iTRAQ reagent-labeled samples (iTRAQ reagents #113, #114, #115 and #116 for the LOH⁺ group and iTRAQ reagents #117, #118, #119 and #121 for the LOH- group) were added to the digested products prepared above, and the mixture was reacted at room temperature for 1 hour. These labeled samples were mixed together, followed by diluting the mixed samples with a buffer (20% v/v ACN; 10 mM potassium phosphate, pH 3.0) and filled in a Mono S column (GE Healthcare) equilibrated with the above buffer. The peptides were eluted by a solvent B concentration gradient (10 mM potassium phosphate, pH 3.0 and 1 M KCl in 20% v/v ACN): 0-2 min, 0 to 7% B; at 6 min, to 14% B; at 8 min, to 32% B; at 13 min, to 70% B; and at 21 min, to 100% B). The eluate from each of the samples labeled with the iTRAQ reagents was subjected to anion exchange HPLC and fractioned into 40 fractions. Each fraction was then dried by vacuum centrifugation and re-hydrated with a solution containing 2% ACN and 0.1% trifluoroacetic acid (TFA), followed by desalination with ZipTip µ-C18 pipette tips (Millipore).

Mass spectrometry analysis by nanoLC-ESI-Qq-TOF and nanoLC-MALDI-TOF-TOF was implemented using an aliquot in a one-tenth amount of each fraction. A C18 nano LC was carried out by spotting eluted fractions on a MALDI plate using DiNaMap (KYA Tech) equipped with a spotting device. The sample was injected into a C18 column (0.5 mm I.D. x 1 mm length, KYA Tech) equilibrated with solvent A (2% ACN, 0.1% TFA), and passed through a C18 nano-column (0.15 mm I.D. x 100 mm length, KYA Tech) to divide the sample by an addition of solvent B (70% ACN, 0.1 % TFA) at a flow rate of 300 nl/minute and a 90-minute concentration gradient (0 - 10 minutes: 0 to 20% B; up to 65 minutes: 50% B; up to 75 minutes: 100% B). The effluents from the column were then mixed with a matrix (50% ACN of 2 mg/ml α-cyano-4-hydroxycinnamic acid, 0.1 % TFA) at a flow rate of 1.4 µl per minute. The fractions were spotted at equal intervals of every 5 second on a stainless steel MALDI target plate (192 wells/plate, Applied Biosystems). The mass spectrum of the peptide was measured by 4000 Series Explorer software (v.3.6) using 4700 Proteomics Analyzer (Applied Biosystems). The mass spectrum (m/z 800-4000) of each fraction was obtained by irradiation with 1,500 laser shots. In order to analyze the peptides present in minute amounts, all peaks having a S/N threshold of from 50 to 75 as well as from 75 to 100 in each mass spectrum were selected for a MS/MS analysis by 5000 laser shots and 4000 laser shots, respectively.
Thereafter, all peaks having an S/N threshold of 100 were selected for a MS/MS analysis by 3000 laser shots. The labeled peptides were fragmented at a pressure of 1 x 10⁻⁶ Torr and impact energy of 1 kV under an environment of an impact gas.

### Analysis step by nano LC-ESI MS/MS analysis:

This analysis step is involved in an analysis of the sample used above using the nanoLC-ESI MS/MS. The sample was filled in a 5mm RP C18 precolumn (LC Packings) at the rate of 30 µl per minute and washed for 10 minutes. After washing, the sample was separated using a separation column (PepMap RP column (inner diameter: 75 µm; length: 150 µm; LC Packings)) filled with C18 beads (3 µm) having a pore size of 100 angstrom. The sample was separated using solvent B (85% ACN, 0.1% formic acid) at a flow rate of 200 nl per minute and a 90-minute concentration gradient (0 - 60 minutes: 0 - 40% B; 70 minutes: 100% B), then followed by dividing the sample into two fractions.

The first fraction was analyzed using QSTAR Pulsar i mass spectrometer (Applied Biosystems/MDS SCIEX, CA). The analysis was performed using a software, Analyst QS 1.1 (Applied Biosystems/MDS SCIEX) set at a scan cycle starting at 1 s MS and then at 3 s MS at which three peaks present in the highest amounts were scanned three times for each peak. The data was collected by deleting the previous target ions for 60 s. On the other hand, the second fraction was used for analyzing peptides present in smaller amounts. This analysis was implemented under the same conditions as the first analysis except the analysis of the peptides present in larger amounts. The labeled peptides were fragmented under CID conditions that were designed to generate iTRAQ reporter ions.

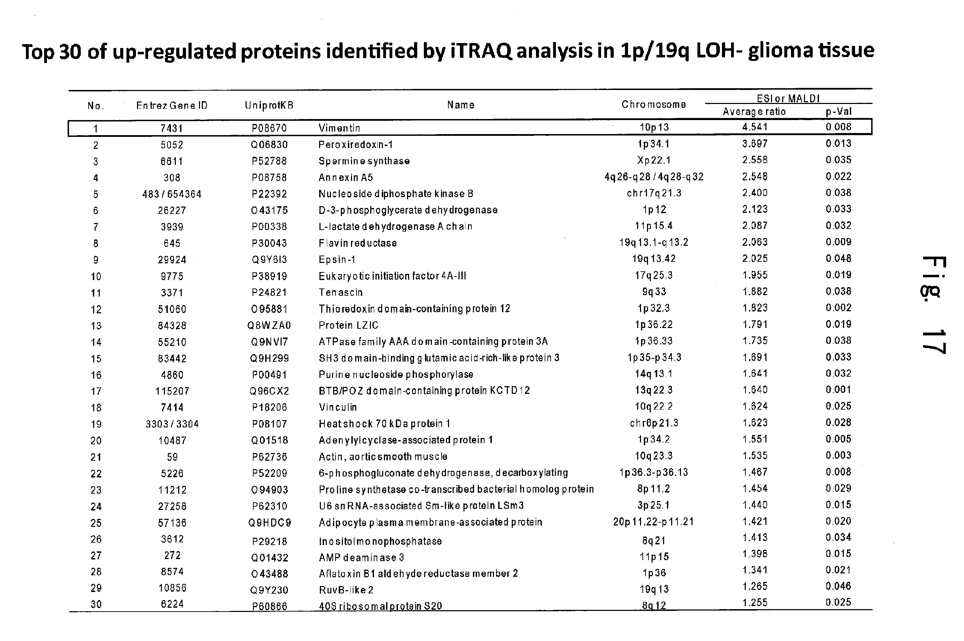

The analysis of the MALDI and ESI data was performed by the data mining using ProteinPilot (Applied Biosystems). The results revealed that a total number of 2,103 proteins were identified on the basis of a quantitative comparison. Among these proteins, 40 proteins were listed as those upregulating expression levels specifically and 32 proteins as those downregulating them specifically in the LOH⁻ cells. Among those upregulating the expression levels, the one having the highest variations in expression levels was found to be vimentin (see Fig. 17).

### Analysis step by two-dimensional fluorescence differential gel electrophoresis (2D-DIGE):

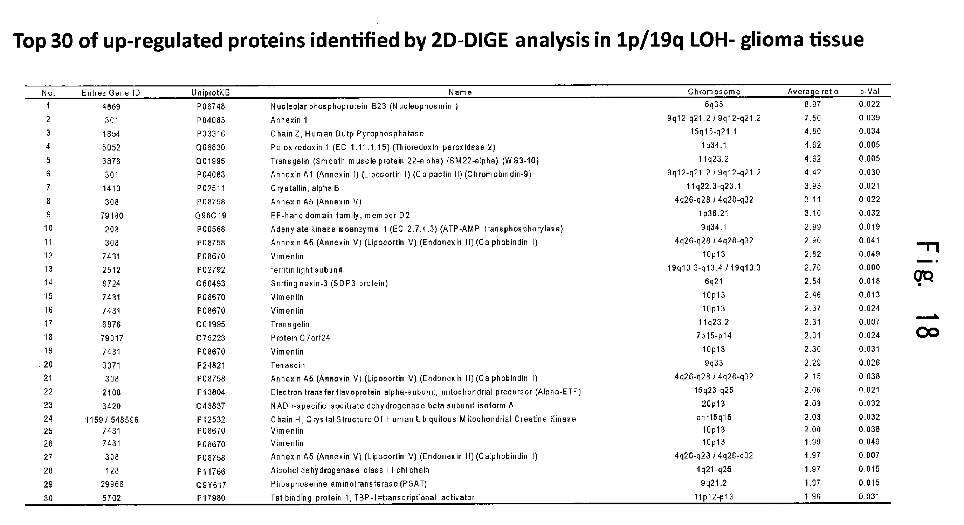

The lysates prepared above as the samples were analyzed by the 2D-DIGE method. As the samples, there were used eight kinds of the same samples as used for the above iTRAQ: four specimens each for the LOH⁺ group (sensitive to anticancer agents) and each for the LOH- group (non-sensitive to anticancer agents) as well as four specimens for a normal group as a control. For four specimens of each of the respective groups, 50 µg of the solubilized proteins were labeled with Cy2 (a mixture of all proteins of all samples), cy3 (four samples of each of the LOH⁺ and LOH- groups), and Cy5 (four samples of each of the LOH⁺ and LOH- groups). They were then mixed in permutations and combinations to prepare three kinds of samples (Cy2, Cy3 and Cy5), and the two-dimensional electrophoresis was implemented for 12 sheets of gel plates (pH 3-10) and another 12 sheets of gel plates (pH 4-7), followed by an analysis with confocal fluorescence gel scanner to give a total number of 48 sheets of pictures. The resulting pictures were then analyzed by the integrated mining using DeCyder (Fig. 18). At the same time, non-labeled samples were subjected to two-dimensional electrophoresis to detect a phosphorylated protein. The Two-dimensional electrophoresis was implemented on an identical two-dimensional electrophoresis gel using a fluorescent reagent for staining phosphorylated proteins specifically to investigate differences in expression levels of the phosphorylated proteins. The results of this analysis revealed that the spots stained violet in color indicate a group of the spots upregulating phosphorylation specifically in the non-sensitive samples, the spots stained yellow in color indicate a group of the spots upregulating phosphorylation in the normal spot samples, that is, downregulating the phosphorylation in the non-sensitive samples.

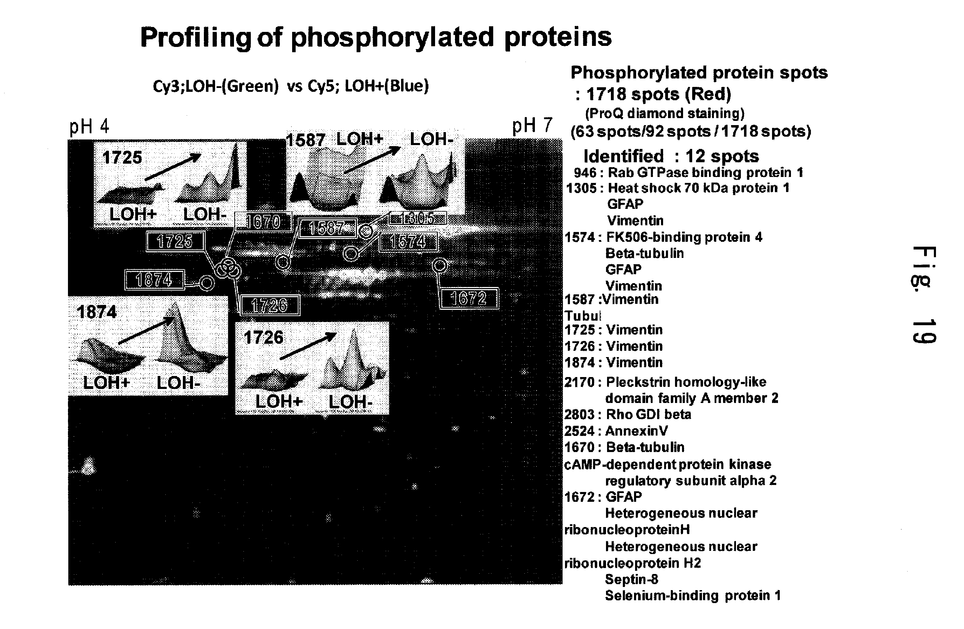

Further, a total number of 1,719 phosphorylated protein spots were detected by staining with a ProQ Diamond solution specific for staining phosphorylated proteins. Among 92 spots of the proteins found varying the expression levels by an analysis using the 2D-DIGE method, 63 spots were found to be spots of phosphorylated proteins. These spots were then identified by mass spectrometry using ProQ Diamond, and the results revealed that they were spots of GFAP, vimentin, tubulin, HSP70, and so on. By taking vimentin, one of the proteins identified, as an example, each of 15 vimentin spots was found that the variations in expression levels in the LOH⁻ group were larger compared with those in the LOH⁺ group. Moreover, as the molecules which differed greatly in the expression states between the LOH- group and the LOH⁺ group, a vimentin of an intermediate-sized filament was identified (Fig. 19).

The primary structures of all of 15 kinds of the vimentin spots identified were analyzed by the mass spectrometry and the N-terminal analysis. The results of this analysis revealed that the phosphorylated sites were identified at 19 locations, and changes of molecular weights were caused to occur by a specific cleavage at the N-terminal site (see Fig. 14).

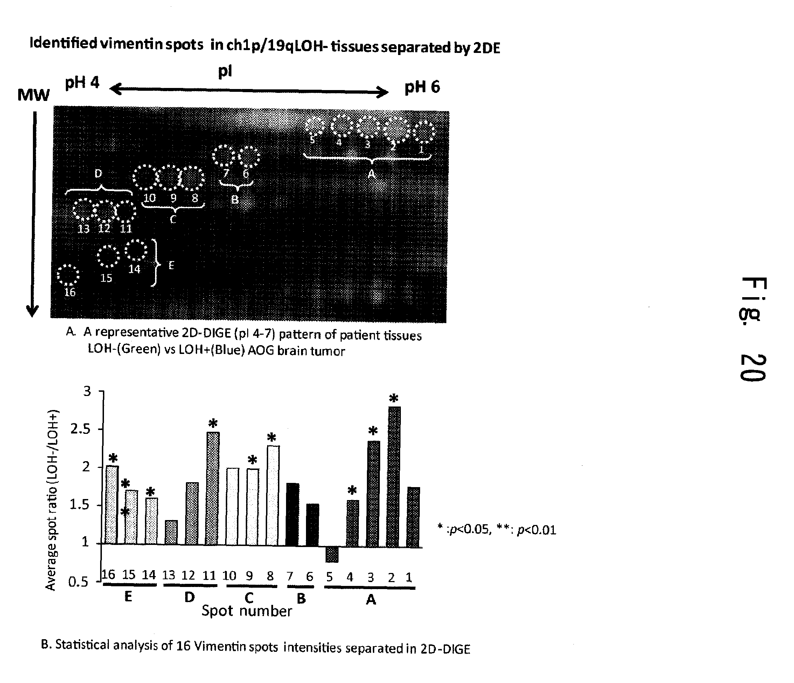

Moreover, plural bands observed by Western blotting were verified so as to what kinds of spots were detected by the 2D-DIGE method. The 2D-DIGE analysis was implemented to observe the positions of the vimentin spots and compare their change rates in detail. The primary structure and the molecular weight of each spot were also analyzed by the mass spectrometry and the N-terminal analysis. The results of the 2D-DIGE analysis revealed that vimentin was identified at a total number of 16 protein spots. The resulting protein spots were provided with group numbers (A to E) in sequential order of descending molecular weights, that is, in the order of descending pI values. In each group, two to five spots were detected, and the spots were provided with spot numbers (1 to 16). Among these spots, the spots with spot numbers 3, 4, 8, 9, 11, 14, 15 and 16 were identified as spots that varied the expression levels significantly. The detailed analysis of these 16 spots revealed that 19 phosphorylated sites were identified. Among these phosphorylated sites, Tyr319, Tyr358, Tyr400 and Thr449 were the phosphorylated sites that were not yet found to be reported, and Ser26, Ser39 and Ser73 were found to be sites phosphorylated by PAK1. In addition, the results of the N-terminal analysis showed that the group A indicates a full length vimentin, the group B indicates a vimentin fragment starting from the forty-second amino acid, the group C indicates a vimentin fragment starting from the fifty-fourth amino acid, the group D indicates a vimentin fragment starting from the seventy-second amino acid, and the group E indicates a vimentin fragment starting from the ninety-third amino acid. It was further found that vimentin fragments having relatively small molecular weights varied the expression levels significantly. Moreover, the sites of cleavage of the groups B, C and D were found to be all based on cleavage by calpain. The above results revealed that sixteen spots were identified by the 2D-DIGE method as those whose changes of the molecular weights and pls are based on the modifications by phosphorylation and cleavage by calpain. In addition, it may be considered that the changes of vimentin are caused to occur based on the activation of a group of enzymes responsible for modifications (Fig. 20).

### Total RNA extraction and microassay step:

This example is involved in a total RNA extraction and a microassay method using the same samples as those used above. The samples was composed of a learning set consisting of a total number of 10 samples including 8 samples identical to those used for the above proteomic analysis, i.e., 4 samples each for LOH- and LOH⁺ groups and additional two mRNA samples (1 sample each for LOH- and LOH⁺ groups).
The total RNA extraction from the AO tissues was carried out using Qiagen kit (Qiagen, Inc., Valencia, CA). The quantity and quality of RNA were determined using RNA 6000 Nano Assay kit and Agilent 2100 BioAnalyzer (Agilent Technologies, CA), respectively.

A brief description will be made hereinafter regarding processes of the microassay experiments. First, RNAs were converted to double-stranded cDNA and transcribed in vivo, thereby preparing biotin-labeled cRNAs. The resulting biotin-labeled cRNAs were then fragmented and hybridized with HG-U133 plus2.0 GeneChips (Affymetrix), followed by staining with phycoerythrin - streptavidin and preparing raw data of the AO/AOA tissue at a probe level and cell files using GCOS (GeneChip Operating Software). The cell files revealed that a total number of the probes were presented: 24,567 probes. Among them, 2,241 probes were found upregulating their expression levels (p<0.05, >1.5), while 863 probes were found downregulating their expression levels (p<0.05, >0.5). The data mining was implemented using Gene Spring GX.

The above analysis methods may enable profiling (ProQ) of proteins that varied expression levels and were phosphorylated as well. The profiling of the proteins shows that a total number of about 4,000 protein spots were discovered on average in each of the samples. The protein spots identified as demonstrating significant variations in expression levels of proteins on the basis of the results of a statistically quantitative analysis of all profiles were then extracted from the electrophoresis gel and then subjected to trypsin digestion. After the trypsin digestion, the protein peptides were extracted and then analyzed by mass spectrometry to identify the proteins. The results then revealed that a total number of 3,922 spots were identified as the proteins upregulating significantly in the LOH⁻ group. Among them, a total number of 106 spots were found upregulating by more than 1.2 times, while a total number of 105 spots were found downregulating by less than 1.2 times. Among them, vimentin was identified as the molecule that upregulated the protein expression variation amount while changing its isoelectric point and molecular weight to the largest extent.

### Integrated data listing step by iPEACH:

A list was generated of all data including, for example, Entrez No., SW No., Gene No., molecule name, expression ratio, p-Value, Yes/No of modification, GO, analysis method, and data line differing by analysis methods (if the identical protein would be identified in plural numbers yet their analysis results would differ from one another by the 2D-DIGE method, such identical protein are presumed to undergo post-translational modifications and numbers of identifications are added). The molecules listed in the above file which were formatted by the notation system of this file were then sorted out in the order of descending change rates, thereby generating an integrated upregulation list and downregulation list. At the same time, the proteomic data and the transcriptomic data rewritten by this format were extracted separately. The data generated by these analyses contained a list in about 30,000 lines representing molecules as analyzed quantitatively. The results of these analyses revealed that the molecule extracted by the proteomic analysis as the molecule indicating the highest upregulated rate was vimentin, while the molecule extracted by the DNA microarray as the molecule indicating the highest upregulated rate was EphA4.

### GO analysis step by GeneSpring GX:

By the proteomic analysis using the iTRAQ method, a total number of 2,103 proteins were identified. Among them, 40 proteins were found upregulating the expression levels (p<0.05, >1.2) and 32 proteins were found downregulating the expression levels (p<0.05, <0.83). The proteomic analysis using the 2D-DIGE method showed that 106 proteins were upregulating the expression levels (p<0.05, >1.2) and 105 proteins were downregulating the expression levels (p<0.05, <0.83). On the other hand, the analysis by the DNA microarray presented a total number of 24,567 probes including 2,241 upregulated probes (p<0.05, >1.5) while 863 downregulated probes (p<0.05, <0.5). All of the resulting data were then integrated by converting UniProt Acession Nos. assigned to the upregulated and downregulated proteins and Affimetrix ID Nos. assigned to the upregulated and downregulated probes to Entrez Gene ID Nos. The above results revealed that a total number of 16,287 proteins were extracted, which included 1,512 upregulated proteins and 846 downregulated proteins. The resulting data were subjected to the GO analysis by GeneSpring GX. The results of this GO analysis confirmed that the items "regulation of gene expression" and "regulation of transcription" were found to be upregulated significantly in "Biological Process" and the items "DNA binding", "phosphotransferase activity" and "phosphate group as acceptor" were found to be upregulated significantly in "Molecular Function".

### Molecular network analysis step by KeyMolnet:

In order to analyze the relationship among a group of molecules extracted by the GO analysis method, the KeyMolnet analysis was implemented by extracting the expression variation amount and the locus information of each molecule from the above unified file on the basis of the list of the important molecules extracted by the GO analysis. This analysis clarified paths containing these molecule groups. More specifically, the results of this KeyMolnet analysis revealed that vimentin at the center, with kinases such as a group of modified enzymatic molecules (p21 activated kinase: PAK1), P13K, PKC, Rho, etc. and proteases such as calpain, etc. surrounding it, upregulated the expression levels. It was further found that the modified enzymatic molecules were located at locus Chrlp/19q. Moreover, an analysis of a portion upstream of these modified enzymatic molecules revealed an upregulation of G protein and EphA4, etc. (Fig. 11). It is noted herein that the molecule located at the most upstream location of these networks was found to be EphA4 that in turn demonstrated the highest upregulate of the expression variation amount at the upstream (upregulated by 14.9 times higher than the expression levels of the LOH⁻ group upregulated by the DNA microarray analysis). The KeyMolnet analysis made it feasible to clarify, for example, a relationship of a group of molecules, which was not known until now by the GO analysis alone, that is, what a pathway is activated.

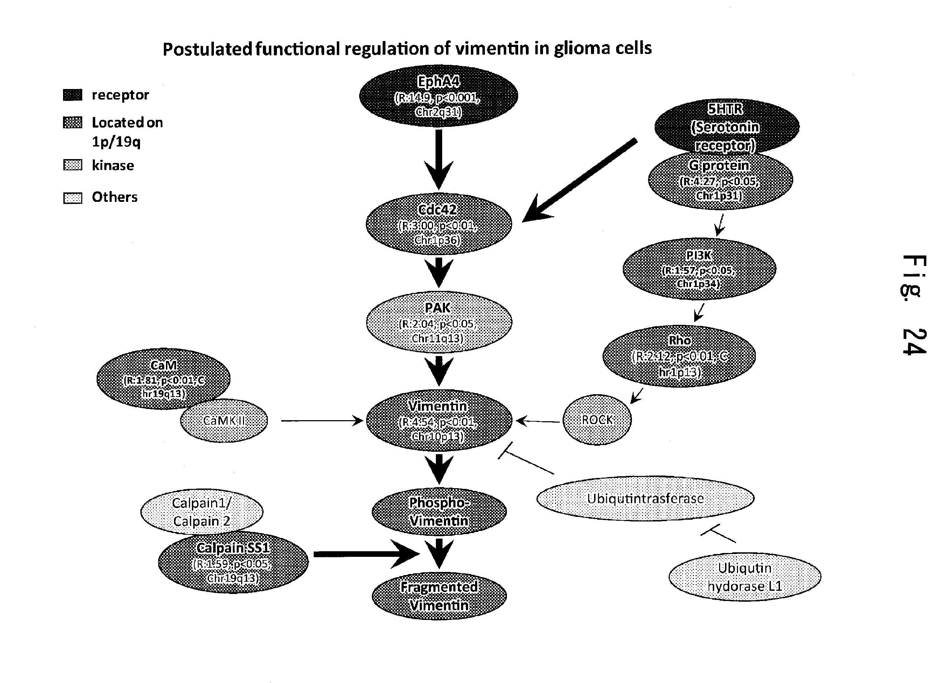

Further, the molecular network analysis by KeyMolnet may extract a network associated with the network starting from EphA4 and ending with vimentin from all data. Finally, a specific cleavage network consisting of EphA4 → CDC42 → p21 dependent protein kinase (PAK1) → vimentin phosphorylation → calpain → specific vimentin cleavage was extracted. Moreover, a network consisting of vimentin → EphA4 was extracted, too, as a network that have not yet been reported (Fig. 24).

It is to be noted herein that this vimentin - EphA4 network (referred to as "vimentin - EphA4 activation loop", too) was confirmed to be a new network and to be activated in an abnormal fashion in malignant glioma cells resistant to chemotherapy. This network is composed of an EphA4 - Cdc42 - PAK1 activation cascade, causing the phosphorylation of vimentin and the fragmentation of vimentin by calpain and translocating the N-terminal fragments into the cellular nuclear. This phenomenon was found to be deeply involved in cancer progress and a life expectancy of patients after treatment by chemotherapy. Surprisingly, it was further observed that the N-terminal vimentin fragments were converted into a transcription factor of EphA4 and they upregulated or downregulated its expression levels by treatment with ephrin A1 or siRNA. These phenomena were also found to be related to an upregulation or downregulation of sensitivity to an anticancer agent, TMZ. The results of this analysis suggest that the N-terminal fragments of intermediate-sized filaments may work as transcription factors that may upregulate the resistance to chemotherapy via the activation loop composed by EphA4 - Cdc42 -PAK1 - vimentin, thereby offering the possibility of becoming a useful tool for retrieving a candidate for a new medicine.

### Example 2:

In this example, the tissue lysates used in Example 1 were subjected to the two-dimensional electrophoresis analysis followed by the blotting analysis using a polyvinylidene fluoride (PVDF) membrane having a pore size of 0.2 µm. The PVDF membrane was stained with simply blue and the vimentin spots were cut off for sequencing, followed by drying in air. The N-terminal amino acid sequence was determined by Edman degradation using a BLAST program compared with the vimentin sequence in the NCBI database.

### Immunohistochemical analysis of vimentin:

By focusing on vimentin as an important molecule that was observed by the GO and KeyMolnet analyses as upregulating its expression variation amount significantly, its expression variation amount was verified by an immunohistological staining method and Western blotting. The experiment was carried out by subjecting the same patient-derived tissues as used for the integrated proteomics analysis to the immunohistological staining and Western blotting using a monoclonal antibody (V9) to vimentin. Western blotting was implemented using the U373 and U251 cells reported as LOH⁻ and the U87MG and A172 cells reported as LOH⁺. Further, patient-derived tissues other than those used for the integrated proteomics analysis were also used for Western blotting.

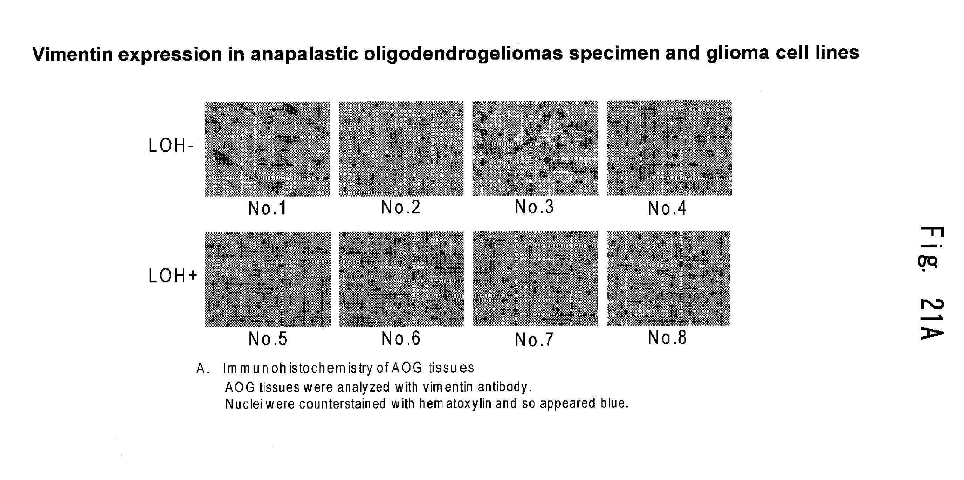

Fig. 21A shows results of an analysis by the histoimmunological staining of the vimentin-expressing glioma tissues and glioma cell line derived from AO patients. The nuclei of cells were stained with hematoxylin. The results of this staining revealed that vimentin was expressed in the vicinity of the cytoplasm and cell nuclei in the LOH⁻ group compared with in the LOH⁺ group.

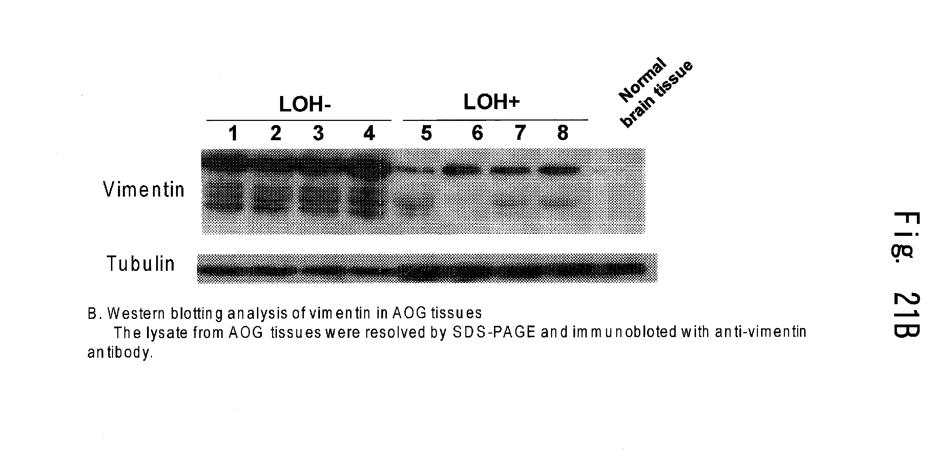

Fig. 21B is a Western blotting drawing showing vimentin in the LOH⁺ and LOH⁻ tissues of an AO patient. Western blotting was implemented by separating a cell lysate sample from the LOH⁺ and LOH⁻ tissues of the AO patients by SDS-PAGE electrophoresis, electroblotting the sample on a PVDF membrane and immunoblotting it with an anti-vimentin antibody. In Western blotting of vimentin using the LOH⁺ and LOH⁻ tissues, it was found that the expression variation amount of vimentin was remarkably upregulated in the LOH⁻ tissues compared with in the LOH⁺ tissues. In addition to vimentin having the molecular weight of approximately 50 kDa, three vimentin bands having molecular weights of about 45 kDa were observed, and these vimentin bands also showed the upregulated expression variation amounts.

Fig. 12A shows the amounts of vimentin in the LOH⁺ and LOH- tissues of AO patients. In this experiment, Western blotting of vimentin was implemented using a total number of 36 specimens (LOH⁺: 18 specimens; LOH⁻: 18 specimens) of tissues derived from the patients other than those used for the integrated proteomics analysis. The quantitation of vimentin revealed that the amounts of variations in expression levels of vimentin were upregulated significantly.

Fig. 12B shows results of verification of the amounts of variations in expression levels of vimentin in glioma cells by Western blotting. In this experiment, Western blotting was implemented by separating cell lysate samples from the LOH⁺ and LOH⁻ patients by SDS-PAGE electrophoresis, electroblotting them on a PVDF membrane and immunoblotting them with an anti-vimentin antibody. As a control, actin was used. As the glioma cell line, LOH- cells (U251 and U373 cells) and LOH⁺ cells (A172 and U87 (ATCC) cells) were used. The results of the Western blotting analysis revealed that vimentin upregulated in a larger amount in the LOH⁻ cells than in the LOH⁺ cells, and a cleavage of vimentin was facilitated.

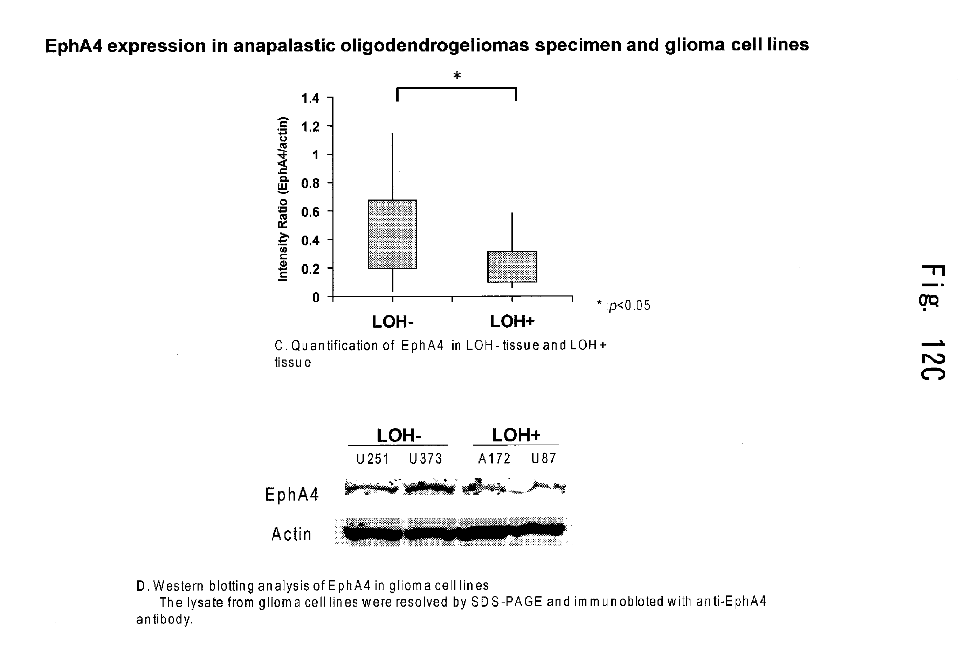

Fig. 12C shows expression states of EphA4 in LOH⁺ and LOH⁻ tissues of AO patients and the glioma cell line. An upper figure shows amounts of EphA4 in the LOH⁺ and LOH⁻ tissues of AO patients, and a lower figure shows results of the Western blotting analysis of EphA4 in the glioma cell line (U251, U373, A172 and U87). Western blotting was implemented by separating cell lysate samples from the LOH⁺ and LOH⁻ patients by SDS-PAGE electrophoresis, electroblotting them on a PVDF membrane and immunoblotting them with an anti-vimentin antibody. As a control, actin was used.

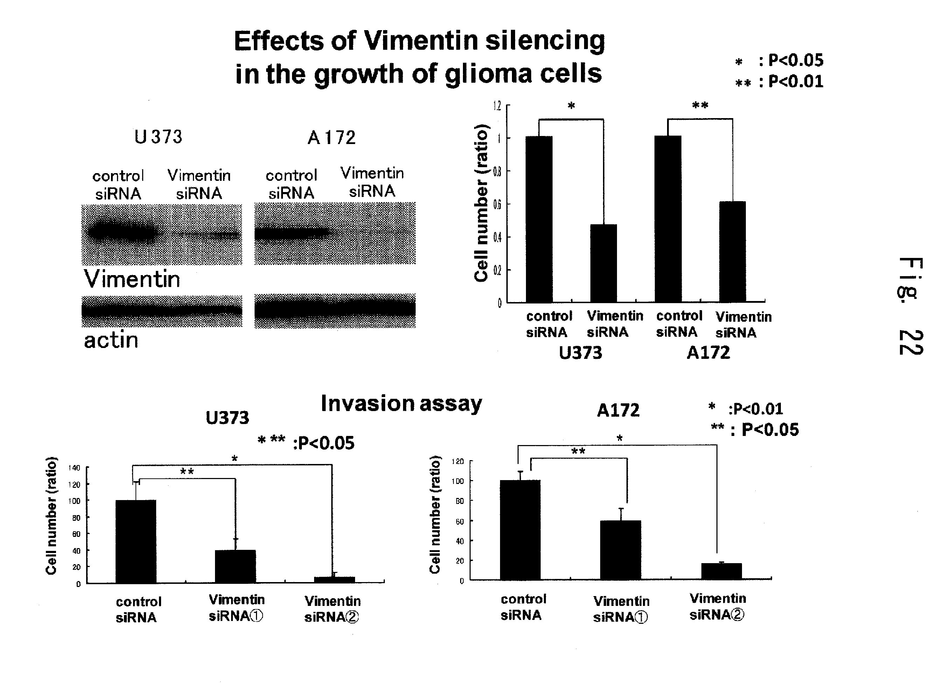

Fig. 22 shows the effect of silencing of vimentin in the growth of glioma cells (U373 and A172 cells) using a siRNA. This experiment was carried out by inoculating glioma cells on a 6-well plate at the rate of 1 x 10⁶ cells per well, and a vimentin siRNA or a control siRNA was introduced after 24 hours using lipofectamin 2000. In 48 hours after introduction of siRNA, the expression levels were measured by Western blotting (Fig. 22A). In 24 hours after the siRNA introduction, a 96-well plate was inoculated with glioma cells at the rate of 1,000 cells per well, and a cell survival rate was observed by Western blotting after 48 hours (Fig. 22A). Further, in 48 hours after the introduction of the vimentin siRNA, an invasion assay was carried out by inoculating glioma cells at the rate of 2 x 10⁴ cells in a Matrigel incubation chamber under a serum-free condition. After 24 hours, a state of culture was observed by staining with Diff-Quic (Fig. 22B).The results of this staining revealed that the glioma cell line caused a decrease in the cell proliferation and the invasive capacity of vimentin by the use of the siRNA. This suggests the possibility that an upregulation of the expression level of vimentin may facilitate the cell proliferation and the invasive capacity, thereby worsening the prognosis.

Fig. 23 shows differences in the chemoresistance between the glioma cell lines (U373 and A172 cell lines). As a medicine, the anticancer agent, temozolomide (TMZ), was used. Fig. 23A shows the chemoresistance to variations in concentrations of TMZ. Fig. 23B shows the chemoresistance of the glioma cell line to TMZ observed by the silencing of vimentin using siRNA, as well as the chemoresistance of a control to TMZ.

Fig. 23A shows an influence of the amounts of variations in expression levels of vimentin on the resistance to anticancer agents in the glioma cell line. The experiment was carried out by inoculating the glioma cell line on a 96-well plate at the rate of 100 cells per well and adding TMZ at varying concentration ranging from 0 to 20 µM to each well after 5 hours of inoculation. After the plate was incubated for 6 days, absorbance at 450 nm was measured by Western blotting. The rate of living cells was expressed by percentage (%) at the defined concentrations of TMZ by setting the concentration rate of 0 µM as a control and as a standard rate of the living cells at the respective TMZ concentrations. The results of the absorbance analysis revealed that the U373 cells demonstrated a higher and significant resistance to anticancer agents at the concentrations of 5, 10 and 20 µM than the A172 cells. In other words, it was found in the glioma cell line that the chemoresistance was increased to a higher extent as the variation expression amounts of vimentin were higher.

Fig. 23B shows results of investigation regarding an influence of the downregulation of variations in expression levels of vimentin by siRNA on resistance to anticancer agents in the glioma cell line. This experiment was implemented by inoculating a 6-well plate with glioma cells at the rate of 10⁶ cells per well, followed by introduction of a vimentin siRNA or a control siRNA into the cells using lipofectamin 2000 after 24 hours of inoculation. In 24 hours after the introduction of vimentin siRNA, the glioma cell line was inoculated on a 96-well plate at the rate of 100 cells per well, and TMZ was added at concentration varying from 0 to 20 µM to each of the wells after 5 hours of inoculation. After the plate was incubated for 6 days, absorbance at 450 nm was measured by Western blotting. The rate of living cells was expressed by percentage (%) at each of the defined concentrations of TMZ by setting the concentration rate of 0 µM as a control and as a standard rate of living cells at the respective concentrations. The results of the absorbance analysis revealed that the treatment of the U373 cells with vimentin siRNA demonstrated a significant increase in sensitivity to anticancer agents at the concentrations of 10 and 20 µM compared with the treatment with the control siRNA. On the other hand, in the A172 cells, no upregulation of sensitivity to anticancer agents was recognized by the treatment with vimentin siRNA. In other words, a tendency was recognized in the U373 cells to the extent that the downregulation of the vimentin expression may cause the upregulation of sensitivity to anticancer agents, however, this tendency was not recognized in the A172 cell. This may be considered to occur that the influence of vimentin on the anticancer agents would be based not only on the expression variation amounts of vimentin but also on modifications of vimentin caused by intercellular circumstances preserved in a LOH- state.

Fig. 24 shows results of reviews on variations of an enzyme group modifying vimentin and a molecule group regulating the enzyme group as well as investigations regarding the activation of the modifications of vimentin in the LOH⁻ group due to the 1p/19q locus of the molecule group.
As a result of re-studies of the KeyMolnet analysis as well as the change rates of the molecule group and the significance difference, the results were obtained in this experiment as will be described below. First, by focusing on the molecules at locus 1p19q, it was found that calpain SS1, Ca²⁺/calmodulin-dependent protein kinase II (CaMKII), Cdc42, Rho, PI3K and G protein were located at the 1p/19q locus. Among those molecules, calpain SS1 was found upregulating significantly as an enzyme located in the 1p/19q locus and cleaving vimentin. As kinases phosphorylating vimentin, there were listed PAK1, ROCK and CaMKII, however, PAK1 demonstrated the highest variation rate by 2.04 times (p<0.05). As the molecule group activating PAK1, Cdc42 was found to demonstrate the highest upregulated variation rate by 3.00 times (p<0.01) and to be located at locus 1p. Moreover, as the molecule activating Cdc42, G protein and EphA4 were listed. G protein was found to be located at locus 1p, while EphA4 was upregulated most in the DNA microarray (14.9 times, p < 0.001).

From the above results, a listing was provided for the molecules that belong to the molecule group modifying vimentin and are located at locus 1p19q. The listing revealed that calpain SS1, CaMKII, Cdc42, Rho, PI3K and G protein were located at locus 1p/19q. Among them, it was suggested that calpain SS1 and Cdc42 as well as G protein may be involved. Moreover, it was suggested that EphA4An upregulating the largest change rate at the most upstream may be involved. The results further revealed that a fragmentation path composed of EphA4 (G protein) → Cdc42 → PAK1 → vimentin → phosphorylated vimentin → Calpain SS1 → vimentin fragmentation is activated.

Fig. 25 shows changes of 2D western blot patterns of vimentin, which may occur by the inhibition of phosphorylation of vimentin by PAK1 in the event that the glioma cells U373 were treated with a PAK inhibitor. The right-hand figure shows the event that an endogenous vimentin was observed by treatment of the cells U373 with the PAK inhibitor. The left-hand figure shows a negative control, wherein spots of cleaved vimentin were observed in addition to the endogenous full-length vimentin.

This experiment was carried out by inoculating a 6-well dish with U373 cells each with a reporter protein, i.e., a GFP-full length vimentin (1.4 kbp), introduced therein at the rate of 1 x 10⁵ cells per well and stimulating the cells with 10 µM of PAK18 (PAK inhibitor) and 10 µM of its negative control, respectively, after 72 hours of inoculation. After collection of the proteins, they were desalinated with a 2D clean-up kit and the resulting 2D pattern of vimentin was confirmed by Auto 2D. As a result, it was confirmed that the phosphorylation of the GFP-vimentin and the endogenous vimentin was inhibited by PAK18 in the glioma cells U373 and the cleavage of the endogenous vimentin was also found to be decreased. In other words, vimentin was found to cause a decrease in changes of spots by the phosphorylation of vimentin due to stimulation with the PAK inhibitor as well as cleavage of vimentin. This is considered that vimentin becomes likely to undergo phosphorylation and to be cleaved.

Fig. 26 shows the upregulated medicine sensitivity of the U373 glioma cells by treatment with the PAK inhibitor. This experiment was implemented to investigate an influence of the PAK1-induced vimentin phosphorylation on the sensitivity to anticancer agents in the glioma cell line. In this experiment, the glioma cell line was inoculated on a 96-well plate at the rate of 100 cells per well, followed by stimulation with the PAK inhibitor after 5 hours of inoculation and then addition of TMZ at concentrations varying from 0 to 20 µM. After incubation for 6 days, the survived cells were measured. The rate of living cells was expressed by percentage (%) at every set TMZ concentration by setting the TMZ concentration of 0 µM and absorbance for the stimulation with the PAK inhibitor as a standard living cell rate for the respective TMZ concentrations. The control at every TMZ concentration was compared with the stimulation with the PAK inhibitor. As a result, it was found that the glioma cells stimulated with the PAK inhibitor at the TMZ concentration of 5 µM and 10 µM caused a significant downregulation of resistance to anticancer agents. This indicates that the glioma cells acquire the chemoresistance to TMZ by the phosphorylation and fragmentation with PAK1.

Fig. 27 shows the fragmentation of vimentin in the glioma cell line U373. In this experiment, calpain was activated A23187 (a calcium ionophor). After the glioma cell line U373 was stimulated with the calpain inhibitor and then confirmed by Western blotting. It was confirmed that the calpain inhibitor inhibited the fragmentation of vimentin while A23187 enhanced the fragmentation of the vimentin. Therefore, it was confirmed that vimentin is cleaved with calpain.

Fig. 28 shows a relationship of the presence or absence of LOH with the cleavage of vimentin in AO/AOA samples. In this experiment, 38 AO/AOA samples were analyzed for vimentin by Western blotting. The vimentin bands were divided into band groups A to E from the top to investigate differences in expressions by the presence or absence of LOH. The results revealed that, in the LOH- group, vimentin upregulated the expression as a whole as well as the bands of cleaved vimentins also upregulated the expression. This suggests the possibility of worsening the prognosis of LOH⁻ patients by the upregulation of the vimentin expression and by the vimentin cleavage as well.

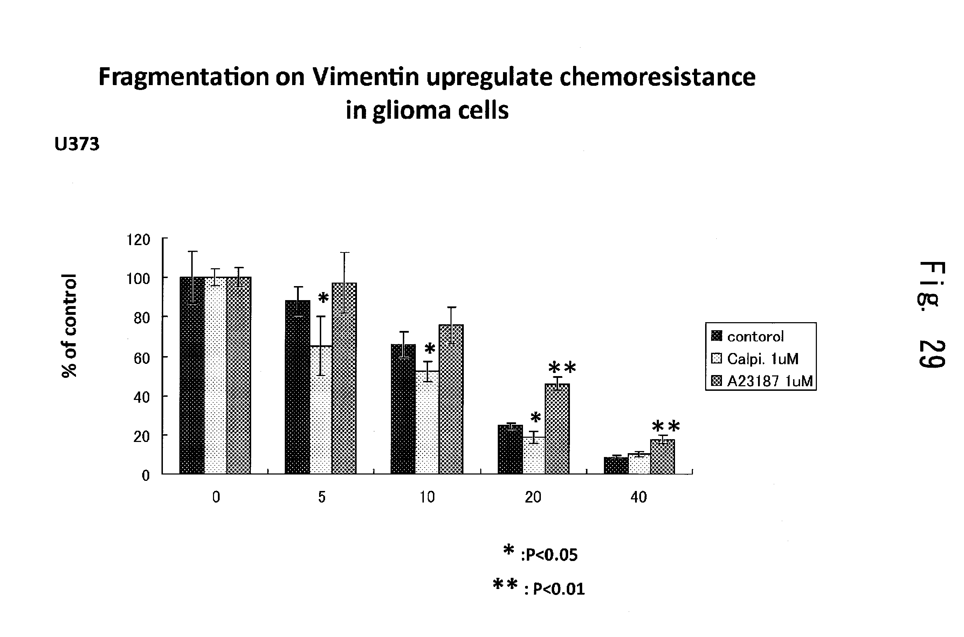

Fig. 29 shows the upregulation of the chemoresistance of the glioma cell line U373 by the fragmentation of vimentin. This experiment was implemented to investigate an influence of stimulation with the calpain inhibitor (Calpi) and stimulation with the calcium ionophor (A23187) on the resistance to anticancer agents.

This experiment was carried out by inoculating a 96-well plate with the glioma cell line at the rate of 100 cells per well, followed by stimulation with 1 µM of Calpi or 1 µM of A23187 after 5 hours of inoculation and addition of TMZ at concentrations varying from 0 µM to 40 µm. After the glioma cell line was incubated for 6 days, the portion having absorbance at 420 nm was measured. The rates of living cells were expressed by percentage (%) at every TMZ concentration by setting the TMZ concentration of 0 µM as a control and setting absorbance at the stimulation with Calpi and A23187, respectively, as a standard for the living cell rate at the respective TMZ concentrations. The control at every TMZ concentration was compared with the stimulation with Calpi andA23187. As a result, it was found that the glioma cells stimulated with Cai at the concentrations of 5, 10 and 20 µM downregulated the resistance to anticancer agents significantly while the glioma cells stimulated with A23187 at the concentrations of 20 and 40 µM upregulated the resistance to anticancer agents significantly. This reveals that the glioma cells acquire the resistance to TMZ by fragmentation of vimentin with calpain.

In order to verify a biological role of the fragmented vimentins, the experiment was implemented, first, to investigate a localization of the vimentin fragments using the U373 cells and the A172 cells. The U373 cells and the A172 cells were incubated on a 6-well plate, respectively, washed with PBS, and fixed with 4% paraformaldehyde. After washing three times with PBS, the cells were treated with 0.1% Triton X-100™ PBS for 5 minutes and blocked with 3% BSA PBS for 20 minutes. After they were washed two times with PBS, an anti-N-terminal vimentin antibody and an anti-C-terminal vimentin antibody, each diluted with 3% BSA PBS, were added, and the mixture was reacted at room temperature for 60 minutes. Further, after they were washed another two times with PBS, Alexa 488-labeled anti-mouse secondary antibody and Alexa 546-labeled anti-rabbit secondary antibody, each diluted with 3% BSA PBS, were added, followed by proceeding with a reaction at room temperature for 60 minutes. After the completion of the reaction, they were washed two times with PBS and a state of the intercellular localization of vimentin was observed with a confocal microscope. It was thus observed that, when the C-terminal vimentin antibody was used, only the cytoplasm of both of the U373 and A172 cell was stained. On the other hand, it was observed that, when the N-terminal vimentin antibody, both of the U373 and A172 cells were stained at their cytoplasm and, surprisingly, in the nuclei in a spot shape (Fig. 30). The staining of the cell nuclei with the anti-N-terminal vimentin antibody suggests the nuclear translocation of the N-terminal vimentin fragments into the cellular nuclear.

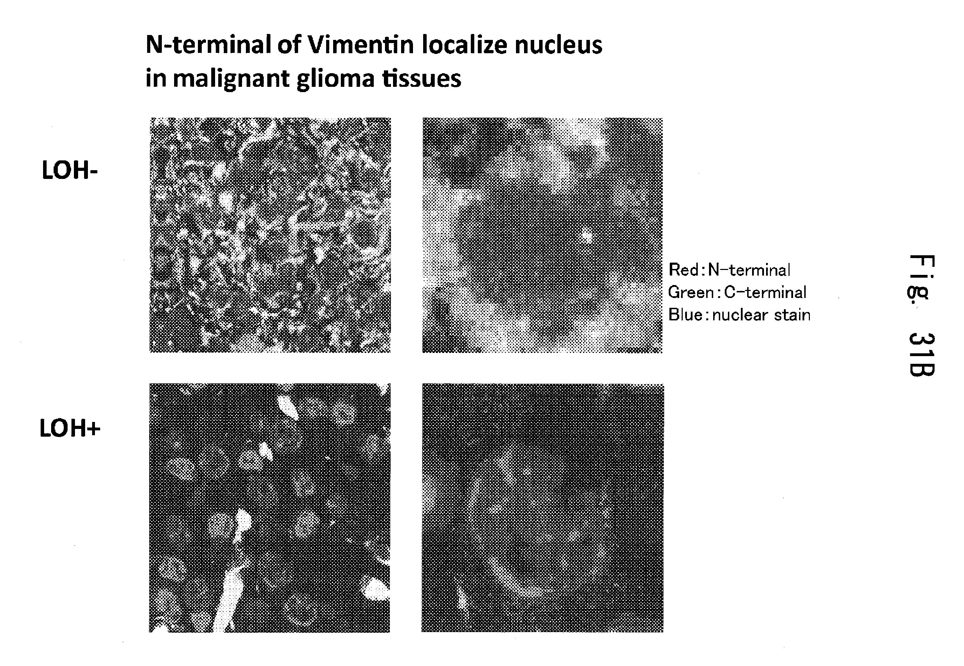

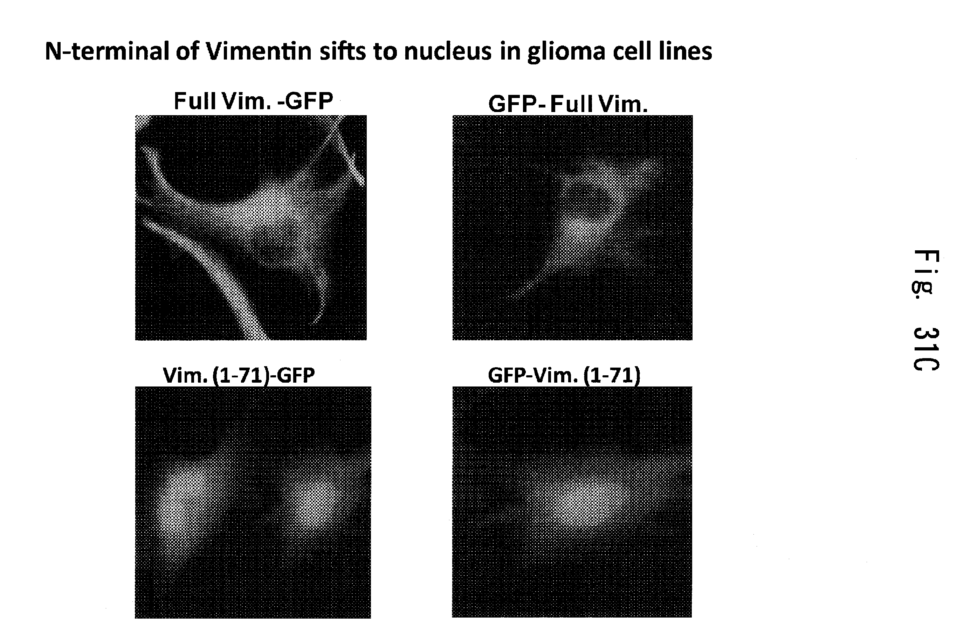

Fig. 31A shows the event of the nuclear translocation of the N-terminal vimentin fragments into the nucleus of glioma cell. Fig. 31B shows the nuclear localization of the N-terminal vimentin fragments in the nucleus of malignant glioma cell. Fig. 31C shows the event of the nuclear translocation of the N-terminal vimentin fragments to the nucleus of the glioma cell.

The localization of the N-terminal vimentin in the glioma cell line and the AO/AOA tissues was investigated. As the glioma cells were stained with an anti-C-terminal vimentin antibody and an anti-N-terminal vimentin antibody, it was confirmed that the inner nuclear portion of the glioma cell was stained in a granular shape with the anti-N-terminal vimentin antibody. This reveals that the N-terminal vimentin is translocated into the nucleus of the glioma cell (Figs. 30 and 31A). A strong reaction with the anti-N-terminal vimentin antibody was recognized in the LOH⁻ tissues (Fig. 31B).

It was further observed by expressing reporter proteins in the glioma cells, i.e., the reporter proteins including, for example, GFP-vimentin (full length), GFP-N-terminal vimentin (71a.a), vimentin (full length)-GFP, and N-terminal vimentin (71a.a). The results of observations revealed that the specific nuclear translocation of the N-terminal vimentin fused with the GFP protein into the cellular nuclear was observed (Fig. 31C).

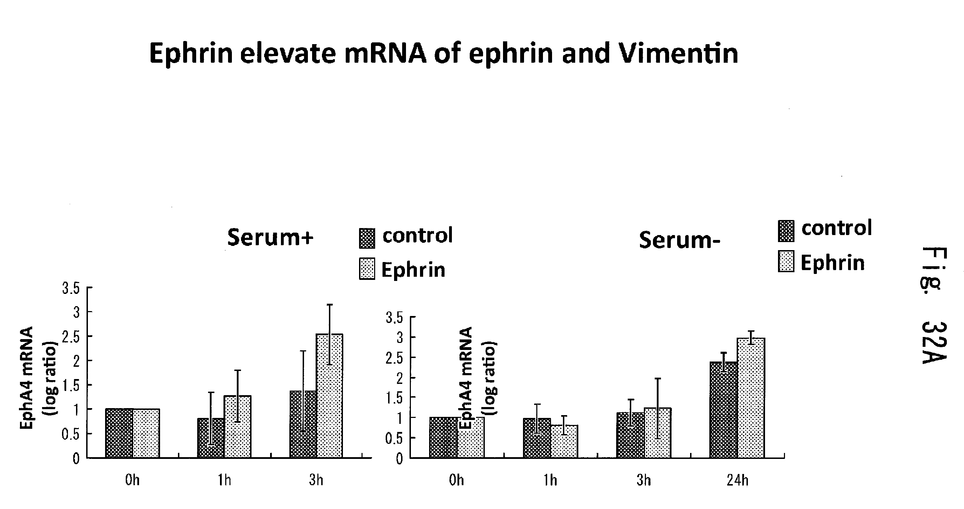

Fig. 32A shows results of confirmation that the N-terminal vimentin upregulated the expression of EphA4 mRNA in the glioma cell line. A vector for expressing the GFP-N-terminal vimentin (71a.a) was introduced into the U373 cells at the rate of 0.5 µg per 1 x 10⁵ cells by electroporation. The cells were then inoculated on a 12-well plate at the rate of 1 x 10⁵ cells per well. In 0, 1, 3, 6, 9, 12, 18 and 24 hours after inoculation, mRNA was collected and the amounts of variations in expression levels of EphA4 mRNA were quantitated by qRT-PCR. The results confirmed that the N-terminal vimentin upregulated EphA4 mRNA.

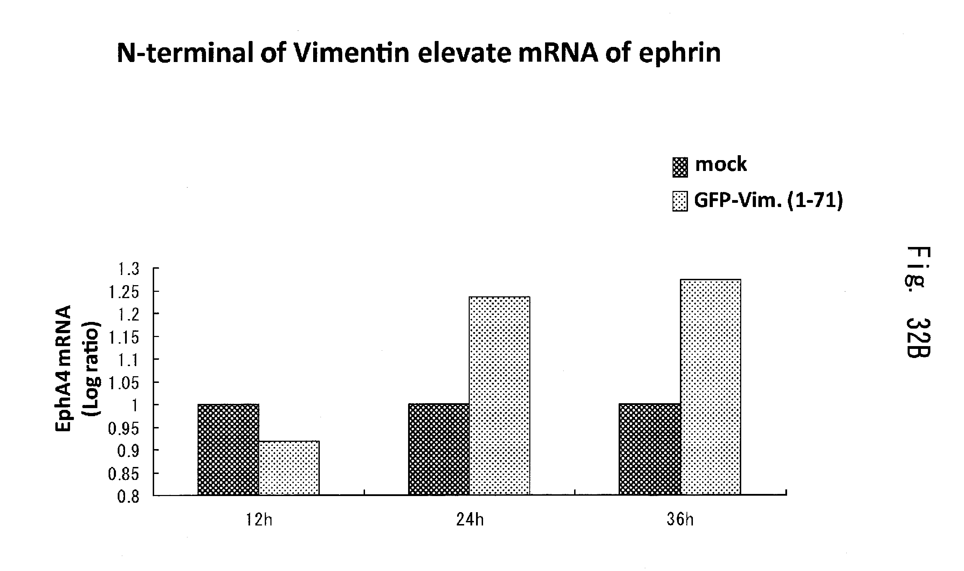

Fig. 32B shows an influence of stimulation by ephrin A1 (a ligand of the EphA4 receptor) on EphA4 mRNA using the U373 cells. The cells were inoculated on a 12-well plate at the rate of 2 x 10⁴ cells per well, and ephrin A1 (an end concentration: 3 µg/ml) was added after overnight incubation. After incubation for 0, 1, 3 and 24 hours, mRNA was collected and the amounts of variations in expression levels of EphA4 and vimentin mRNA were quantitated by qRT-PCR. The results confirmed that ephrin A1 upregulated EphA4 mRNA.

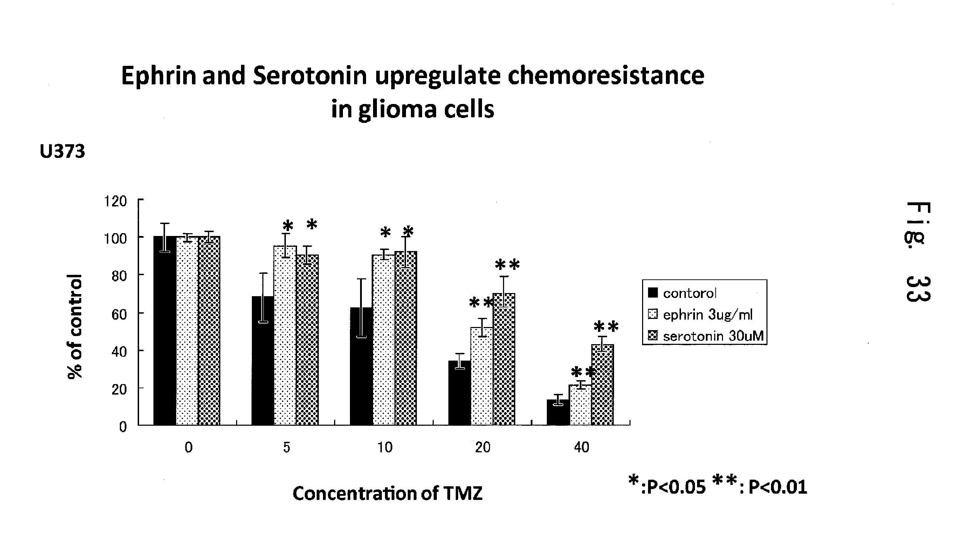

Fig. 33 shows an influence of ephrin receptor and serotonin stimulation on the resistance to anticancer agents in the glioma cell line. The results revealed that the ephrin receptor and serotonin upregulated the medicine resistance of the U373 glioma cells.

In this experiment, the glioma cell line was inoculated on a 96-well plate at the rate of 100 cells per well, followed by stimulation with 3 µg/ml of EphA4 or 30 µM of serotonin and addition of TMZ at concentrations varying from 0 µM to 40 µM after 5 hours of inoculation. After the cells were incubated for 6 days, the survived cells were measured. The rate of living cells was expressed by percentage (%) at every TMZ concentration by setting the TMZ concentration of 0 µM as a control and as a standard living cell rate at absorbance of the ephrin A1 stimulation and the serotonin stimulation, respectively, for the respective TMZ concentrations. The control at every TMZ concentration was compared with the ephrin A1 stimulation and the serotonin stimulation. The results revealed, as shown in the drawing, that the sensitivity to anticancer agents was upregulated at the TMZ concentrations of 5, 10, 20 and 40 µM by the ephrin A1 stimulation and the serotonin stimulation in the glioma cell line.

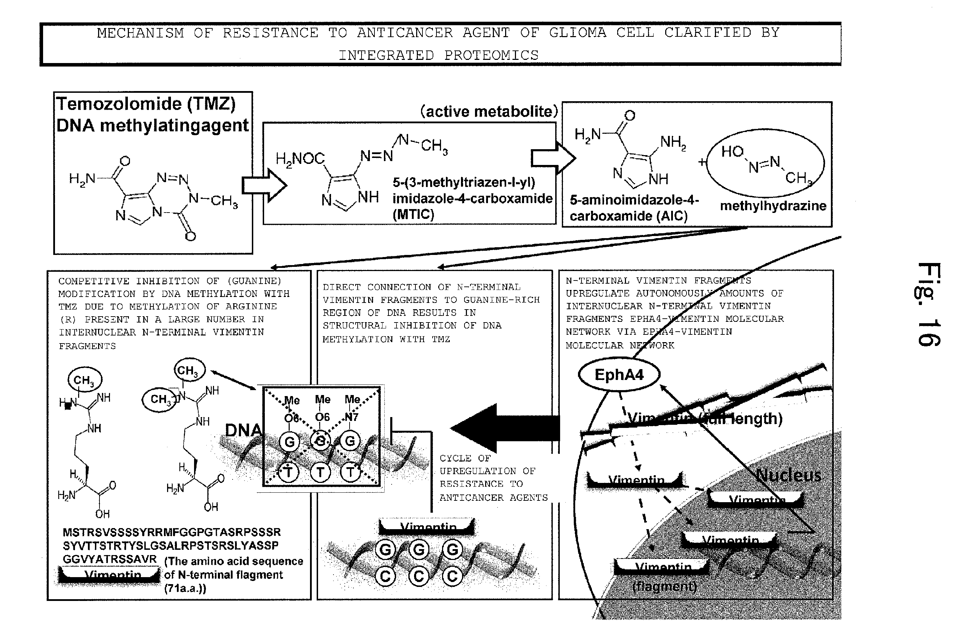

As shown in Figs. 15 and 16, attention was paid particularly to PAK1 (p21 activated kinase 1) and calpain SS1, each with the modified vimentin site identified, among the metabolic products obtained by the metabolism of TMZ in the glioma cells. As the molecule linking to the PAK1 activating factor at the upstream, it was confirmed by the analysis by Western blotting using an inhibitor that the expression levels of Cdc42 and EphA4 were upregulated significantly and an activation cascade of the molecules linking to each signal was activated in the glioma cell significantly. Finally, it was found that vimentin in the filament form was phosphorylated to be converted to a stable soluble form, thereby becoming likely to be cleaved specifically, and an enzyme responsible for the cleavage was calpain. More interestingly, the N-terminal vimentin cleaved specifically was found to be translocated into the nuclei of glioma cells.

The integrated proteomic analysis according to the present invention clarified the mechanism of the resistance of the glioma cells to medicine. By taking an anticancer agent, TMZ, as an example, the mechanism of TMZ will be described hereinafter. TMZ is metabolized to 5-(3-methyltriaz-1-en-1-yl)imidazole-4-carboxamide (MTIC) and then to 5-amino-imidazole-4-carboxamide (AIC) and methylhydrazine. It was thus considered that these metabolic products methylated arginine (R) present in the intranuclear N-terminal vimentin fragments in larger amounts, thereby competitively suppressing the DNA (guanine) methylation modification by TMZ. As the N-terminal vimentin fragments can be linked directly to a guanine-rich DNA region, it may be anticipated that the methylation of DNA is suppressed structurally by TMZ (Fig. 16).

Moreover, the EphA4-vimentin molecular network revealed that the N-terminal vimentin fragments increased an amount of the intranuclear N-terminal vimentin fragments in glioma cells autonomously, thereby upregulating the resistance to medicine.
This analysis made it clear that there was a vimentin signal that was activated in the glioma cells resistant to medicine. In particular, it was further confirmed that Cdc42 was activated through EphA4 outside the cells and vimentin was then phosphorylated by the activation of PAK1, thereby cleaving the resulting phosphorylated vimentin in a soluble form with calpain. Moreover, the cleaved N-terminal vimentin fragments were translocated into the cellular nuclei.

By the verification experiment by Western blotting using the inhibitor, it was confirmed that the activation cascade linking to each signal was activated significantly in the glioma cells. Thus, it was finally confirmed that vimentin in a filament form was phosphorylated and converted to a stable soluble form that was likely to be cleaved specifically and, further, the enzyme responsible for cleavage was calpain. More interestingly, the N-terminal vimentin fragments cleaved specifically were confirmed to be translocated into the nuclei of glioma cells. In another words, it was confirmed that, in the event that the GFP-full length vimentin and the GFP-N-terminal vimentin were expressed in the glioma cells, the GFP-full length vimentin was expressed in a fibrous form over the whole areas of cytoplasm while the GFP-N-terminal vimentin was translocated significantly into the cellular nuclei. In the clinical slice of the LOH⁻ AO glioma specimen, it was confirmed that the N-terminal vimentin fragments were accumulated significantly (Figs. 31Bs and 31C).

### Example 3:

In this example, there were used cell lines derived from two kinds of cells (SQUU-A cells and SQUU-B cells), respectively, which were collected from tongue cancer tissues at local recurrence sites of Japanese women and had properties different from each other. The SQUU-A cells were low-metastatic cancer cells without causing expansive proliferation and intravascular infiltration as well, while the SQUU-B cells were high-metastatic cancer cells with expansive proliferation and intravascular infiltration as well. The SQUU-B cells grow in a more predominant way under mixed culture conditions than the SQUU-A cells.

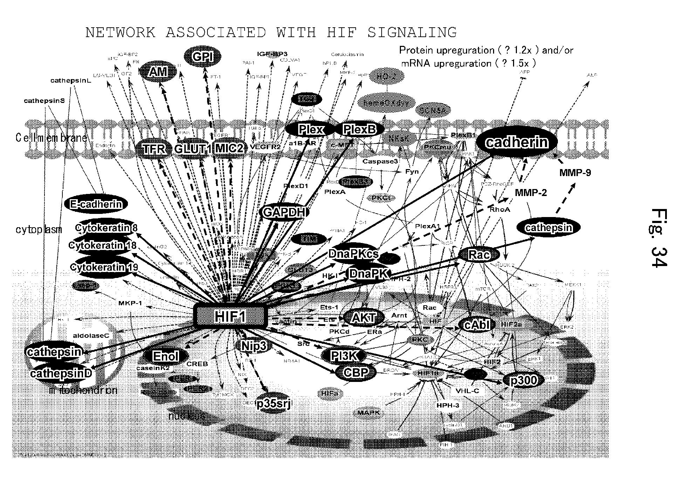

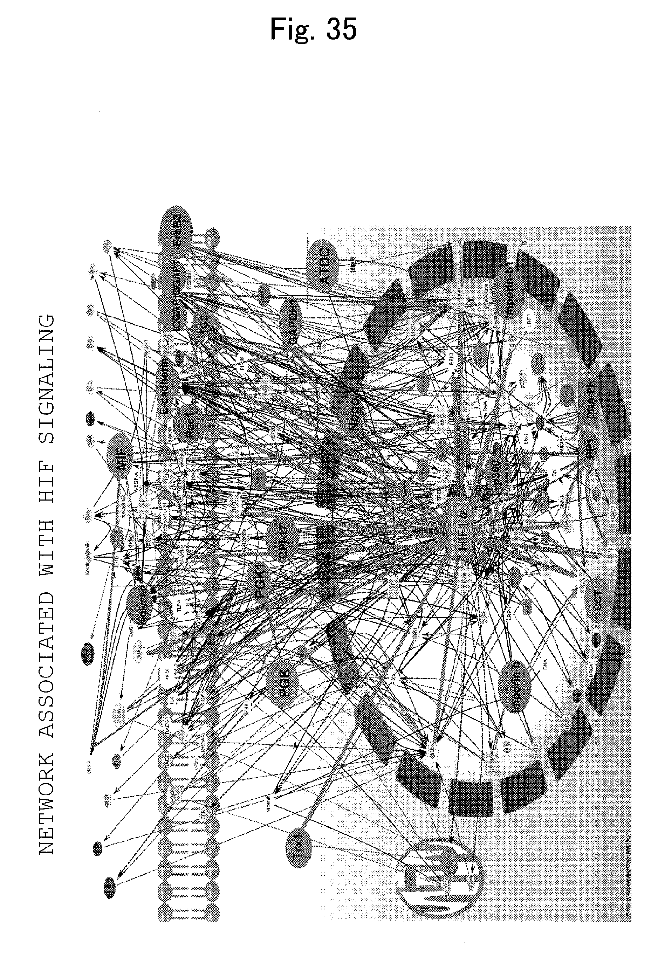

In the SQUU-B cells, the molecules which upregulated their expression levels, that is, which were considered to be involved in metastaticity or grew predominantly under mixed culture conditions under which the SQUU-A cells were mixed therewith, were selected for analyses using the proteomic analysis and the DNA microarray analysis methods. The results of the proteomic analysis confirmed that the expression of 41 proteins and 2,665 mRNAs were upregulated significantly. These data were analyzed by MANGO and iPEACH in the manner as described above, thereby ranking paths involved in the upregulation of the protein expression levels in the SQUU-B cells using the KeyMolnet analysis. The results of the KeyMolnet analysis clarified a path through HIF-1 (Hypoxia Inducible Factor 1). In other words, it was confirmed that the 41 molecules were recognized as upregulating the protein expression at high levels in the high-metastatic line of the SQUU-B cells and 32 molecules out of the 41 molecules contained a HIF signal network system. Moreover, in many SQUU-B cells, it was confirmed that signals generated from many highly upregulated proteins present upstream of HIF-1 were linked to HIF-1 through this network and many highly upregulated proteins present downstream received signals from HIF-1 (Figs. 34 and 35).

In order to investigate an influence of HIF-1 on the in vivo predominant growth of metastatic cancer cells in a mass of cancer cells, studies were performed regarding the states of expression and activation in the metastatic SQUU-B cells. At this end, reviews were made using a 3-DCC (3-Dimensional Cell Culture) system regarding the possibility of an occurrence of the in vivo phenomenon in an in vitro circumstance. The 3-DCC system used was an experimental system in which each well of a 96-well plate was injected with 2% Matrigel and the SQUU-A cells, the SQUU-B cells, and a mixture thereof were inoculated, respectively, at the rate of 2 X 10⁴ cells per well and then incubated.

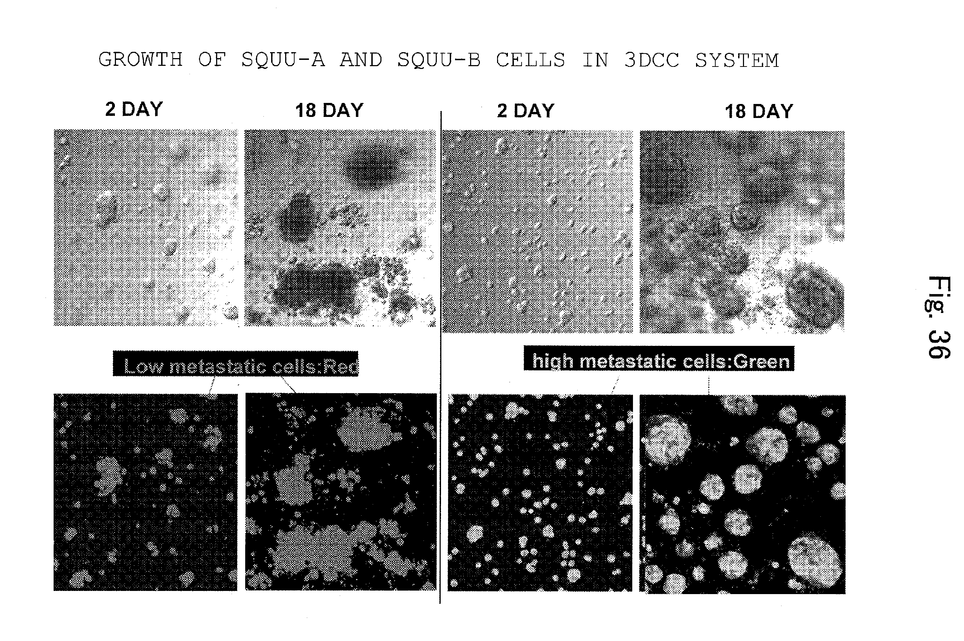

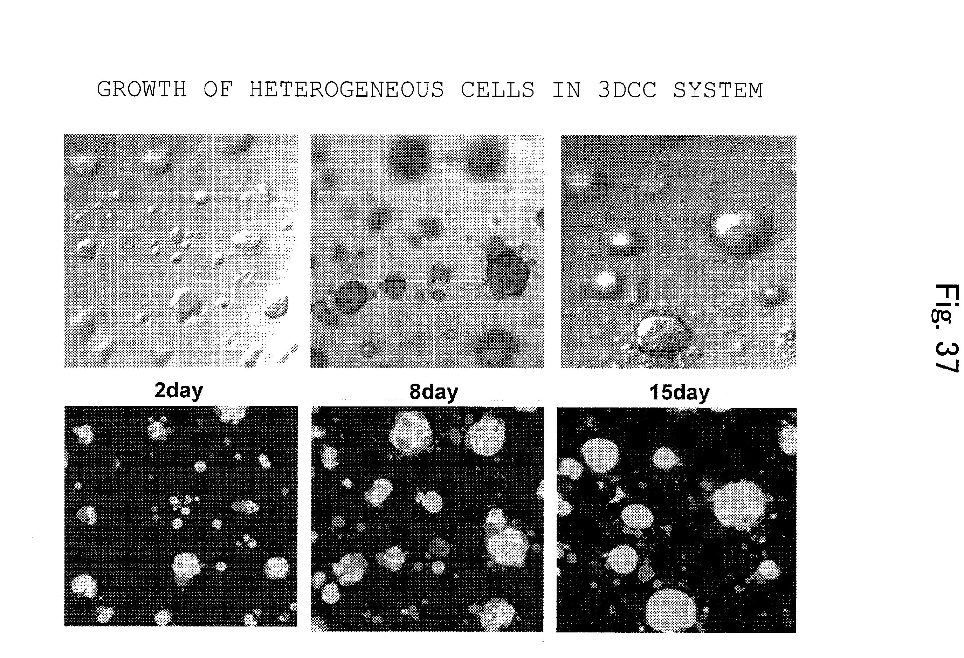

The temporal observations of the experimental systems revealed that, in the experimental system where the SQUU-A cells and the SQUU-B cells were each incubated solely, each of the cells formed a large cell aggregation, but the SQUU-B cells formed a globular cell cluster in which the cells adhered together to each other with a stronger binding force than the SQUU-A cells (see Fig. 36). In the experimental system in which a mixture of the SQUU-A cells with the SQUU-B cells was incubated, the SQUU-A cells decreased and changed the localization from the central portion of the cell cluster toward the peripheral portion as time elapsed, while the SQUU-B cells grew predominantly than the SQUU-A cells (Fig. 37).

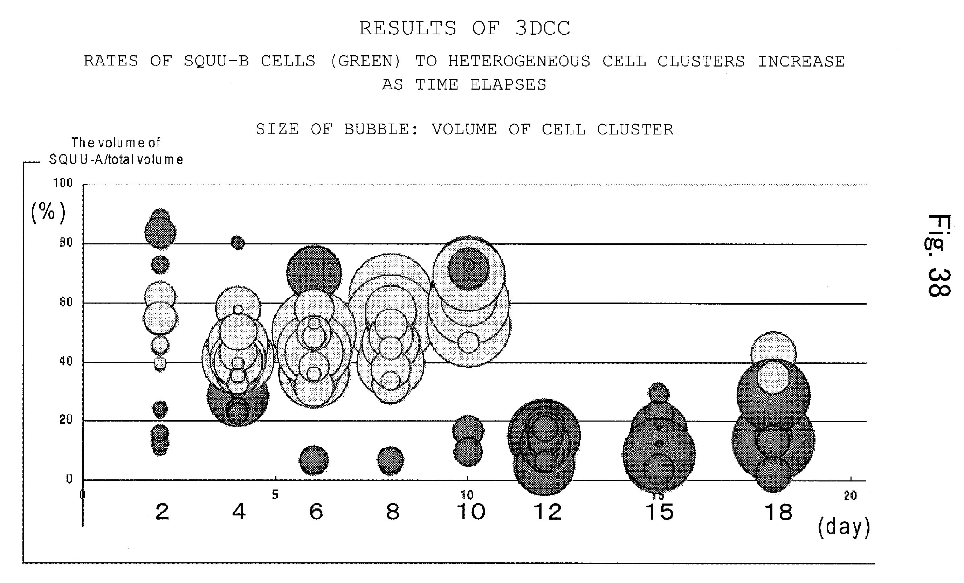

The cell clusters obtained in the experimental system, accordingly, wherein the mixture of the SQUU-A cells with the SQUU-B cells was incubated, were measured for a volume ratio using a confocal laser microscope. The results of the laser microscopic observations showed that a ratio of the SQUU-B cells to the SQUU-A cells was increased, while a ratio of the SQUU-A cells to the SQUU-B cells was decreased, as time elapsed (see Fig. 38).

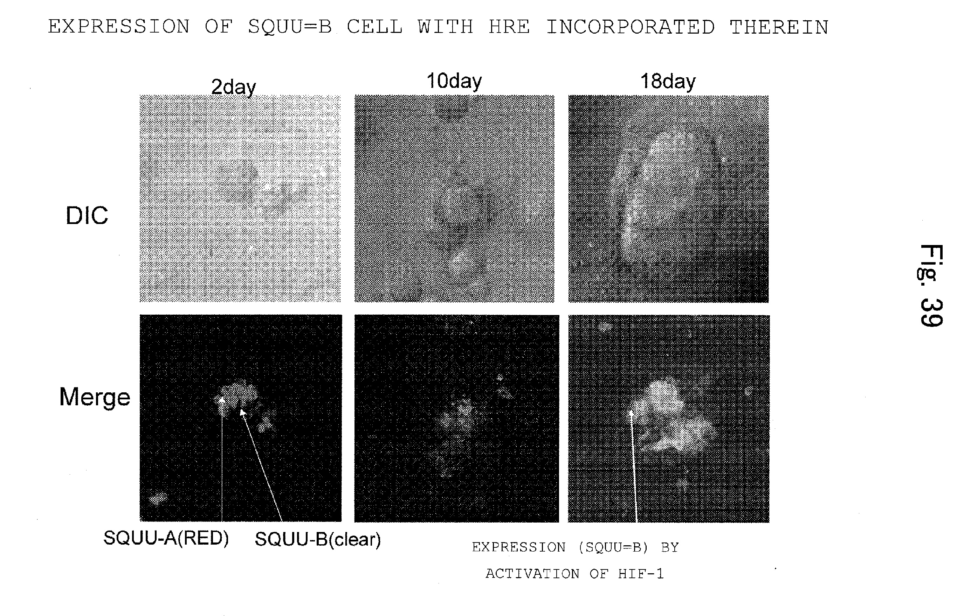

On the other hand, reviews were made regarding the molecules exerting an influence of HIF-1 upon metastaticity of cancer cells. In order to evaluate a HIF-1 activated state of the cell aggregation in Matrigel by 3-DCC, the cells were introduced each with a vector having a GFP reporter gene (GFP-HRE: HRE, Hypoxia Responsive Element) containing a gene sequence generating an illumination of GFP by HIF-1 and the intercellular HIF-1 activated state was then analyzed. The results of evaluation revealed that the GFP expression was recognized in the cell aggregation of the SQUU-B cell at its central portion while the GFP expression was not recognized in the SQUU-A cells. A difference in a response to HIF-1 between the SQUU-A cells and the SQUU-B cells may be considered to be involved in the predominant growth of the SQUU-B cells (Fig. 39).

Next, in order to evaluate a difference in metastaticity between the SQUU-A cells and the SQUU-B cells, a comparison was made of the expression of HIF-1 and cadherin (E-cadherin) by immunocytochemistry (ICC). In this experiment, it was found that HIF-1 suppressed the expression of cadherin at the site at which HIF-1 was accumulated and activated, when cobalt chloride was used to suppress the decomposition of HIF-1.

The above experimental results also revealed that the expression of HIF-1 in the high-metastatic cancer cells (the SQUU-B cells) was accompanied gradually by the progress of tumor proliferation, while no expression of HIF-1 was shown in the low-metastatic cancer cells (the SQUU-A cells). It was further observed that the numbers of the SQUU-A cell were decreased gradually and the localization of the cell aggregation shifted from the central portion toward the peripheral portion as time elapsed, whereas the numbers of the SQUU-B cell were increased as their growth advanced. Moreover, the expression of cadherin (E-cadherin) was downregulated as the expression of HIF-1 was upregulated in the SQUU-B cells. This suggests that HIF-1 would be involved in the predominant growth of the high-metastatic cancer cells (the SQUU-B cell) and the decrease in the cell numbers of the low-metastatic cancer cells (the SQUU-A cell). Furthermore, it may be considered that the cells having a high metastaticity caused an epithelial mesenchymal transformation (EMT) by regulating the expression of an adhesion molecule by changes of circumstances.

### Example 4:

As described above, the signaling network obtained by the KeyMolnet was analyzed by the iPEACH program. As shown in Fig. 11, it was confirmed that the network extracted from the data of the proteins with their expression levels upregulated in the LOH⁻ group was concentrated to a higher extent than that in the LOH⁺ group. From this network, vimentin was found as a modified core protein that upregulated the protein expression variation amount to the highest extent. More specifically, from two to twelve spots were detected by the 2D-DIGE analysis and the rates of the upregulated protein expression variation amounts were increased by 1.6 times to 2.84 times. Further, five fold changes were identified from 12 protein spots having 34 kinds of modifications and upregulating their protein expression variation amounts by mass spectrometry (4.5 times by ESI and 2.6 times by MALDI). It was also found that a group of the enzymes responsible for modifications was linked to some kinases, kinase activators (e.g., PAK1, P13K, PKC, Rho, Cdc42, etc.) or proteases (e.g., calpain, etc.). It was further found, more interestingly, that the gene locus of the responsible enzyme group on the chromosome was localized at 1p and 19q and EphA4 was identified as the molecule that was located at the uppermost upstream of these networks. Moreover, this EphA4 was found by the DNA microarray analysis that it upregulated its protein expression variation amount by as much as 14 times in the LOH⁻ tissues. The EphA4 was also found to be related to the upregulation of the expression levels of Cdc42 and the facilitation of the activation of PAK1 (p21 activated kinase 1), thereby resulting in phosphorylation of vimentin. The phosphorylated vimentin is also known as a target of the protein decomposition by calpain. Moreover, calpain small subunit 1 (CAPNS1/calpain 4) which was an inhibitory subunit of calpain known as a protease of vimentin was identified in the protein group upregulating variations in their protein expression levels significantly in the LOH- tissues. This protein was also found to be located at the 19q locus on the chromosome.

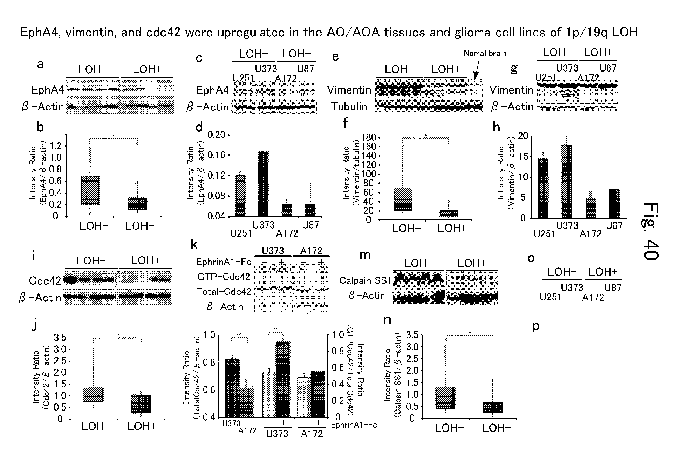

A description will be made hereinafter regarding the activation of the vimentin-EphA4 network in 1p/19q LOH- AO/AOA tissues and glioma cell line.
An investigation was performed for a molecular network composed of EphA4 (EphA4), Cdc42, PAK1, calpain and vimentin, which was extracted and identified in a particular network upregulating a variation in expression levels in the 1p/19q LOH-AO/AOA tissues. The expression levels of EphA4 and vimentin in the 1p/19q LOH- and LOH⁺ AO/AOA tissues as well as the glioma cell lines (1p/19q LOH⁻: U-251 and U373; 1p/19q LOH⁺: A172 and U87MG) were quantitated by Western blotting using a particular antibody to each of those molecules. The average expression level of EphA4 in the LOH- tissues was higher by 2.5 times than that in the LOH⁺ tissues (see Fig. 40a, b). The average expression level of EphA4 in the U251 and U373 cells was higher by 2.3 times than that in the A172 and U-87MG cells (see Fig. 40c, d). Similarly, the average expression level of vimentin in the LOH- tissues was higher by 3.1 times than that in the LOH⁺ tissues (see Fig. 40e, f). Moreover, the average expression level of vimentin in the LOH- glioma cells was higher by 2.7 times than that in the LOH⁺ glioma cells (see Fig. 40g, h). In addition to the higher upregulation of the vimentin expression levels, interestingly, a particular vimentin-decomposed fragment was observed in the cell line of the 1p/19q LOH- tissues (Figs. 40e, g).

The average expression level of Cdc42 in the 1p/19q LOH- tissues was higher by 1.2 times than that in the LOH⁺ tissues, and the average expression level of Cdc42 in the 1p/19q LOH⁻ glioma cell line was higher by 1.4 times than that in the LOH⁺ glioma cell line (see Fig. 40i, j). Further, in order to investigate an increase in the activation of Cdc42 via EphA4, the level of activating Cdc42 was analyzed after stimulation with ephrin A1 which was a ligand of EphA4. The results revealed that the Cdc42 activation level was higher by 1.6 times on average in the U373 cells than in the U172 cells after stimulation with ephrin A1 (Fig. 40k, 1).

The above results suggest that Cdc42 is caused to upregulate its expression level specifically and activated via the EphA4 signal in the 1p/19q LOH- glioma cell line. The verification experiment conducted concurrently herewith revealed that the specific expression level of calpain SS1 (CAPNS1; calpain small subunit 1) was upregulated, too, in the 1p/19q LOH- glioma cell line (Figs. 40m, n, o, p). These results further suggest that the specific expression levels of EphA4, Cdc42, calpain and vimentin were upregulated in the 1p/19q LOH- glioma cell line.

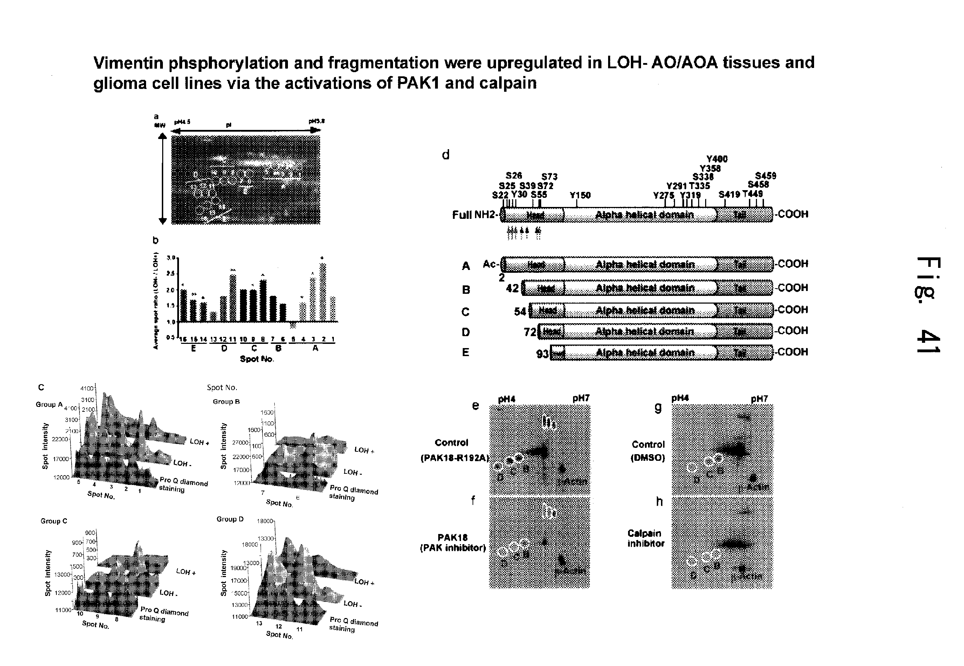

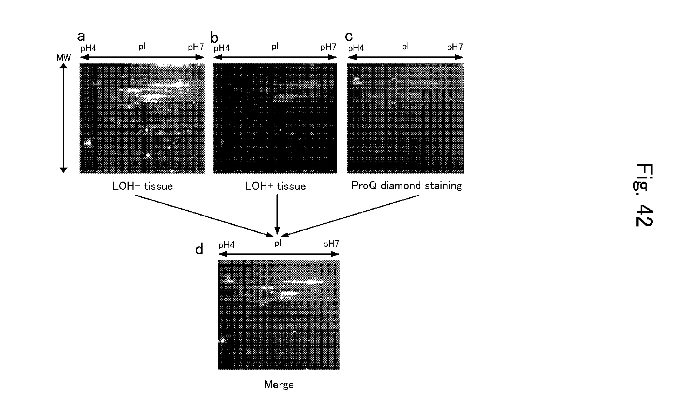

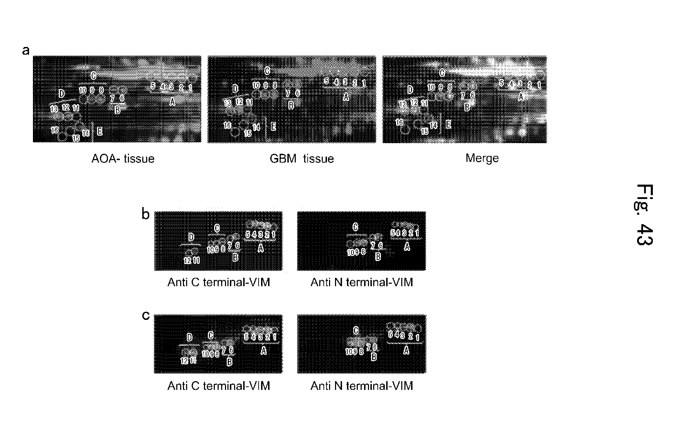

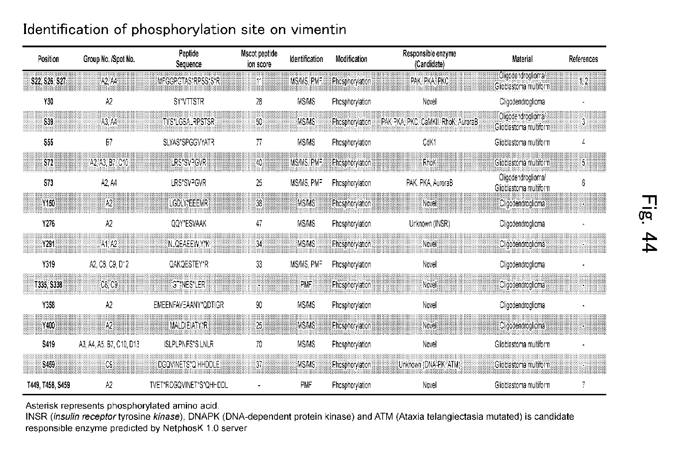

Next, in order to confirm an occurrence of phosphorylation and fragmentation of vimentin by the activation of PAK1 and calpain, respectively, in the 1p/19q LOH⁻ AO/AOA tissues, the spots of the vimentin-related proteins identified by the 2D-DIGE analysis using ProQ Diamond were analyzed by Western blotting. Among ca. 4,000 spots appeared on a two-dimensional gel (pH: 4-7), a total number of 16 spots (pH: 4.7 - 5.1; MW: 37 - 51 KDa) was identified as vimentin spots (Figs. 41a, Fig. 2). Among the 16 vimentin spots, 9 spots of the 1p/19q LOH- AO/AOA tissue group demonstrated intensities higher than those of the 1p/19q LOH⁺ tissue group (see Fig. 41b). Interestingly, 13 spots out of the 16 vimentin spots translocated to the acidic side were positive to ProQ Diamond and they were the phosphorylated vimentin spots (Fig. 42). These spots were divided into five groups by molecular weight: group A: 51 KDa; group B: 48 KDa; group C: 45 KDa; group D: 41 KDa; group E: 37 - 39 KDa (see Figs. 41a, b). In substantially the same manner as the vimentin spots of the AO/AOA tissues, vimentin spots derived from glioblastoma (GBM) tissues were analyzed similarly by the mass spectrometry analysis and the N-terminal amino acid sequence analysis. The results of these analyses revealed that the primary structures of all of the 16 vimentin spots of the GBM tissues were almost identical to those of the AO/AOA tissues (Fig. 41d). The 16 vimentin spots detected in the GBM tissues and the AO/AOA tissues showed a completely identical pattern obtained by the 2D-PAGE analysis and Western blotting (Figs. 43a - c). Therefore, it was confirmed that the phosphorylation and the fragmentation of vimentin in the AO/AOA tissues were almost identical to those in the GBM tissues. In addition thereto, 21 different phosphorylated sites including Ser26, Ser27, Ser39 and Ser73, which were known as sites modified with PAK1, were identified by the MS/MS analysis (Fig. 44).

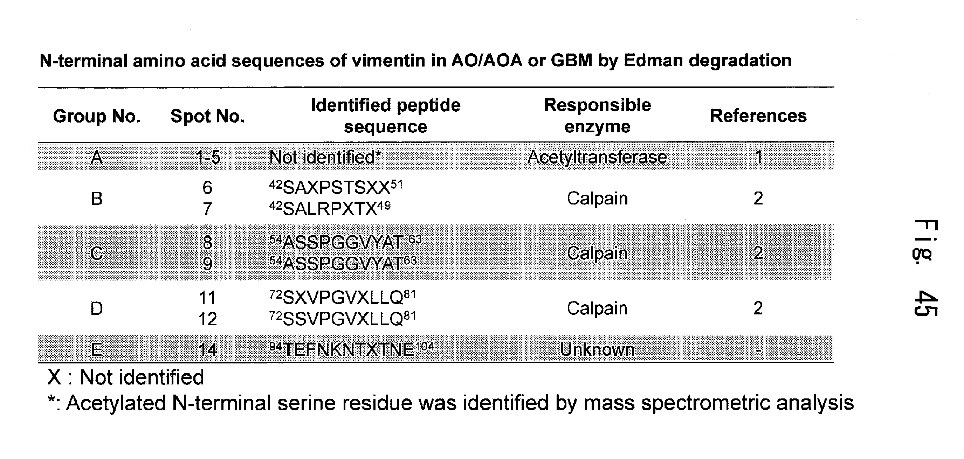

It is known that, although the N-terminal serine residue of group A can be acetylated, the N-terminal amino acid sequence of the vimentin fragments of groups B, C and D, respectively, can be cleaved by calpain (Figs. 41d, 45). Therefore, in order to confirm the phosphorylation of vimentin and the cleavage of the N-terminal vimentin with PAK1 and calpain, respectively, a pattern of the vimentin spots with GFP-vimentin (1-464.aa full length) overexpressed in the U373 glioma cell line was analyzed by 2D Western blotting using an anti-C-terminal vimentin antibody. The results revealed, interestingly, that PAK18, a PAK inhibitor peptide, suppressed not only the phosphorylation of the GFP-vimentin but also the fragmentation of vimentin by an action of the protein decomposition of both of the overexpressed vimentin and the endogenous vimentin (Fig. 25). Further, the particular fragmentation of vimentin was suppressed with the calpain inhibitor (Fig. 27). These results suggest that vimentin is phosphorylated with PAK1, followed by fragmentation with calpain.

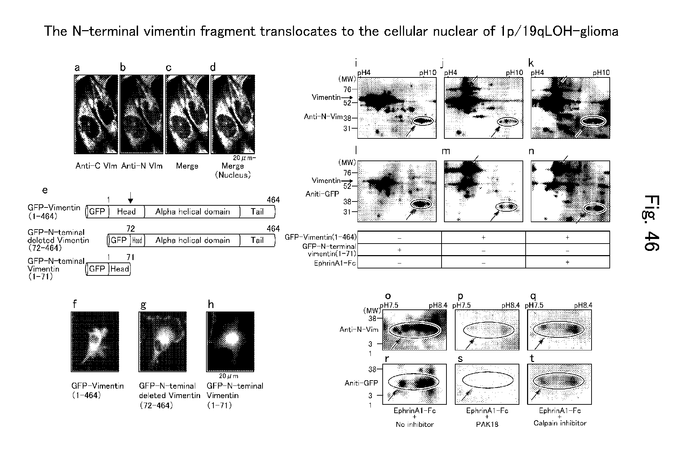

A description will now be made regarding the cellular nuclear translocation of the N-terminal vimentin fragments into the nuclear of the 1p/19q LOH⁻ AO/AOA glioma cell line.
The observations by the 2D-DIGE analysis revealed that the p-value of an intensity ratio of the spot D11 of the vimentin fragment (Vim: 72-464) was the lowest among all the vimentin spots (A1- E16) of the LOH⁻ cells and the LOH⁺ cells. Therefore, changes of functions of the vimentin fragments in the LOH⁻ cells were investigated using an anti-vimentin antibody, which recognized the N-terminal vimentins (H84: 1-84 terminal amino acids) but did not react with the vimentin spots D11 - E16, and an anti-C-terminal vimentin antibody (V9: around amino acid 417; anti-C-terminal Vim). As a result, it was confirmed that the vimentin fragments were localized in the cytoplasm and nuclei of the LOH- U373 glioma cells. Although both of the antibodies recognized vimentin skeletal filaments in the cytoplasm (Figs. 46a - d), only the anti-N-terminal vimentin antibody (H84) recognized the nuclear region of the cell (Figs. 46b - d). These results revealed that the cellular nuclear translocation of the N-terminal vimentin fragments was caused to occur after fragmentation of vimentin. In order to confirm this cellular nuclear translocation of the vimentin fragments, a vimentin- overexpressed plasmid vector containing a GFP-N-terminal vimentin fragment (1-71), a GFP-C-terminal vimentin fragment (72-464) and a GFP-fused full length vimentin, respectively, was constructed (Fig. 46e) and then introduced into the U373 glioma cells to induce the plasmids therein. The results revealed, as anticipated, that the GFP-C-terminal vimentin fragment and the GFP-fused full length vimentin were overexpressed in the cytoplasm region, inducing fibrogenesis, like the endogenous vimentin (Figs. 46f, g). The GFP-N-terminal vimentin fragment, however, was translocated significantly into the cellular nuclear region (Fig. 46h). Moreover, in order to verify this biologically, the GFP-full length vimentin was overexpressed and the nuclear protein was prepared from the U373 cells, followed by implementing Western blotting using the anti-N-terminal vimentin antibody H84 and anti-GFP antibody. The results revealed, as shown in Figs. 46i to Fig. 46n, that the spot representing the N-terminal vimentin fragment was identified with both of the antibodies as a nuclear fraction (pI 7.6 - 8.3; MW: 33 KDa) (Fig. 46o - Fig. 46t; indicated by arrow). Further, interestingly, the N-terminal vimentin fragments identified in the nuclear fraction were increased by stimulation with ephrinA1-Fc (Figs. 46j, k, m, n), while they were caused to disappear by stimulation with PAK18 (Figs. 46p, s) and with the calpain inhibitor (Figs. 46q, t). These results revealed that the calpain-induced fragmentation of the vimentin phosphorylated with PAK1 was upregulated in the LOH- glioma cells and then the N-terminal vimentin fragments were readily translocated into the nuclei of the LOH⁻ glioma cells.

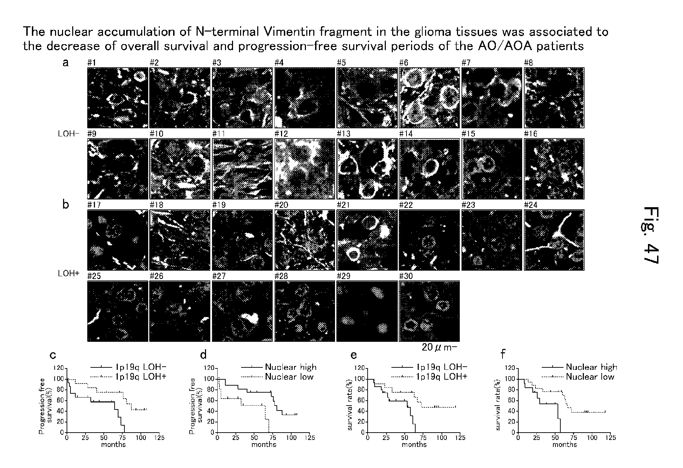

Further, in order to confirm the nuclear translocation of the N-terminal vimentin fragment into the nuclei of glioma cells, an immunohistological analysis were implemented for AO/AOA glioma tumor tissues (n = 30) using the anti-N-terminal vimentin antibody (H84) and the anti-C-terminal vimentin antibody (V9). As shown in Figs. 47a and 47b, it was observed that the N-terminal vimentin fragments in the tissues from LOH⁻ AO/AOA patients (n = 16) were nuclear-stained significantly. A total remaining lifetime (OS) and a non-progressive lifetime (PFS) of LOH⁻ AO/AOA patients who were very likely to be nuclear-stained with the anti-N-terminal vimentin antibody were very shorter, on average, than those of LOH⁺ AO/AOA patients who were unlikely to be nuclear-stained therewith (OS: p = 0.014; PFS: p = 0.0059). These results revealed that not only the OS but also the PFS were almost equal between the LOH⁻ AO/AOA patients and the LOH⁺ AO/AOA patients (OS: p = 0.0092; PFS: p = 0.0066; Figs. 47 c-f). This can be said to suggest that the cellular nuclear translocation and accumulation of the N-terminal vimentin fragments in the AO/AOA tumor tissues may become a diagnosis marker useful for examining the survival of patients.

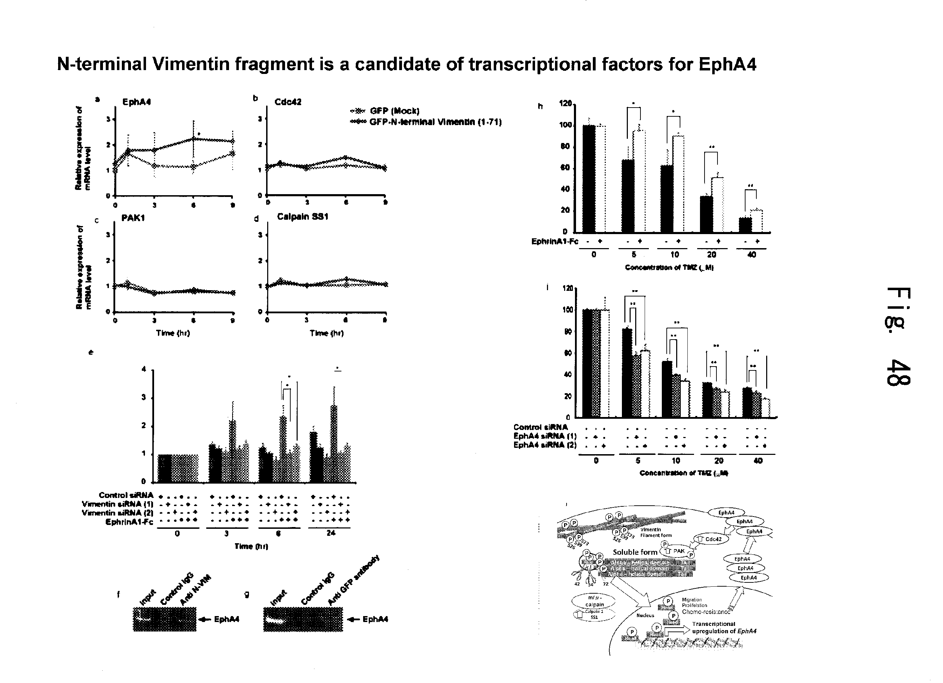

Moreover, investigations were performed into the possibility of the N-terminal vimentin fragments translocated into the cellular nuclear of AO/AOA glioma cells working as an agent for regulating a transcription factor. From the data of the integrated proteomic analysis, EphA4, Cdc42, PAK1 and calpain SS1, which were increased by upregulation of their expression levels or activation in the network signals extracted from the LOH⁻ tissues, were found to work as candidates for transcription target genes of the nuclear N-terminal vimentin fragments. RT-PCR was then implemented for EphA4, Cdc42, PAK1 and calpain CAPNS1 using the A172 glioma cells after the overexpression of the GFP-N-terminal vimentin fragments (1-71) (GFP-N-Vim). The results of the RT-PCR analysis revealed that only EphA4 (EphA4) mRNA was proliferated in the GFP-N-Vim cells in a time-dependent fashion (Fig. 48a). On the other hand, no variations of the other molecules were recognized (Figs. 48b-d). This suggests that EphA4 may become a candidate for the gene responsible for the GFP-N-terminal vimentin (N-Vim).

Furthermore, in order to confirm the possibility of EphA4 gene working as a transcription candidate of N-Vim, an analysis was implemented by comparing the expression of EphA4 (EphA4) mRNA after stimulation with ephrin A1 in the U373 cells with the cells from which vimentin was knocked down with siRNA. Although the upregulation of the time-dependent expression of E(EphA4) mRNA after stimulation with ephrin A1 was observed in the U373 cells treated with control siRNA, the expression of EphA4 mRNA was completely suppressed in the U373 cells treated with Eph4 mRNA (Fig. 48e). Therefore, a connection activity of the N-Vim to a consensus site in an EphA4 promoter was analyzed by a chromatin immunoprecipitation (ChIP) assay in order to clarify whether the N-terminal vimentin fragment may become a transcription factor of EphA4. The results of the assay revealed that the endogenous vimentin in the U373 cells as well as the vimentin that was temporarily overexpressed and immunoprecipitated with the anti-N-terminal vimentin fragment antibody (H84) and an anti-GFP antibody in the U373 cells upregulated the EphA4 promoter DNA significantly. This suggests that the N-Vim can be connected to the promoter site of the EphA4 gene and the transcription was activated (Figs. 48f, g).

In addition, in order to investigate a relationship of the transcription activation of EphA4 with the sensitivity to chemotherapy of the glioma cells, an analysis was implemented for the sensitivity of the U373 cells to TMZ, i.e., an alkylating agent clinically applied most to the treatment of patients with malignant glioma, after stimulation with ephrin A1 or knocking-down of EphA4 with siRNA. The results of this analysis indicated that the sensitivity of the U373 cells to TMZ was downregulated by stimulation with ephrin A1 (Fig. 48h). On the other hand, the sensitivity of the U373 cells to TMZ was upregulated after transduction with EphA4 siRNA (Fig. 48i). These results revealed that the activation of EphA4 upregulated the resistance of the U373 cells to chemotherapy.

Given the results of this are combined with the results of the researches achieved by the present inventors such that the glioma cells with vimentin knocked down upregulated the resistance to chemotherapy, the N-terminal vimentin fragments may become a transcription factor for upregulating the resistance of the glioma cells to chemotherapy because of the activation of Cdc42 and PAK1 accompanied with the activation of EphA4 by the vimentin activation loop (Fig. 15) and the phosphorylation of vimentin by way of the activation of calpain due to this activation as well as the activation of the transcription activity of EphA4 by the nuclear translocation.

As described above, the present invention has established the integrated proteomics analysis technique for an analysis of the activation networks specific to tumors by constructing the application software iPEACH. This software iPEACH is able to integrate molecular lists obtained separately and independently from several kinds of mRNA data from the DNA microarray method, the iTRAQ method, the 2D-DIGE method, etc., the proteomic expression data and modifications data on the identical platform. This software is also useful for treating a new data set for functional analyses including the GO analysis, network analysis, and so on. By implementing these analyses consecutively, information on the molecule important for further studies can be readily selected by focusing on or narrowing the particular molecule down from an enormous volume of information of all molecules.

By utilizing the strategy as described above, the present invention has established an analysis of the chemosensitivity of malignant cancers, in particular 1p/19q LOH⁻ and LOH⁺ AO/AOA. It is to be noted herein, however, that molecular mechanisms involved in the significance of the associated genes located on the chromosomal arm or the deletion from the locus 1p/19q on diagnosis are left still unknown.

In accordance with the present invention, the new EphA4-to-vimentin activation loop which was activated in an abnormal way and related to the sensitivity to chemotherapy of malignant tumors was identified by the comprehensive molecular analysis of the AO/AOA using the data mining software, iPEACH, established by the present inventors. As described above, this network is composed of the consecutively activated cascade consisting of EphA4 - Cdc42 - PAK1, the phosphorylation of vimentin with the fragmentation of vimentin with calpain, and the N-terminal vimentin fragments translocated into the cell nuclei, thereby activating the transcription of EphA4. Interestingly, the genes encoding Cdc42, PAK1 and calpain small subunit 1 (CAPNS1), each upregulating the expression levels in the 1p/19q LOH⁻ group resistant to chemotherapy, were localized on the chromosomal arm 1p or 19q and the EphA4-to-vimentin activation loop upregulating the expression of Cdc42, PAK1 and calpain was associated significantly with the loss of heterozygosity (LOH) of 1p/19q as well as they are deeply involved in non-progressive life expectancy of patients after the treatment by chemotherapy.

In accordance with the present invention, evidence of the nuclear translocation of the N-terminal vimentin fragments in the glioma cells and the glioma tissues of patients was shown, for the first time, by immunocytological and immunohistological analyses of the products extracted from cell nuclei using Western blotting with a particular N-terminal vimentin antibody. Interestingly, the ChIP and RT-PCR analyses revealed that the nuclear translocated N-terminal vimentin fragments were working as a transcription factor of EphA4. In the glioma cells, the expression of EphA4 mRNA was upregulated by the overexpression of the N-terminal vimentin fragments, thereby upregulating the expression of EphA4 by stimulation with ephrin A1 and suppressing it by the treatment with EphA4 mRNA.

In addition, it is found that the upregulation of the expression of EphA4 by stimulation with EphA1 or the downregulation of the expression of EphA4 by treatment with a chemotherapeutic agent, TMZ, is deeply associated with a decrease or an increase in the sensitivity to TMZ, respectively. A combination of these results clarifies that the new EphA4-to-vimentin activation loop is associated with the sensitivity to chemotherapy. This loop upregulates the expression of the N-terminal vimentin fragments for the cellular nuclear translocation, which in turn upregulated the expression of the Cdc42 - PAK1 - calpain activation cascade. This cascade consists of a series of reactions involved in the vimentin activation of Cdc42, the vimentin phosphorylation with PAK1, the vimentin fragmentation with calpain, and the cellular nuclear translocation of the N-terminal vimentin fragments upregulating the expression of the EphA4 transcription). In accordance with the present invention, it is confirmed, for the first time, that the N-terminal vimentin fragments which are intercellular filament proteins function as a transcription factor upregulating the resistance of malignant glioma to chemotherapy.

Vimentin of type III intermediate-sized filament (IF) protein is a major IF protein in mesenchymal cells and is used commonly as a cell or tissue marker. As vimentin has a high sequence homology among species, this suggests that it may play a physiologically important role. Recently, vimentin attracts attention to its role as a factor of an epithelial to mesenchymal transition occurring during embryogenesis and metastasis, but its functions are not yet clarified in detail. The N-terminal and C-terminal vimentins which have a typical head-to-tail relationship as shown in IF proteins are interacted with various structuring and signaling molecules, and the phosphorylation of the vimentin may function as a key regulator of the vimentin-dependent IF dynamics and adjusts the configuration of the IF networks and the intercellular distribution of IF proteins.

Vimentin is also known as having a very complex pattern of phosphorylation. It is found that cAMP-dependent protein kinase, protein kinase C, Ca²⁺/calmodulin-dependent protein kinase II (CaM kinase II), PAK1, Cdk1/Cdc2 kinase, Rho kinase, Aurora-B and polo-like kinase 1 (Plk-1) have sites or kinases specific to different states of cells including, for example, mitosis, differentiation, stress, and so on. It is also found that a target of phosphokinase, PAK1 and Cdc42/Rac is desmin, which is a muscle-specific type III IF protein and works as a key kinase to vimentin. There are reports that Ser25, Ser38, Ser50, Ser65 and Ser72 of vimentin in the terminal amino acid terminal head domain are biologically major sites for phosphorylation with PAK1 and the phosphorylated vimentin has lost its ability to create a 10 nm filament, thereby its soluble form missing the ability of forming the filament as well as inducing filament reconstruction, solubility and stability.

The phosphorylation of the IF proteins shifts its equilibrium to the soluble form side protecting the proteins from being decomposed, thereby maintaining a large pool and facilitating the re-formation of the IF proteins. It is reported that, in the case of vimentin, as in the case of GFAP, the phosphorylation of vimentin may delay its decomposition, but cleave specific proteins which may be associated with other cell functions.Calpain-1 and caspase are reported as enzymes for cleaving vimentin. The present inventors have conducted an observation of the vimentin cleavage site 71R in malignant glioma, which is reported as involved in the vascular smooth muscle cell. These results suggest that the specific vimentin fragmentation with calpain is suppressed by the phosphorylation of vimentin with PAK1.

There is also a report suggesting that vimentin possesses an ability of interaction with specialized DNA structures including, for example, a satellite DNA, a telomere DNA retroposon, mitochondria DNA, and so on. The N-terminal vimentin head is identified as an in vitro responsible region of the DNA linkage. Further, it is made clear that vimentin is associated with a protein connected to a nuclear matrix attachment region (MAR) and it is remarkably associated with a MAR motif containing DNA. In accordance with the present invention, the N-terminal vimentin fragments connected in vivo to the nuclear DNA of the glioma cells is identified as a transcription factor involving in chemotherapy.

An ephrin receptor (Eph) represents the largest family called a receptor-type tyrosine kinase and interacts with ephrin as its ligand. ThEphrin-to-Eph signaling exerts an influence mainly on a morphology and motor ability of a cell by regulating the cytoskeletal construction and the cell adhesion as well as on the determination of the cell proliferation and the cell fate. Therefore, the Eph signaling may be considered to play a role somewhat in oncogenesis. Recently, the genes encoding the Eph receptor and ephrin are recognized to be differentially expressed in various tumors including, for example, malignant melanoma, glioma, prostatic cancer, breast cancer, lung small cell carcinoma, endometrial carcinoma, esophageal cancer, gastric cancer, and colorectal cancer. A serious distortion of the expression patterns is considered to be involved in worsening of a prognosis of patients due to variations in the tumor behaviors including, for example, an upregulation of invasion or metastatic potential. Regardless of the downregulation observed widely in the overexpression of the Eph receptor, a role in the process of its malignant phenotype is not yet clarified thoroughly. Recent evidence indicates, however, that the Eph signaling path is involved in the progress of tumors. Studies on a gene expression analysis suggest an abnormal expression of the EphA4 receptor in advanced astrocytic cancer and an influence of the overexpressed EphA4 on the malignant phenotype of tumor cells, the EphA4 being caused to be overexpressed by proliferation and an increase in translocation by way of an interaction with FGFR 1 (fibroblast growth factor receptor 1). In addition, there is also a report of an increase of active type Rac and Cdc42 in the EphA4-overexpressed cells. The present invention, however, is the first report that vimentin is the most potential candidate which may be regulated by the activation loop through vimentin involved in the resistance of glioma to chemotherapy.

As described above, the quantitative proteomics strategy is potent and useful to present the comprehensive features of biological phenomena. Although these techniques have been applied to retrieve biomarkers for tumors and new medicines, the methods for competing with technical issues occurring upon treatment of an enormous volume of data obtained by experiments are limited, when the quantitative proteomics strategy will be attempted to be used for biological or functional analyses. There may be mentioned four technical issues as will be described below.

(1) A scope of applying the proteome is theoretically restricted because the quantitative identification by a proteomics of one type such as a MS system based on a LC shotgun or an analysis based on a 2D gel electrophoresis cannot cover a whole proteome due to differences in categories of identifying molecules resulting from the specificity of each of the treatment methods; (2) as public protein databases use their original accession numbers or synonyms, they has only a limited number of information on genes, they use search engines which are inappropriate in many cases to efficiently and comprehensively analyze original raw data obtainable from experiments, and they are too inconvenient and complex to retrieve an enormous number of information on molecules in a lump; (3) as there is no software which is simple and ready to use for automatically providing annotations such as GO, genetic locus, OMIM, interactome, etc., an enormous volume of work is required for information processing of analysis results of expression of several thousand kinds of proteins and, at the same time, no means is devised to automatically acquire functional information by converting the results of analysis of organism species whose genomic information is not elucidated thoroughly; and (4) transcriptomic data obtainable from DNA microarrays cannot be integrated readily with proteomic data because methods for quantitation and modes of analyzing results are different from those of the proteomic data even if the original raw material would be identical to each other.

Therefore, the data set for the integrated proteomic analysis and the programs (iPEACH/MANGO) according the present invention is installed with a simple and quickly processible tool that integrates (a) comprehensive proteomic data containing post-translational modifications, (b) comprehensive transcriptomic data and (c) information on molecular functions sorted out from several kinds of public databases integrated with information on original data (raw data) without impairing the information on the original data reconstructs these data, reinforces, classifies (data-mine), and specifies a candidate molecule relating to other processings. The present invention makes it possible, for the first time, to complete studies on mechanisms of resistance of malignant tumors, such as glioma, to chemotherapy by utilizing the data set for the integrated proteomic analysis according to the present invention.

### INDUSTRIAL APPLICABILITY

The integrated proteomic analysis data set (iPEACH) according to the present invention can provide the effects as will be summarized below: (1) to provide differential proteomic data containing data on post-translational modifications; (2) to provide differential transcriptomic data; and (3) to provide a useful and quickly processible tool for providing the data with useful annotations from various public databases, organizing results of comprehensive analysis by the integration of the above data set, reinforcing, classifying, and specifying a candidate molecule relating to other methods.

The present invention illustrates a portion of examples of molecular signal network analyses using human tumor tissues or cells. It is to be noted herein as a matter of course that this methodology underlying in the present invention can be applied to an analysis of all kinds of diseases and pathological conditions as well as approaches to an acquisition of basic molecular information to be applicable to cell biology. Under current circumstances, it is impossible to cover information on all proteomes by a single methodology so that there is no way but to proceed with an implementation of an analysis in such a manner as have so far been done. In accordance with the present invention, however, several methodologies are combined together, and information obtained by the respective methodologies can be introduced therein one after another and then subjected to integrated mining, thereby bringing interesting molecular signal networks into relief in a simulation-like fashion. These processes may offer ideas to be applicable to a new way of elucidation of pathological mechanisms and development of markers and medicines useful for diagnosis and treatment of diseases. It may further be noted that, although development of integrated software that could integrate data from these databases and extract it upstream is delayed, the basic information including, but being not limited to genomic information of tissues and cells and transcriptomic information nevertheless are very useful. Moreover, pathological, morphological and clinical-medical opinions and observations may drive the highest motivation so that the proteomics may generate high-quality information by standing over the above information. From these points of view, it is essential and requisite to construct a large-scale integrated database relating to molecular functions of the life of a living body and its analysis system, which can be shared with people engaging in researches of all life science including, for example, genome, transcriptomics, proteinomics, cytobiology, pathology, information technology, medical science, pharmaceutical science, physics, agricultural technology, life science, and so on.

By the present invention, the algorithm (iPEACH/MANGO) has been developed, which can integrate the proteomic analysis data and the transcriptomic analysis data by merging approaches of new, highly sensitive and high-throughput technologies of proteomics, and it has proved to effectively extract a group of signaling molecules activated in tumor tissues and cells. As the molecular signals involved in the sensitivity of malignant glioma to anticancer agents can be analyzed in detail by the integrated proteomic analysis method according to the present invention, a new vimentin activation loop composed of vimentin and a group of the molecules responsible for its post-translational modifications as well as a group of its activated signaling molecules are extracted. The results of verification experiments make it clear that variations in expression levels and structures of the molecule groups are greatly involved in the resistance of malignant glioma to chemotherapy. Moreover, the results of an analysis of expression patterns of the pathological samples reveal that these molecule group may become a marker for predicting the treatment or prognosis or a target for treatment and, in addition, that they can be applied to an analysis of mechanisms of numerous diseases.

## Claims

1. A generation method for generating a data set for an integrated proteomic analysis based on a data set of comprehensive protein expression variation amounts between two different sample groups and a data set of comprehensive gene expression variation amount between the two different sample groups; comprising:
a common protein identity number assignment step for providing a comprehensive protein expression variation amount data of an individual protein, constituting the data set of comprehensive protein expression variation amounts, with a common protein identity number linking to a protein identity number of the individual protein in a first database and a gene identity number of a gene encoding the individual protein in a second database;
a common gene identity number assignment step for providing a comprehensive gene expression variation amount data of an individual gene, constituting the data set of comprehensive gene expression variation amount, with a common gene identity number linking to a protein identity number of the individual gene in a third database and the protein identity number of the individual protein expressed from the individual gene in the first database;
a data connection step for forming a connected data composed of the protein expression variation amount data of the individual protein and the gene expression variation amount data of the individual gene by connecting the data set of comprehensive protein expression variation amounts obtained in the protein identity number assignment step to the data set of comprehensive gene expression variation amounts obtained in the gene identity number assignment step;
a data rejection step for rejecting data having a p-value equal to or higher than a predetermined value, the p-value being obtained by a significance test of a protein expression variation amount or a gene expression variation amount between the two different sample groups among the expression variation amount data constituting the connected data set or a F-value equal to or lower than a predetermined value obtained by a variance analysis (ANOVA) thereof; and
a data acceptance step for accepting either of data which is provided with the equal common identity number for both of the protein expression variation amount data and the gene expression variation amount data among the integrated data set obtained through the data rejection step on the basis of a predetermined condition in order to generate a data set for an analysis of a protein function.

2. The generation method for generating the integrated proteomic analysis data set as claimed in claim 1, wherein said comprehensive protein expression variation amount data set comprises a data set obtainable by a comprehensive protein expression analysis using liquid chromatography and mass spectrometry and/or a data set of information on proteins including post-translational modified proteins obtainable by a fluorescence-labeled two-dimensional difference gel electrophoresis and mass spectrometry.

3. The generation method for generating the integrated proteomic analysis data set as claimed in claim 1 or 2, wherein said comprehensive gene expression variation amount data set comprises a data set obtainable by DNA microarray analysis.

4. The generation method for generating the integrated proteomic analysis data set as claimed in any one of claims 1 to 3, wherein said predetermined condition in the data acceptance step is the protein expression variation amount data.

5. The generation method for generating the integrated proteomic analysis data set as claimed in any one of claims 1 to 4, wherein said two different sample groups are each a sample which has an observation identical to each other yet dynamics different from each other.

6. An integrated proteomic analysis method for implementing an integrated proteomic analysis of the integrated proteomic analysis data set generated by the generation method for generating the integrated proteomic analysis data set as claimed in any one of claims 1 to 5.

7. The integrated proteomic analysis method as claimed in claim 6, wherein a color indicative of a molecule corresponding to each of the expression variation amount data visualized by the integrated proteomic analysis is changed in accordance with an expression variation amount value of the expression variation amount data constituting the integrated proteomic analysis data set.

8. A method for identifying a causative substance wherein a protein having the maximal expression variation amount is identified as a causative substance among proteins linked to each other and retrieved by implementing GO analysis and network analysis of the integrated proteomic analysis data set generated by the generation method for generating the integrated proteomic analysis data set as claimed in any one of claims 1 to 5.

9. A method for identifying a causative substance, wherein the protein having the maximal expression variation amount is identified as a causative substance among the proteins linked to each other and retrieved by the integrated proteomic analysis method as claimed in claim 6 or 7.

10. The method for identifying the causative substance as claimed in claim 7 or 8, wherein a protein linked to said causative protein adjacent upstream or downstream or post-translationally modified protein of said causative protein is identified as the causative substance by network analysis.

11. The method for identifying the causative substance as claimed in any one of claims 8 to 10, wherein said causative protein identifies a protein associated with dynamics in a living body.

12. The method for identifying the causative substance as claimed in any one of claims 8 to 11, wherein said dynamics is pharmacokinetics and is involved in abnormality relating to cell proliferation, cell differentiation or apoptosis.

13. The method for identifying the causative substance as claimed in any one of claims 8 - 12, wherein said causative substance is vimentin, phosphorylated vimentin, vimentin fragment, ephrin, ephrin receptor or hypoxia-inducible factor-1 or a network structuring factor group containing one of the above causative substances as a core.

14. A method for using a causative protein comprising using the causative protein identified by the method for identifying the causative substance as claimed in any one of claims 8 to 13 as a marker for retrieving dynamics associated with a medicine.

15. The method for using the causative protein as claimed in claim 14, wherein said causative protein is used as a tumor marker.

16. The method for using the causative protein as claimed in claim 14 or 15, wherein said causative protein is vimentin, phosphorylated vimentin, vimentin fragment, ephrin, ephrin receptor or hypoxia-inducible factor-1 or a network structuring factor group containing one of the above causative substances as a core.

17. A method for inhibiting an expression of a causative protein comprising treating or preventing an event caused to occur in the causative protein by inhibiting the expression of the causative protein identified by the method for identifying the causative protein as claimed in any one of claims 8 to 13.
